# EUROPEAN PATENT APPLICATION

(11) **EP 1 795 524 A1**
(43) Date of publication of application: **13.06.2007**
(21) Application number: 05785275.8
(22) Date of filing: 20.09.2005
(51) Int. Cl.: C07C 217/54

(54) **CYCLIC AMINE DERIVATIVE OR SALT THEREOF**

(30) Priority: 21.09.2004 JP 2004274105
(71) Applicant: Astellas Pharma Inc., Tokyo 103-8411 (JP)
(72) Inventor: HAYASHIBE, Satoshi, c/o Astellas Pharma Inc.,, Tokyo 1038411 (JP); KANAYAMA, Takatoshi, c/o Astellas Pharma Inc.,, Tokyo 1038411 (JP); OHMORI, Junya, c/o Astellas Pharma Inc.,, Tokyo 1038411 (JP); TOBE, Takahiko, c/o Astellas Pharma Inc.,, Tokyo 1038411 (JP); MAENO, Kyoichi, c/o Astellas Pharma Inc.,, Tokyo 1038411 (JP); SHITAKA, Yoshitsugu, c/o Astellas Pharma Inc.,, Tokyo 1038411 (JP); SUZUKI, Jotarou, c/o Astellas Pharma Inc.,, Tokyo 1038411 (JP); KAWABATA, Shigeki, c/o Astellas Pharma Inc.,, Tokyo 1038411 (JP); SHIRAISHI, Nobuyuki, c/o Astellas Pharma Inc.,, Tokyo 1038411 (JP); YAMASAKI, Shingo, c/o Astellas Pharma Inc.,, Tokyo 1038411 (JP); SUZUKI, Daisuke, c/o Astellas Pharma Inc.,, Tokyo 1038411 (JP); HOSHII, Hiroaki, c/o Astellas Pharma Inc.,, Tokyo 1038411 (JP)
(74) Representative: HOFFMANN EITLE
(86) International application number: PCT/JP2005/017272
(87) International publication number: WO 2006/033318

(57) **Abstract**

Provided are compounds which are an NMDA antagonist having a broad safety margin and are useful as a treating agent or a preventing agent for Alzheimer's disease, cerebrovascular dementia, Parkinson's disease, ischemic apoplexy, pain, etc. Concretely provided are an amine derivative or its salt **characterized in that** the amine-containing structure A therein bonds to a 2- or 3-cyclic condensed ring (e.g., indane, tetralone, 4,5,6,7-tetrahydrobenzothiophene, 4,5,6,7-tetrahydrobenzofuran, 7,8-dihydro-6H-indeno[4,5-b]furan, 2,3-dihydro-1H-cyclopenta[1]naphthalene) via X¹ (bond or lower alkylene); and an NMDA antagonist containing it as an active ingredient thereof.

## Description

### TECHNICAL FIELD

The present invention relates to a cyclic amine derivative and its salt useful as medicines, especially as NMDA receptor antagonists, and to an NMDA receptor antagonist comprising it as the active ingredient thereof. The cyclic amine derivative and its salt and the NMDA receptor antagonist comprising it as an active ingredient according to the present invention are useful for treatment and prevention of Alzheimer's disease, cerebrovascular dementia, Parkinson's disease, ischemic apoplexy, pain, etc.

### BACKGROUND ART

Glutamic acid acts as a neurotransmitter in the central nervous system of mammals, and it controls the activity of neurocytes or the release of neurotransmitters via the glutamate receptor existing in synapses. At present, the glutamate receptor is classified into an "ion-channel glutamate receptor" and a "metabotropic glutamate receptor" from many physiological and biological studies (Hollmann M. and Heinemann S., Annu. Rev. Neurosci., 17 (1994) 31-108). NMDA (N-methyl-D-aspartate) receptor is an ion-channel glutamate receptor specifically sensitive to the agonist NMDA (Moriyoshi K. et al., Nature, 354 (1991) 31-37; Meguro H. et al., Nature, 357 (1992) 70-74); and this has high Ca²⁺ permeability (Iino M. et al., J. Physiol., 424 (1990) 151-165). NMDA receptor is expressed with a specific pattern in a central nervous system (Ozawa S. et al., Prog. Neurobiol., 54 (1998) 581-618).
From many pharmacological and biological studies, it is considered that NMDA receptor may participate in high-order neurologic functions such as memory and learning (Morris RG., et al., Nature, 319 (1986) 774-776; Tsien JZ. et al., Cell, 87 (1996) 1327-1338). On the other hand, it is suggested that acute or chronic NMDA receptor hyperactivity or hypoactivity may participate in various nervous system diseases, for example, ischemic apoplexy, hemorrhagic brain injury, traumatic brain injury, neurodegenerative disorders (e.g., Alzheimer's disease, cerebrovascular dementia, Parkinson's disease, Huntington's disease, amyotrophic lateral sclerosis), glaucoma, AIDS encephalopathy, dependence, schizophrenia, depression, mania, stress-related diseases, epilepsy, pain (Beal MF., FASEB J., 6 (1992) 3338-3344; Heresco-Levy U. and Javitt DC., Euro. Neuropsychopharmacol., 8 (1998) 141-152; Hewitt DJ., Clin. J. Pain, 16 (2000) S73-79). Accordingly, drugs capable of controlling the activity of NMDA receptor would be extremely useful in clinical application.

As drugs capable of controlling the activity of NMDA receptor, a large number of non-competitive NMDA receptor antagonists are reported, but many of them are not as yet used in clinical application because of their side effects based on the NMDA receptor-antagonizing effect thereof, for example, mental aberration such as hallucination or confusion, or giddiness. Some already-existing NMDA receptor antagonists, for example, ketamine and dextromethorphan have been tried for pain in clinical application (Fisher K. et al., J. Pain Symptom Manage., 20 (2000) 358-373), but the safety margin in the treatment with them is narrow and their clinical use is limitative (Eide PK., et al., Pain, 58 (1994) 347-354). Memantine is known as a non-competitive NMDA receptor antagonist that has comparatively few side effects (Parsons CG., et al., Neuropharmacol., 38 (1999) 735-767); and recently, it has been reported that this may be effective for Alzheimer's disease (Reisberg B., et al., N. Engl. J. Med., 348 (2003) 1333-1341). However, the safety margin of memantine as a medicine is still not satisfactory, and an NMDA receptor antagonist having a broader safety margin is desired (Ditzler K., Arzneimittelforschung, 41 (1991) 773-780; Maier C., et al., Pain, 103 (2003) 277-283; Riederer P., et al., Lancet, 338 (1991) 1022-1023). It is expected that the creation of such an NMDA receptor antagonist having a broader safety margin may bring about new clinical usefulness of the NMDA receptor antagonist.

Patent Reference 1 describes a pharmaceutical composition for prevention and treatment of cerebral ischemia, which comprises an adamantane derivative of the following formula or its pharmaceutically-acceptable acid-addition salt. 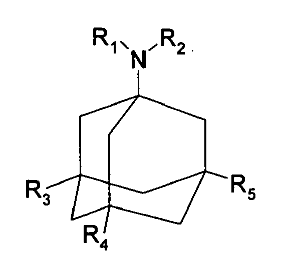 (wherein R₁ and R₂ are the same or different, each representing a hydrogen atom, or a linear or branched alkyl group having from 1 to 6 carbon atoms, or the like; R₃ and R₄ are the same or different, each representing a hydrogen atom, or an alkyl group having from 1 to 6 carbon atoms, or the like; R₅ represents a hydrogen atom, or a linear or branched alkyl group having from 1 to 6 carbon atoms; for the details of the symbols in the formula, the patent publication is referred to).
In Patent Reference 1, the above-mentioned memantine is described as Test Compound No. 1 (memantine is a compound of the formula where R₁, R₂ and R₃ are hydrogen atoms, and R₄ and R₅ are methyl groups).

Patent Reference 2 describes 1-amino-alkylcyclohexane of the following formula as an NMDA receptor antagonist. 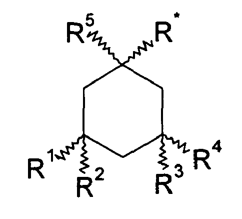 (wherein R* is -(CH₂)ₙ-(CR⁶R⁷)ₘ-NR⁸R⁹; n + m = 0,1 or 2; R¹ to R⁹ are independently selected from a group consisting of a hydrogen atom and a C₁₋₆ lower alkyl group, and at least R¹, R⁴ and R⁵ are lower alkyl groups; for the details of the symbols in the formula, the patent publication is referred to).

Patent Reference 3 describes an indane derivative of the following formula, which is a type of a compound having an indane ring and a piperidine ring, and useful as a treating agent for allergy and asthma. 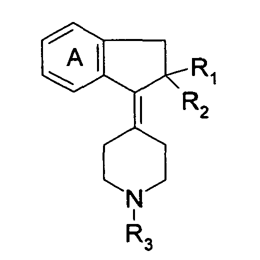 (wherein R₁ and R₂ each independently represent a C₁₋₄ alkyl group; R₃ represents a hydrogen atom, a C₁₋₄ alkyl group, a C₁₋₄ alkoxy group, or the like; and the ring A may have a substituent; for the details of the symbols in the formula, the patent publication is referred to).
However, in the compound described in this reference, the indane ring and the piperidine ring are bonded to each other via a double bond therebetween, and the structure of the compound differs from that of the compound of the present invention in that in the latter, the two rings are bonded to each other via a single bond or a lower alkylene group, etc. In addition, the compound described in the reference is useful as a treating agent for allergy and asthma, but the reference does neither disclose nor suggest the NMDA receptor-antagonizing effect of the compound and the usefulness of the compound as a treating agent for Alzheimer's disease, cerebrovascular dementia, Parkinson's disease, ischemic apoplexy, pain, etc. In Example 1 in this reference, described is 6-chloro-2,2-dimethyl-1-(1-methyl-4-piperidinyl)indan-1-ol of the following formula, as an intermediate for producing the claimed compound. However, this reference discloses nothing relating to the use of the intermediate for medicine.

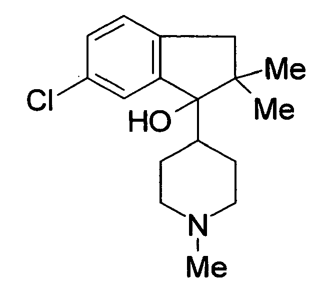

Non-Patent Reference 1 describes a compound of the following formula, which is a type of a compound having an indane ring or a tetrahydronaphthalene ring and a piperidine ring, and may be used as an analgesic agent. However, the structure of this compound differs from that of the compound of the present invention described hereinunder, in that in the former, the saturated ring is substituted with a phenyl group. 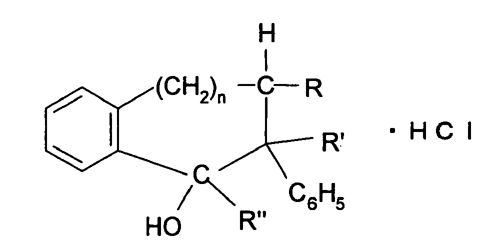 (wherein R represents a hydrogen atom, or a methyl group; R' represents a methyl group; R" represents -(CH₂)₃N(CH₃)₂, -(CH₂)₃N(CH₃)(CH₂)₂C₆H₅, an N-methyl-piperidyl group, - (CH₂)₂N(CH₃)₂; n indicates 0 or 1; for the details of the symbols in the formula, the patent publication is referred to).

Patent Reference 1: Japanese Patent No. 2821233,
Patent Reference 2: WO 99/01416,
Patent Reference 3: JP-A 56-135472,
Non-Patent Reference 1: Journal of Medicinal Chemistry, 1967, Vol. 10, No. 5, pp. 823-825.

### DISCLOSURE OF THE INVENTION

### PROBLEMS THAT THE INVENTION IS TO SOLVE

With the increase in the old age population, cases of Alzheimer's disease, cerebrovascular dementia, ischemic apoplexy and the like are increasing these days; and it is earnestly desired in the field of medicine to create an NMDA receptor antagonist which is effective for treatment and prevention of such diseases as well as Parkinson's disease, pain and others and has a broader safety margin. An object of the present invention is to provide a novel cyclic amine derivative and its salt having an excellent NMDA receptor antagonistic activity and having a broader safety margin, and to provide a pharmaceutical composition containing it.

### MEANS FOR SOLVING THE PROBLEMS

The present inventors have found that a novel amine derivative and its salt [1] represented by the following formula (1), which is characterized in that the amine-containing structure A therein bonds to a 2-cyclic or 3-cyclic condensed ring (e.g., indane, tetralone, 4,5,6,7-tetrahydrobenzothiophene, 4,5,6,7-tetrahydrobenzofuran, 7,8-dihydro-6H-indeno[4,5-b]furan, 2,3-dihydro-1H-cyclopenta[a]naphthalene) via X¹ (e.g., bond or lower alkylene), have an excellent NMDA receptor antagonistic activity and a broad safety margin, and have completed the present invention. Specifically, the present invention relates to an amine derivative of the following formula (I) and its salt (hereinafter this may be referred to as "the compound (I) of the present invention"). Further, the present invention also relates to an NMDA receptor antagonist comprising the compound (I) or its salt of the present invention, especially a treating agent or a preventing agent for Alzheimer's disease, cerebrovascular dementia, ischemic apoplexy, pain, etc. In the present invention, the compounds of the following [2] are preferred, and the compounds of the following [3] are more preferred. The compounds of [4] are most preferred.

[1] A cyclic amine and its salt of the following formula (I): 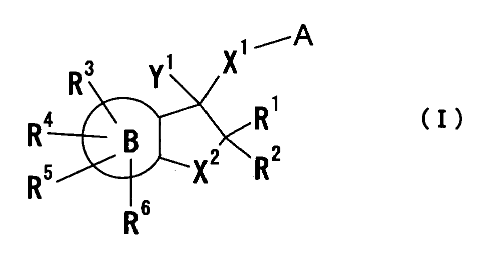 (wherein the symbols have the following meanings:
   A: a 5- to 8-membered cyclic amine optionally having a double bond, optionally having a bridge structure and optionally having a substituent of R⁷ to R¹¹ in the ring, or -NH₂, -NH(lower alkyl), or -N(lower alkyl)₂;
   Ring B: benzene, thiophene, furan, pyrrole, a 5- to 7-membered cycloalkane, or a 5- to 7-membered cycloalkene;
   X¹: a bond, a lower alkylene, or -L³-D-L⁴-;
   L³ and L⁴: the same or different, and a bond or a lower alkylene;
   D: a 5- or 6-membered carbon ring or hetero ring optionally having a substituent;
   X²: -(CR¹²R¹³)n-, -N(R¹⁴)-, -N(R¹⁴)CO-, -CON(R¹⁴)-, -O-, -S-, -CO-, -CH(OH)-, -N(R¹⁴)-(CR¹²R¹³)n-, -(CR¹²R¹³)n-N(R¹⁴)-, -CON(R¹⁴)-(CR¹²R¹³)n-, -N(R¹⁴)CO-(CR¹²R¹³)n-, -(CR¹²R¹³)n-N(R¹⁴)CO-, -(CR¹²R¹³)n-CON(R¹⁴)-, -CO-(CR¹²R¹³)n-, -(CR¹²R¹³)n-CO-, -O-(CR¹²R¹³)n-, -(CR¹²R¹³)n-O-, -S-(CR¹²R¹³)n-, or -(CR¹²R¹³)n-S-;
   Y¹: -OH, -O-lower alkyl, -NH₂, or -N₃;
   R¹ and R²: the same or different, and a halogen atom, a lower alkyl, or a lower alkylene-OH;
   R³ to R⁶: the same or different, and a hydrogen atom, a halogen atom, a lower alkyl, a lower alkenyl, a lower alkynyl, -O-lower alkyl, -OH, -NH₂, -NH(lower alkyl), -N(lower alkyl)₂, -NH-CO-lower alkyl, -N(lower alkyl)-CO-lower alkyl, -NH-CO-O-lower alkyl, -N(lower alkyl)-CO-O-lower alkyl, -CN-, -NO₂, -CF₃, -lower alkylene-OH, -lower alkylene-halogen atom, -O-lower alkylene-OH, -lower alkylene-O-lower alkyl, -CO-N(lower alkyl)₂, -CO-NH-(lower alkyl), -O-CO-N(lower alkyl)₂, -O-CO-NH-(lower alkyl), a 5- to 8-membered cyclic amine, -CO-5- to 8-membered cyclic amine, -COOH, -COO-lower alkyl, -COO-lower alkylene-aryl, a 5- or 6-membered hetero ring, -lower alkylene-5- or 6-membered hetero ring, an aryl optionally having a substituent, or an aryl optionally having -lower alkylene-substituent;
   R⁷: a hydrogen atom, a lower alkyl, -lower alkylene-aryl, or -lower alkylene-heteroaryl:
   R⁷ is a substituent on the nitrogen atom of the cyclic amine;
   R⁸ to R¹⁴: the same or different, and a hydrogen atom, or a lower alkyl;
   n: an integer of 1, 2 or 3;
      wherien R⁵ and R⁶, R⁴ and R⁵, or R³ and R⁴ may together form a lower alkylene, -O-lower alkylene-O-, -O-lower alkylene-, -lower alkylene-O-, -S-lower alkylene-, -lower alkyleneS-, -N(R¹⁹)-lower alkylene-, -lower alkylene-N(R¹⁹)-, -C(R¹⁵)=C(R¹⁶)-O-, -O-C(R¹⁵)=C(R¹⁶), -C(R¹⁵)=C(R¹⁶)-C(R¹⁷)=C(R¹⁸)-, -S-C(R¹⁵)=C(R¹⁶)-, -C(R¹⁵)=C(R¹⁶)-S-, -N(R¹⁹)-C(R¹⁵)=C(R¹⁶)-, -C(R¹⁵)=C(R¹⁶)-N(R¹⁹)-, -N(R¹⁹)-C(R¹⁵)=N-, -N=C(R¹⁵)-N(R¹⁹)-, -N=C(R¹⁵)-C(R¹⁶)=C(R¹⁷)-, -C(R¹⁵)=C(R¹⁶)-C(R¹⁷)=N-, -C(R¹⁶)=N-C(R¹⁶)=C(R¹⁷)-, or -C(R¹⁵)=C(R¹⁶)-N=C(R¹⁷); R³ and Y¹ may together form -O-lower alkylene-O-, -lower alkylene-O-, or -N(R¹⁹)-lower alkylene-O-; R¹ and Y¹ may together form -lower alkylene-O-;and Y¹ and the branch on -X¹-A may together form -O-, or -O-lower alkylene;
   R¹⁵ to R¹⁸: the same or different, and a hydrogen atom, a halogen atom, or a lower alkyl group;
   R¹⁹: a hydrogen atom, or a lower alkyl group;
      provided that 6-chloro-2,2-dimethyl-1-(1-methyl-4-piperidinyl)indan-1-ol is excluded from the compound).

[2] A cyclic amine derivative and its salt of the following formula (II): 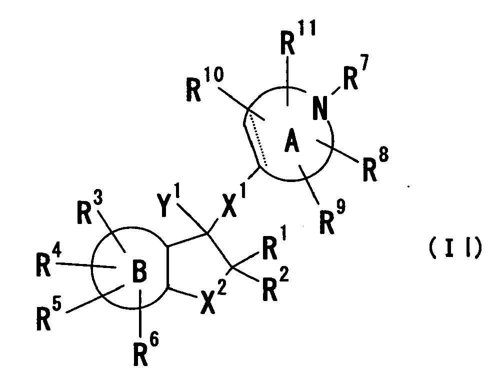 (wherein the symbols have the following meanings:
   Ring A: a 5- to 7-membered cyclic amine optionally having a double bond in the ring;
   Ring B: benzene, thiophene, furan, pyrrole, a 5- to 7-membered cycloalkane, or a 5- to 7-membered cycloalkene;
   X¹: a bond, or a lower alkylene;
   X²: -(CR¹² R¹³)n-, -N(R¹⁴)-, -N(R¹⁴)CO-, -CON(R¹⁴)-, -O-, -S-, -CO-, -N(R¹⁴)-(CR¹²R¹³)n-, -(CR¹²R¹³)n-N(R¹⁴)-, -CON(R¹⁴)-(CR¹²R¹³)n-, -N(R¹⁴)CO-(CR¹²R¹³)n-, -(CR¹²R¹³)n-N(R¹⁴)CO-, -(CR¹²R¹³)n-CON(R¹⁴)-, -CO-(CR¹²R¹³)n-, -(CR¹²R¹³)n-CO-, -O-(CR¹²R¹³)n-, -(CR¹²R¹³)n-O-, -S-(CR¹²R¹³)n-, or -(CR¹²R¹³)n-S-;
   Y¹: -OH, -O-lower alkyl, -NH₂, or -N₃;
   R¹ and R²: the same or different, and a halogen atom, or a lower alkyl;
   R³ to R⁶: the same or different, and a hydrogen atom, a halogen atom, a lower alkyl, -O-lower alkyl, -OH, -CN, or -CF₃;
   R⁷: a hydrogen atom, a lower alkyl, -lower alkylene-aryl, or -lower alkylene-heteroaryl:
   R⁸ to R¹⁴: the same or different, and a hydrogen atom, or a lower alkyl;
   n: an integer of 1, 2 or 3;
      provided that 6-chloro-2,2-dimethyl-1-(1-methyl-4-piperidinyl)indan-1-ol is excluded from the compound).

[3] A cyclic amine and its salt of the following formula (III): 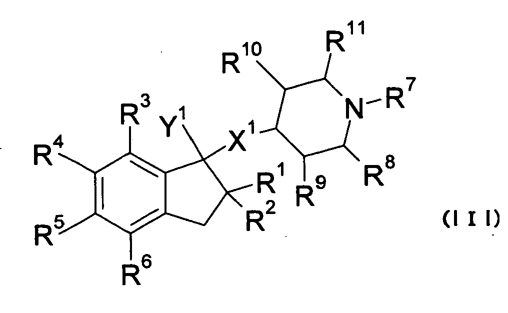 (wherein the symbols have the following meanings:
   X¹: a bond, or a lower alkylene;
   Y¹: -OH, -O-lower alkyl, -NH₂ or -N₃;
   R¹ and R²: the same or different, and a halogen atom, or a lower alkyl;
   R³ to R⁶: the same or different, and a hydrogen atom, a halogen atom, a lower alkyl, -O-lower alkyl, -OH, -CN, or -CF₃;
   R⁷: a hydrogen atom, a lower alkyl, -lower alkylene-aryl, or -lower alkylene-heteroaryl;
   R⁸ to R¹¹: the same or different, and a hydrogen atom, or a lower alkyl;
provided that 6-chloro-2,2-dimethyl-1-(1-methyl-4-piperidinyl)indan-1-ol is excluded from the compound).

[4] The compound and its salt of [1], selected from 2,2-dimethyl-1-(1-methylpiperidin-4-yl)indan-1-ol, 2,2,6-trimethyl-1-(1-methylpiperidin-4-yl)indan-1-ol, 6-fluoro-2,2-dimethyl-1-(1-methylpiperidin-4-yl)indan-1-ol, 5-bromo-2,2-dimethyl-1-(1-methylpiperidin-4-yl)indan-1-ol, 6-chloro-5-fluoro-2,2-dimethyl-1-(1-methylpiperidin-4-yl)indan-1-ol, 6-bromo-4-fluoro-2,2-dimethyl-1-(1-methylpiperidin-4-yl)indan-1-ol, 7-bromo-6-fluoro-2,2-dimethyl-1-(1-methylpiperidin-4-yl)indan-1-ol, 7-bromo-4-fluoro-2,2-dimethyl-1-(1-methylpiperidin-4-yl)indan-1-ol, 6-fluoro-7-methoxy-2,2-dimethyl-1-(1-methylpiperidin-4-yl)indan-1-ol, 4-fluoro-7-methoxy-2,2-dimethyl-1-(1-methylpiperidin-4-yl)indan-1-ol, 2,2-dimethyl-1-(1-methylpiperidin-4-yl)-1,2,3,4-tetrahydronaphthalen-1-ol, 4-fluoro-6-methoxy-2,2-dimethyl-1-(1-methylpiperidin-4-yl)indan-1-ol, 7-ethoxy-6-fluoro-2,2-dimethyl-1-(1-methylpiperidin-4-yl)indan-1-ol, 4-fluoro-6,7-dimethoxy-2,2-dimethyl-1-(1-methylpiperidin-4-yl)indan-1-ol, 5,6-difluoro-7-methoxy-2,2-dimethyl-1-(1-methylpiperidin-4-yl)indan-1-ol, 6-cyano-4-fluoro-7-methoxy-2,2-dimethyl-1-(1-methylpiperidin-4-yl)indan-1-ol, 1-(2-amino-2-methylpropyl)-2,2,6-trimethylindan-1-ol, 5-fluoro-7,7-dimethyl-8-(1-methylpiperidin-4-yl)-3,6,7,8-tetrahydro-2H-indeno[4,5-b]furan-8-ol, 4-(9-fluoro-5,5-dimethyl-2,3,5,6-tetrahydro-4aH-indeno[1,7-ef][1,4]dioxepin-4a-yl-1-methylpiperidine, 1-[(6'-fluoro-7'-methoxy-2',2'-dimethyl-2',3',4,5-tetrahydro-3H-spiro[furan-2,1'-inden]-5-yl)methyl]pyrrolidine.

### EFFECT OF THE INVENTION

The compounds of the present invention have an NMDA receptor antagonistic activity and are useful for treatment and prevention of Alzheimer's disease, cerebrovascular dementia, Parkinson's disease, ischemic apoplexy, pain.

### BEST MODE FOR CARRYING OUT THE INVENTION

The present invention is described concretely hereinunder.
Unless otherwise specifically indicated, the term "lower" used in the definition of the formulae in this description means a linear or branched carbon chain having from 1 to 6 carbon atoms. Accordingly, "lower alkyl" is preferably a linear or branched C₁₋₆ alkyl group, including, for example, methyl, ethyl, propyl, isopropyl, butyl, isobutyl, sec-butyl, tert-butyl, pentyl, isopentyl, hexyl, isohexyl. Of those, preferred are the groups having from 1 to 3 carbon atoms; and more preferred are methyl and ethyl.
"Lower alkylene" includes methylene, ethylene, propylene, butylene, and also other branched lower alkylene groups. Preferred are lower alkylene groups having from 1 to 3 carbon atoms; more preferred are methylene and ethylene; and even more preferred is methylene.
"Halogen atom" includes a fluorine atom, a chlorine atom, a bromine atom, an iodine atom. Of those preferred are a fluorine atom, a chlorine atom and a bromine atom.
"Aryl" means a mono- to tri-cyclic aromatic hydrocarbon ring group having from 6 to 14 carbon atoms. Preferably, it includes phenyl, naphthyl, anthryl, phenanthryl. More preferred are phenyl and naphthyl. "-Lower alkylene-aryl" is especially preferably benzyl or phenethyl.
"Heteroaryl" means a mono- to tri-cyclic aromatic hetero ring group having from 6 to 14 carbon atoms. Preferably, it is a 5- or 6-membered monocyclic aromatic hetero ring group; more preferably, pyridyl, pyrimidyl, pyrazyl, thienyl, furyl, oxazolyl, thiazolyl; even more preferably pyridyl. "-Lower alkylene-heteroaryl" is especially preferably picolyl.
"5- or 6-membered carbon ring" means a 5- or 6-membered cycloalkane or cycloalkene, and also benzene.
"5- or 6-membered hetero ring" includes a saturated ring concretely such as pyrrolidine, piperidine, piperazine, morpholine, and a condensed ring such as tetrahydropyridine, furan, thiophene, pyrrole, pyridine, pyrazine, pyrimidine, oxazole, thiazole.
"5- to 7-membered cycloalkane" includes cyclopentane, cyclohexane, cycloheptane.
"5 to 7-membered cycloalkene" includes cyclopentene, cyclohexene, cycloheptene.
"5- to 8-membered cyclic amine optionally having a double bond, optionally having a bridge structure and optionally having a substituent of R⁷ to R¹¹ in the ring" means an unsaturated or saturated 5- to 8-membered cyclic amine ring having a double bond in the ring in the former, or a bicycloamine. Preferably, it is a saturated 5- to 7-membered cyclic amine ring, more preferably pyrrolidine, piperidine, homopiperidine, morpholine, piperazine, even more preferably piperidine. The bicycloamine is preferably quinuclidine, 7-azabicyclo[2,2,1]heptane, 8-azabicyclo[3,2,1]octane, 9-azabicyclo[3,3,1]nonane, 3-azabicyclo[3,2,2]nonane.
The substituent for "5- or 6-membered carbon ring or 5- or 6-membered hetero ring optionally having a substituent" and "aryl optionally having a substituent" includes a halogen atom, a lower alkyl, a lower alkenyl, -O-lower alkyl, -OH, -NH, -NH(lower alkyl), -N(lower alkyl)₂, -NH-CO-(lower alkyl), -N(lower alkyl)-CO-(lower alkyl), -NHCO-(O-lower alkyl), -N(lower alkyl)-CO-(O-lower alkyl), -CN, -NO₂, -CF₃, -lower alkylene-OH, -lower alkylene-halogen atom, -O-lower alkylene-OH, -lower alkylene-O-lower alkyl, -CO-N(lower alkyl)₂, -COOH, -CO-O-lower alkyl, to which, however, the present invention should not be limited.

The compounds of the present invention include mixtures of various isomers such as tautomers and optical isomers, as well as individual isomers isolated from them.
The compounds of the present invention may form acid-addition salts. Depending on the type of the substituent therein, the compounds may form salts with bases. Concretely, the salts include acid-addition salts with a mineral acid such as hydrochloric acid, hydrobromic acid, hydroiodic acid, sulfuric acid, nitric acid, phosphoric acid; an organic acid such as formic acid, acetic acid, propionic acid, oxalic acid, malonic acid, succinic acid, fumaric acid, maleic acid, lactic acid, malic acid, tartaric acid, citric acid, methanesulfonic acid, ethanesulfonic acid; or an acidic amino acid such as aspartic acid, glutamic acid; as well as salts with an inorganic base such as sodium, potassium, magnesium, calcium, aluminium; an organic base such as methylamine, ethylamine, ethanolamine; or a basic amino acid such as lysine, ornithine; and ammonium salts.
Further, the compounds of the present invention include hydrates, pharmaceutically-acceptable various solvates, and crystalline polymorphic substances.
Naturally, the compounds of the present invention are not limited to those described in the Examples given hereinunder, and include all the compounds of the above formula (I) and their pharmaceutically-acceptable salts.

In addition, the compounds of the present invention include prodrugs that are metabolized in living bodies to give the compounds of the above formula (I) or their salts. Groups to form prodrugs of the compounds of the present invention are described in, for example, Prog. Med., 5:2157-2161 (1985) and Development of Medicines (by Hirokawa Publishing, 1990), Vol. 7, Molecular Planning, p. 163-198.

### [Production Methods]

Taking advantage of the characteristics based on the backbone skeleton thereof or on the type of the substituent therein, the compounds of the present invention may be produced according to various known production methods. Depending on the type thereof, the functional group in the starting compounds or intermediates may be suitably protected, or that is, may be modified into a protected group that may be readily converted into the functional group, and this may be technically effective in producing the compounds. After the process, the protective group may be optionally removed, and the intended compounds may be thus obtained. The functional group includes, for example, a hydroxyl group and a carboxyl group. Their protective groups are described, for example, in Greene & Wuts' Protective Groups in Organic Synthesis, 2nd Ed. Depending on the reaction condition, these may be used suitably.
Typical production methods for the compounds of the present invention are described below.

### (Production Methods)

The following schemes 1 to 6 are production methods for cyclic ketones that are the starting compounds for the compounds (I) of the present invention. An indanone (6) that may be a starting compound for the compounds (I) of the present invention may be produced according to the method of the following scheme 1. Specifically, an aldehyde (1) is subjected to Knoevenagel reaction, concretely, the aldehyde (1) is reacted under heat with a malonic acid derivative (2) in a solvent such as pyridine in the presence of a base such as piperidine serving as a catalyst; or it is subjected to Horner-Emmons reaction, concretely, the aldehyde (1) is reacted with a phosphonate (3) in a solvent such as THF in the presence of a base such as NaH, with cooling on ice or with heating. Then, the resulting cinnamic acid derivative (4) is subjected to catalytic reduction, concretely, it is processed with a palladium-carbon catalyst (hereinafter referred to as Pd-C) in a solvent such as EtOH, THF or acetic acid, in a hydrogen atmosphere or in the presence of a hydrogen source such as ammonium formate. In case where the compound has a substituent active to Pd reduction such as a bromine group in R³ to R⁶ therein, a rhodium-carbon catalyst (hereinafter referred to as Rh-C) may be used as the catalyst. Then, the resulting propionic acid derivative (5) is processed in the presence of an acid such as polyphosphoric acid, methanesulfonic acid (hereinafter referred to as MsOH), MsOH-P₂O₅, trifluoromethanesulfonic acid (hereinafter referred to as TfOH) or sulfuric acid, in an inert solvent (as the case may be, the acid may serve also as a solvent), at room temperature or under heat; or an acid chloride of the compound (5) is processed under heat in the presence of a Lewis acid such as AlCl₃, in a solvent such as 1,2-dichloroethane or nitromethane, or in the absence of a solvent to give an indanone (6). (In the scheme, R¹³ represents a hydrogen atom or a lower alkyl group; R¹⁴ and R¹⁵ each represent a lower alkyl group; R¹⁶ is any of R¹³ or R¹⁴.)

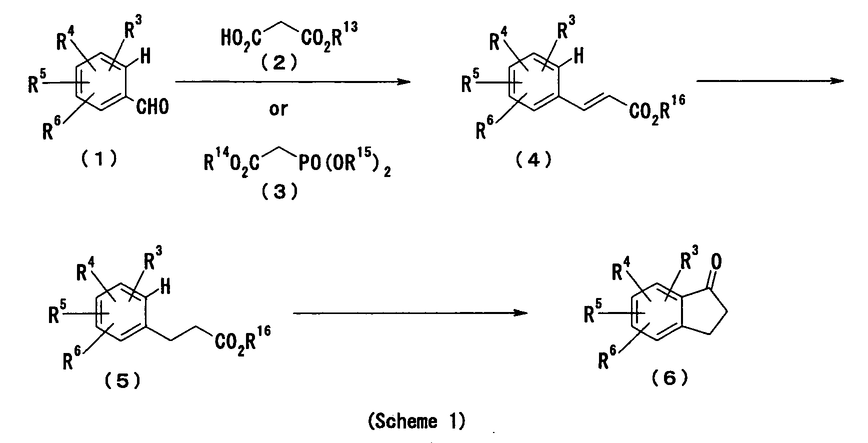

A tetralone (10) that may be a starting compound in producing the compounds (I) of the present invention may be produced according to the method of the following scheme 2. An aldehyde (1) and a cyclopropane derivative (7) are reacted in an inert solvent such as methylene chloride, in the presence of a Lewis acid catalyst such as TiCl₄ with cooling or at room temperature to give a chloride (8), and this is catalytically reduced in a hydrogen atmosphere in the presence of a catalyst such as Pd-C, in a solvent such as EtOH, THF or acetic acid to give a butanoic acid derivative (9). Then, (9) is cyclized in the same manner as that for the cyclization reaction of the scheme 1 to give the tetralone (10).

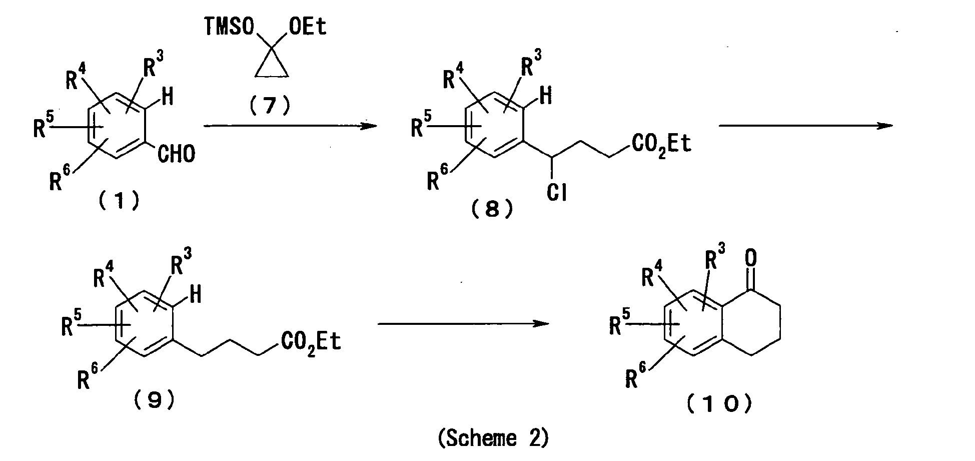

The cyclic ketone may also be produced according to the method of the following scheme 3. Concretely, a bromide (11) is cyclized through directed lithiation with a base such as n-BuLi, lithium or N,N-diisopropylamide (hereinafter referred to as LDA) in an inert solvent such as THF or diethyl ether, with cooling or at room temperature.

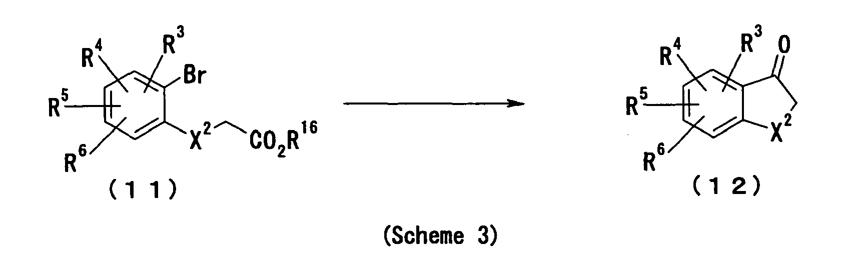

Ketones (15a) and (15b) that are the starting compounds for the compounds (I) of the present invention where R¹ and R² are both lower alkyl groups may be produced according to the method of the following scheme 4. Specifically, when R¹ and R² are the same lower alkyl groups, then an unsubstituted ketone (13) is reacted with at least 2 equivalents of a lower alkyl halide or dialkyl sulfate, in the presence of a base such as NaH, KN(TMS)₂, t-BuOK or KOH and optionally a phase-transfer catalyst such as an ammonium salt, in a solvent such as THF, DMF or DMSO, with cooling or under heat. when R¹ and R² are different lower alkyl groups, then LDA is used as a base and the ketone is stepwise alkylated to give the intended product. (In the scheme, X³ is a halogen atom.)

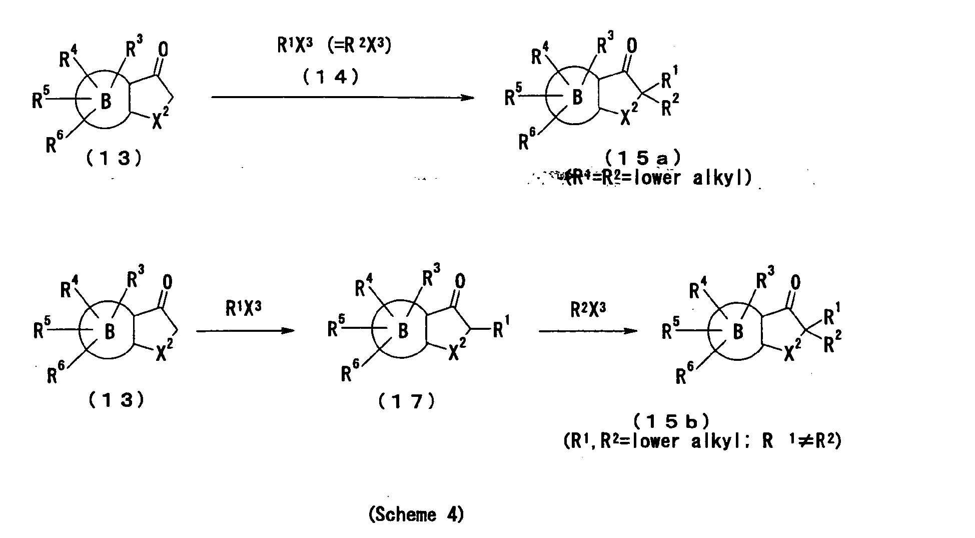

A ketone (15d), which is a starting compound in producing the compounds (I) of the present invention where R¹ and R² are both fluorine atoms, may be produced according to the method of the following scheme 5. Specifically, a ketone (13) is reacted under heat with an N-fluoropyridinium salt (18) in the presence of sulfuric acid and dimethyl sulfate, in an solvent such as MeCN to give the ketone (15a). The compounds where one of R¹ or R² is a lower alkyl group and the other is a fluorine atom may be produced by fluorinating a monoalkyl compound (17) in the same manner as above, as in the scheme 4.

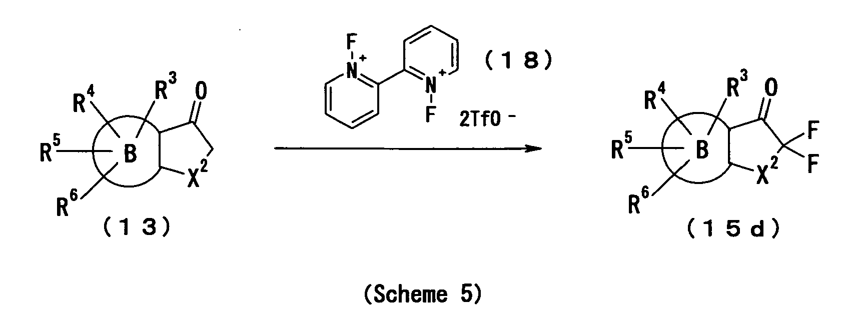

A ketone (15f) may be produced by cyclizing a carboxylic acid derivative (19) previously having R¹ and R², in the same manner as that for the cyclization of schemee 1 to 3, as in the scheme 6. (In the scheme, R¹⁷ represents a hydrogen atom or a bromine atom.)

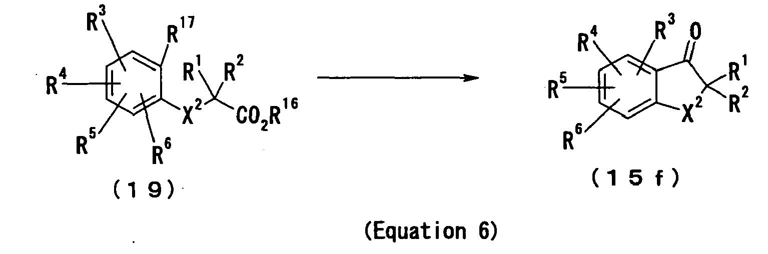

Production methods for the compounds (I) of the present invention are described below.
Of the compounds (I) of the present invention, a compound (Ia) where Y¹ is a hydroxyl group may be produced by reacting a cyclic ketone derivative (15) with a Grignard reagent or an organic lithium reagent (20) prepared from the corresponding halide, in a solvent such as THF, diethyl ether or methylene chloride, with cooling or at room temperature, as in the following scheme 7. (In the scheme, Z¹ is a lithium atom, or a halomagnesium.)

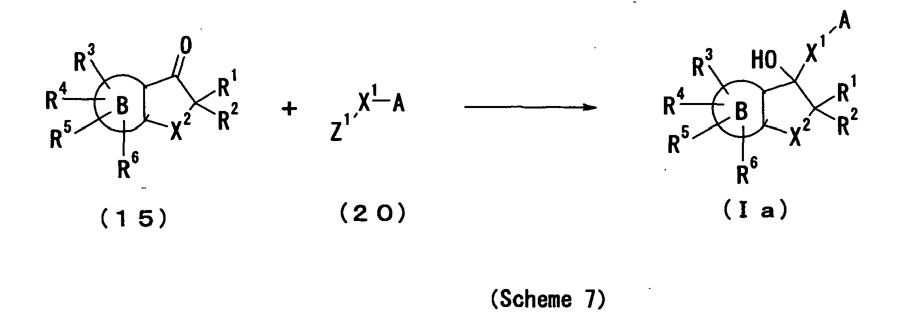

Of the compounds (I) of the present invention, a compound (Ib) where Y¹ is a hydroxyl group and A is N(R²⁰)(R²¹) may be produced by reacting a cyclic ketone derivative (15) with a nucleating reagent such as a Grignard reagent or an organic lithium reagent having a hydroxyl group protected with a suitable protective group, and then converting the protected hydroxyl group into an amino group, as in the following scheme 8. Concretely, a cyclic ketone derivative is reacted with a Grignard reagent or an organic lithium reagent (21) that has a hydroxyl group protected with a suitable protective group such as a group of t-Bu(Me)₂Si or a benzyl group, in a solvent such as THF, diethyl ether or methylene chloride, at a temperature falling between -78°C and room temperature to give an alcohol (22). When the protective group is a silyl group, then the product is processed with Bu₄NF, KF or CsF in a solvent such as THF or MeOH with cooling or under heat; and when the protective group is a benzyl group, the product is catalytically reduced for deprotection to give a dialcohol (23), and thereafter this is processed with MsCl or TsCl in the presence of a base such as Et₃N in a solvent such as methylene chloride to give a sulfonyl compound (24), and then this is further reacted with an amine (25) in the presence of a base such as (i-Pr)₂EtN, Et₃N or K₂CO₃ in a solvent such as MeCN or THF, or making an excessive amount of the amine (25) itself serve as a base to give an amine (Ib). (In the scheme, G is an ordinary protective group for a hydroxyl group, such as Me₃Si, t-Bu(Me)₂Si, Bu₃Si or benzyl; R²⁰ and R²¹ are the same or different, each representing a hydrogen atom, or an optionally-substituted lower alkyl group; regarding N(R²⁰)R²¹, R²⁰ and R²¹ may bond to each other to form a 5- to 8-membered cyclic amine optionally having a substituent of R⁸ to R¹¹, ring optionally having a double bond and optionally having a bridge structure in the ring.) 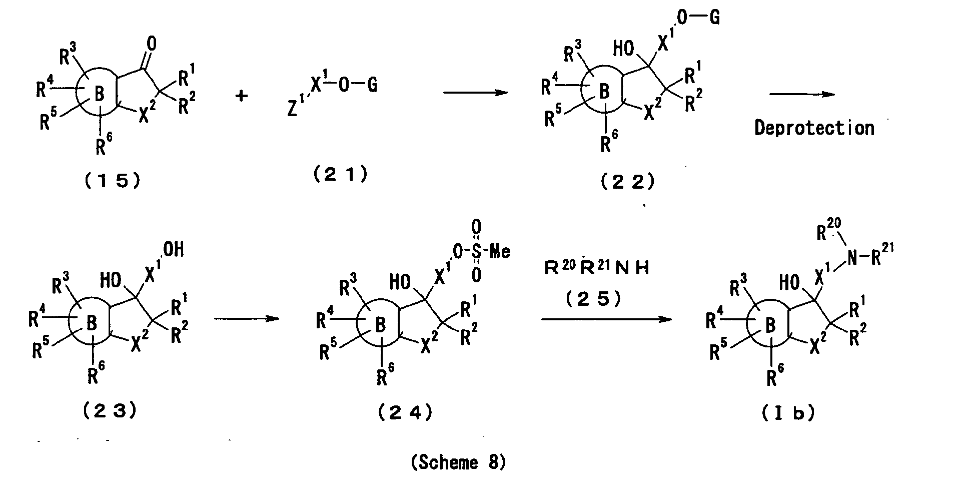 Of the compounds (I) of the present invention, a compound (Ic) where X¹-A is represented by the structural scheme mentioned below may be produced by reducing a compound (27) produced from a ketone (15) and an amide (26), as in the scheme 9. Concretely, a ketone (15) is reacted with an amide α-lithiate (26) prepared with LDA in a solvent such as THF or diethyl ether, at a temperature falling between -78°C and room temperature to give (27), and the amide bond moiety in the resulting compound is reduced with LiAlH₄ or BH₃ in a solvent such as THF with cooling or under heat. (In the scheme, R²² to R²⁵ each represent a hydrogen atom or an optionally-substituted lower alkyl group; and R²² to R²⁵ may bond to each other to form a lower alkylene optionally having a hetero atom in the chain thereof.)

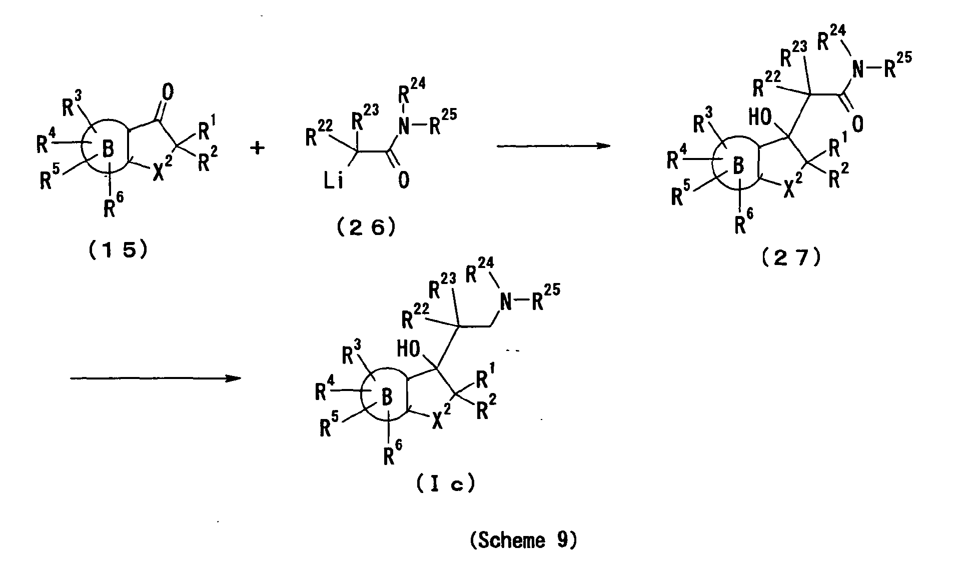

Of the compounds (I) of the present invention, a compound (Id) where Y¹ is a hydroxyl group and X¹-A is a group of the following structural formula may be produced according to the method of the following scheme 10. Concretely, a ketone (15) is reacted with an α-lithiate ester (28) prepared from the corresponding ester with LDA, at a temperature falling between -78°C and room temperature to give an ester (29), then this is processed with a reducing agent such as LiAlH₄, iBu₂AlH or LiBH₄ in a solvent such as THF or MeOH with cooling on ice or at room temperature to give an alcohol (30); then in the same manner as in the above scheme 8, the hydroxyl group in the resulting product is converted into an amino group to give the amine (Id). (In the scheme, R²⁶ and R²⁷ are the same or different, each representing a hydrogen atom or a lower alkyl group; and R²⁶ and R²⁷ may bond to each other to from a lower alkylene optionally having a hetero atom in the chain.)

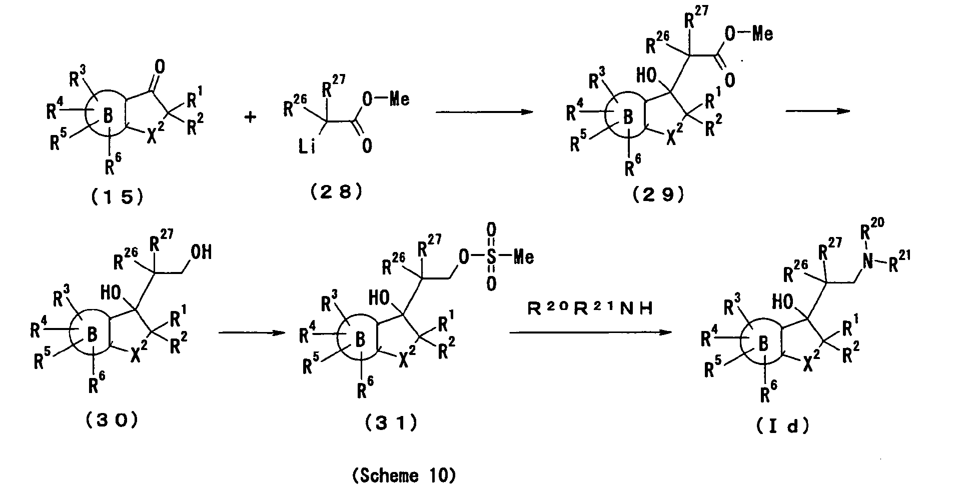

Of the compounds (I) of the present invention, a compound (Ie) where Y¹ is a hydroxyl group and X¹-A is a group of the following structural formula may be produced according to the method of the following scheme 11. Specifically, a ketone (15) is reacted with a dilithio-oxime (32) prepared from an oxime and n-BuLi, in a solvent such as THF or diethyl ether, at a temperature falling between -78°C and room temperature to give an oxime (33); then, this is reacted with P₂O₅ in a solvent such as chloroform or methylene chloride with cooling, or is reacted with a sulfonylating agent such as MsCl to give a dihydroisoxazole (34). Then, this is reacted with an alkyllithium reagent (35) in the presence of BF₃·Et₂O or CeCl₃ in a solvent such as THF, methylene chloride or toluene at a temperature falling between -78°C and room temperature to give an isoxazolidine (36); and this is reduced with LiA1H₄ in THF at room temperature, or reduced in a mode of ordinary catalytic reduction to give the amine (1e). (In the scheme, R²⁸ and R²⁹ each represent a lower alkyl group.)

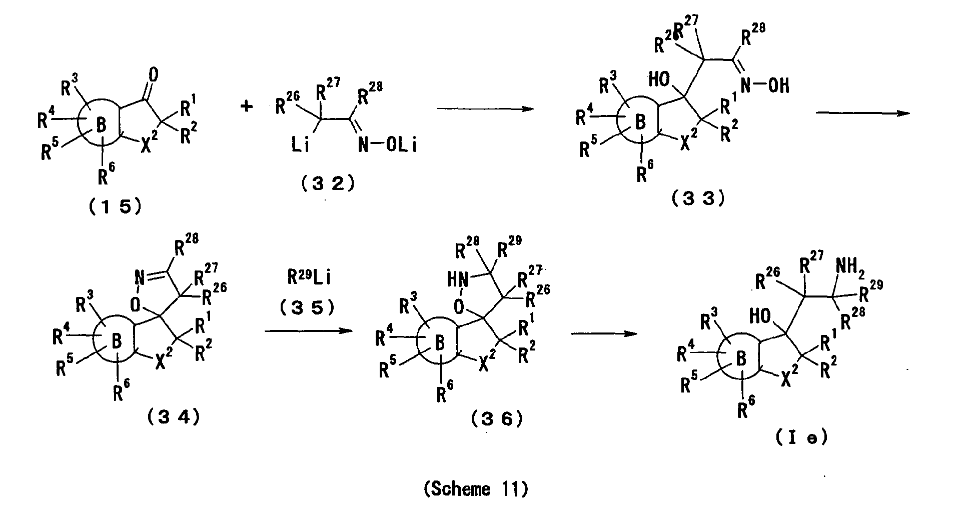

Of the compounds (I) of the present invention, a compound (If) or (Ig) where the ring A is a piperidine ring, the ring B is a benzene ring, Y¹ is a hydroxyl group, X¹ is a bond, and X² is a methylene group, or a compound (Ih) where the ring A is a tetrahydropyridine ring may be produced by reducing the corresponding pyridine compound as in the following scheme 12. Concretely, a benzyl bromide (37) and an acylpyridine (38) are reacted in the presence of a base such as LDA in a solvent such as THF or diethyl ether with cooling or at room temperature to give a compound (39); this is cyclized with a base such as LDA in a solvent such as THF or diethyl ether with cooling or at room temperature, or is cyclized under heat in the presence of a base such as Na₂CO₃ with a Pd catalyst such as Pd(OAc)₂, a ligand such s tricyclohexyl phosphine, and an alcohol such as n-hexanol, in a solvent such as DMF to give a cyclized compound (40); and this is reduced with Rh-C in a hydrogen atmosphere under normal pressure or increased pressure to give a piperidine (If). (40) may be reacted with an alkyl halide in a solvent or in the absence of a solvent at room temperature or under heat to give an ammonium salt (41); then this is processed with a reducing agent such as NaBH₄ in a solvent such as MeOH with cooling on ice or at room temperature to give a tetrahydropyridine (Ih). Further, (Ih) may be processed in a hydrogen atmosphere in the presence of Pd-C in a solvent such as EtOH at room temperature; or (41) may be subjected to direct reduction with platinum oxide in a solvent such as EtOH in a hydrogen atmosphere at normal pressure or increased pressure to give a piperidine (Ig).

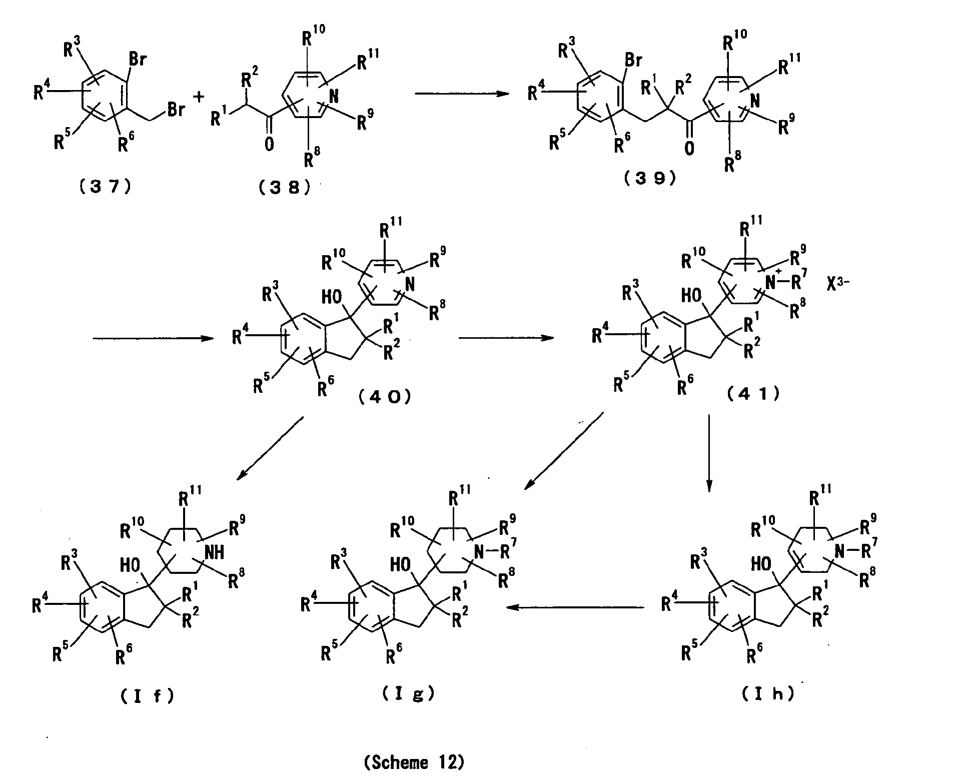

Of the compounds (I) of the present invention, a compound (Ii), (Ij) or (Ik) where the ring A is an unsubstituted piperidine ring or a tetrahydropyridine ring, X¹ is a methylene group optionally substituted with a lower alkyl group, and Y¹ is a hydroxyl group may be produced according to the method of the following scheme 13. Specifically, an alkylsubstituted pyridine (42) is metallized with a base such as n-BuLi or KN(i-Pr)₂; then this is reacted with a ketone (15) in a solvent such as THF with cooling or at room temperature to give a pyridine (43); this is reduced in various methods, for example, as in the scheme 12 to give the piperidine (Ii) or (Ij) or the tetrahydropyridine (Ik). (In the scheme, R¹⁷ and R¹⁸ are the same or different, each representing a hydrogen atom or a lower alkyl group; and R¹⁷ and R¹⁸ may form a lower alkylene optionally having a hetero atom in the chain thereof.).

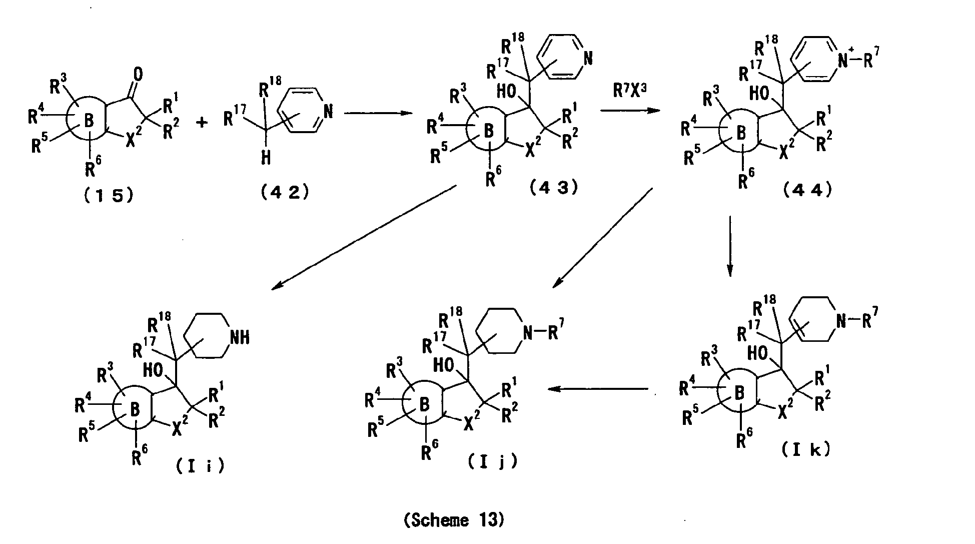

Of the compounds (I) of the present invention, a compound (Im), (In), (Io) or (Ip) where the ring B is a benzene ring, X² is -CO-, -CH(OH)-, -CH(C1)- or -CH₂-, and Y¹ is a hydroxyl group may be produced according to the method of the following scheme 14. Specifically, a ketone (45) and a hydrazide (46) are reacted under heat in a solvent such as EtOH, i-PrOH or acetic acid, optionally in the presence of an acid catalyst such as acetic acid or sulfuric acid to give a hydrazide (47). This is reacted with PhI(OAc)₂ or Pb(OAc)₄ in an inert solvent such as 1,2-dichloroethane, THF or toluene at room temperature or under heat to give a diketone (48), and this is then cyclized with a base such as t-BuOK, NaOEt, DBU, KN(TMS)₂ or LDA in a solvent such as THF, 1,2-dichloroethane, toluene or EtOH at a temperature falling between -78°C and an elevated temperature to give a cyclic ketone (Im). This is reduced with a reducing agent such as LiA1H₄, NaBH₄ or (i-Bu)₂AlH in a solvent such as THF, MeOH or EtOH at a temperature falling between -78°C and room temperature to give an alcohol (In). (In) may be halogenated with a halogenating agent such as SOCl₂ in a solvent such as MeCN with cooling or at room temperature to give a halide (Io). (Io) may be reduced under an acidic condition with an acetic acid solvent, using Zn (powder) under heat, or is catalytically reduced in a hydrogen atmosphere in the presence of a catalyst such as Pd-C or Raney-Ni to give a reduced product (Ip).

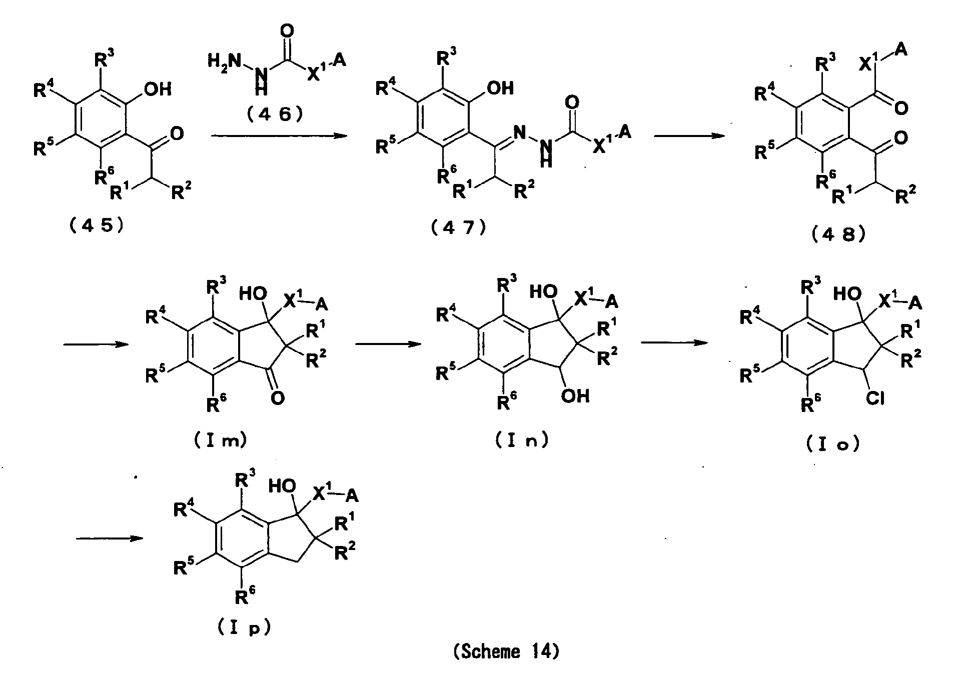

Of the compounds (I) of the present invention, a compound (Iq) to (Is) where Y¹ forms an oxygen-containing 5- to 7-membered ring along with R³, or any one of R¹ or R², or the branch on -X¹-A may be produced according to the method of the following scheme 15. Specifically, a diol (49), (50) or (51) is processed in the presence of concentrated sulfuric acid, hydrochloric acid or MsOH in a solvent such as MeOH, 1,4-dioxane or water, at room temperature or under heat to give the intended compound. (In the scheme, X⁴ represents a lower alkylene, -O-lower alkylene, or -N(R³⁰)-lower alkylene; X⁵ represents a lower alkylene; Q represents a hydroxyl group-having -X¹-A as a form of -Q-OH.)

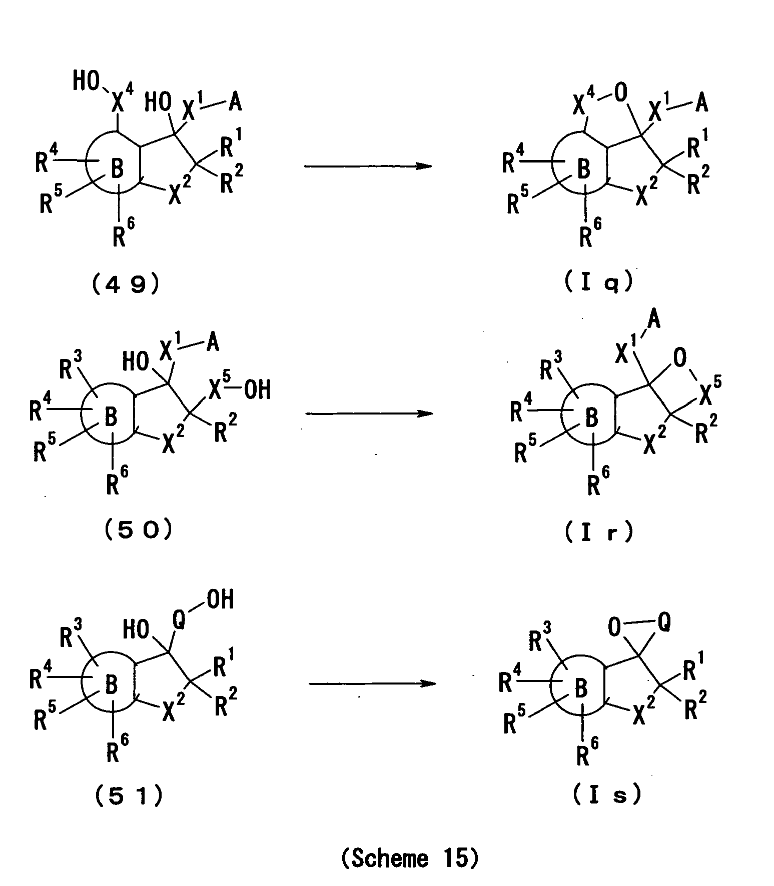

The compounds (I) of the present invention may be subjected to substituent modification known to anyone skilled in the art to give compounds having a desired substituent. Typical reactions for it are described below.
Of the compounds (I) of the present invention, a compound where the substituent R⁷ on the nitrogen atom of the ring A is a hydrogen atom or a lower alkyl group may be produced by reacting a compound (I) of the present invention where R⁷ is a methyl group or a benzyl group with a chloroformate in the presence of a base such as Et₃N or in the absence of a base, in a solvent such as toluene or methylene chloride at room temperature or under heat to give a carbamate, and then processing it in the presence of a base such as NaOH or KOH in a solvent such as water or ethanol under heat to give a compound where R⁷ is a hydrogen atom. In case where 1-chloroethyl chloroformate is used as the reactant, the resulting carbamate may be heated in methanol for deprotection. The compound where R⁷ is a hydrogen atom may be reacted with an aldehyde for reductive amination in a solvent such as methylene chloride or 1,2-dichloroethane in the presence of an acid such as acetic acid or a Lewis acid catalyst, using a reducing agent such as NaB(OAc)₃H or NaB(CN)H₃ with cooling or at room temperature; or it is alkylated with an alkyl halide in the presence of a base such as K₂CO₃ in a solvent such as MeCN at room temperature or under heat to give a compound where R⁷ is an alkyl group.

Of the compounds (I) of the present invention, a compound where R³ is a cyano group and the ring B is an aromatic ring may be produced by processing the corresponding compound where R³ is a bromine atom with Zn(CN)₂ in the presence of a catalyst such as Pd(PPh₃)₄ in a solvent such as DMF or N-methylpiperidone under heat.
Of the compounds (1) of the present invention, a compound where any of R³ to R⁶ is an optionally-substituted aryl, lower alkenyl or lower alkynyl group may be produced through Suzuki reaction, or that is, by reacting the corresponding compound where any of R³ to R⁶ is a bromine atom or an iodine atom, with an arylboronic acid, an alkenylboronic acid, an alkynylboronic acid or their boronate ester in the presence of a catalyst such as Pd(PPh₃)₄, PdCl₂(dppf) or Pd₂(dba)₃ along with a base such as K₂CO₃, Na₂CO₃, KOH, CsF or NaOEt in a solvent such as DMF, N-methylpiperidone, DME or toluene or a mixed solvent thereof with water, under heat.
Of the compounds (I) of the present invention, a compound where X¹ is a methylene group and A is NH₂ may be produced by reducing the corresponding cyanohydrin with LiAlH₄ in a solvent such as THF. The starting compound, cyanohydrin may be produced by reacting the corresponding ketone with (TMS)CN in the presence of ZnI₂ in a solvent such as methylene chloride or 1,2-dichloroethane under heat, or by reacting it with KCN or NaCN under an acidic condition with acetic acid or sulfuric acid.
Of the compounds (I) of the present invention, a compound where Y¹ is an azido group may be produced by reacting a compound (I) where Y¹ is a hydroxyl group with NaN₃ in the presence of an acid such as trifluoroacetic acid in a solvent such as chloroform, or using the acid itself as a solvent, with cooling or under heat. This may be reduced in a hydrogen atmosphere in the presence of Pd-C in a solvent such as EtOH, or reduced with a reducing agent such as LiA1H₄ in a solvent such as THF under heat to give a compound where Y¹ is an amino group.
Of the compounds (I) of the present invention, a compound where the ring B is an aromatic ring and any of R³ to R⁶ is a nitro group may be produced by nitrating the corresponding compound where any of R³ to R⁶ is a hydrogen atom under an ordinary nitrating condition, concretely with fuming sulfuric acid in acetic anhydride with cooling, or with concentrated nitric acid in an acetic acid solvent with cooling or at room temperature, or with NO₂BF₄ in a solvent such as sulforane or THF at low temperature or room temperature. This may be catalytically reduced with Pd-C to give the corresponding aniline.
Of the compounds (I) of the present invention, a compound where Y¹ is a lower alkoxy group may be produced by processing the corresponding compound where Y¹ is a hydroxyl group in a lower alcohol solvent in the presence of an acid catalyst such as camphorsulfonic acid with cooling or under heat.

The deprotection may be attained in the presence of a suitable base in a suitable solvent. Examples of the base are NaOH, KOH, NaOMe, NaOEt. Examples of the solvent are ethers such as tetrahydrofuran, dioxane, diglyme; alcohols such as MeOH, EtOH, i-PrOH; MeCN, water; and their mixtures. Depending on the type of the reaction substrate and the reaction condition, the solvent may be suitably selected. The reaction temperature may vary depending on the type of the starting compound and the reaction condition, generally covering from cooling to refluxing, preferably from about 0°C to about 100°C.
The deprotection may also be attained in the presence of a metal catalyst such as Pd-C, Pd(OH)₂ or PtO₂ in a suitable solvent in a hydrogen atmosphere, but may be attained in the presence of a suitable Lewis acid in a suitable solvent. Examples of the Lewis acid are BCl₃, BBr₃, AlCl₃, and examples of the solvent are ethers such as tetrahydrofuran, dioxane; esters such as ethyl acetate; alcohols such as MeOH, EtOH; MeCN; and their mixtures. Depending on the type of the reaction substrate and the reaction condition, the solvent may be suitably selected. The reaction temperature may vary depending on the type of the starting compound and the reaction condition, generally covering from cooling to refluxing, preferably from about -80°C to about 30°C.
Thus produced, the compounds (I) of the present invention may be isolated as free compounds or as their pharmaceutically-acceptable salts. Salts of the compounds (I) of the present invention may be produced by processing free bases of the compounds (I) of the present invention for ordinary salt formation.
The compounds (I) of the present invention or their pharmaceutically-acceptable salts may be isolated and purified as their hydrates, solvates or crystalline polymorphic substances. The isolation and purification may be attained through ordinary chemical treatment of extraction, concentration, evaporation, crystallization, filtration, recrystallization, chromatography.
Various isomers may be isolated by selecting suitable starting compounds, or by separating them based on the difference between the isomers in the physical or chemical properties thereof. For example, optical isomers may be led into stereochemically-pure isomers by selecting suitable starting compounds or by racemic resolution of racemic compounds (for example, leading them into diastereomer salts with ordinary optically-active acid for optical resolution).

The NMDA receptor antagonistic activity of the compounds of the present invention was confirmed by the following test methods.

### 1. MK-801 binding test:

### 1) Preparation of Specimens of Rat Meninges:

The whole brain was taken out from 10-week-age SD rats (30 heads, by Nippon SLC), and the cerebellum was removed from it. A 0.32 M sucrose solution was added to the part containing the cerebrum, cut in a mixer, and homogenized with a Teflon^{™} homogenizer. This was centrifuged at 2800 rpm and 4°C for 15 minutes, and the resulting supernatant was again centrifuged at 15000 g and 4°C for 20 minutes. The pellets were suspended in 50 mM Tris-HCL (pH 7.5) containing 0.08% Triton X-100, and kept statically on ice for 30 minutes, then centrifuged at 15000 g and 4°C for 20 minutes. The pellets were suspended in 50 mM Tris-HCl (pH 7.5) added thereto, and centrifuged at 15000 g and 4°C for 20 minutes. 50 mM Tris-HCl (pH 7.5) was again added to the pellets, and centrifuged in the same manner as before. The pellets were suspended in 20 ml of 50 mM Tris-HCl (pH 7.5) added thereto, and homogenized with a Teflon^{™} homogenizer. The membrane specimen was divided into small tubes and stored in a deep freezer (-80°C). Before use, this was washed twice with 5 mM Tris-HCl (pH 7.5) of five times that of the membrane specimen. Its concentration was controlled at 1 mg protein/ml with 5 mM Tris-HCl (pH 7.5) added to it, and this was used for assay.

### 2))[³H]MK-801-binding Assay:

50 µl of the rat membrane specimen (1 mg protein/ml) was added to a solution of a test compound dissolved in 1 µl of DMSO. Then, 50 µl of a ligand solution (600 nM glutamate, 600 nM glycine, 8 nM [³H] MK-801 (by Perkin-Elmer) was added to it and well stirred, and reacted at room temperature for 45 minutes. Using Uni Filter Plate GF/B 96 (by Perkin-Elmer) previously coated with 0.2% polyethyleneimine, the membrane specimen was collected, and the filter was well washed with 5 mM Tris-HCl (pH 7.5). 30 µl of Microscinti 20 (by Perkin-Elmer) was added to the filter, and the radioactivity trapped by the filter was determined by a microplate scintillation counter (TopCount^{™}, by Beckman). Based on the MK-801 (final 1 µM) inhibition, 100%, of a control case of DMSO alone, the concentration of the compound for 50% inhibition, IC₅₀ was computed. The [³H]MK-801 binding affinity for the rat membrane specimen was obtained through Scatchard analysis and Kd = 1.6 nM. The Ki value of the compound was computed according to the calculation equation: Ki = IC₅₀/(1 + radioligand concentration (4 nM) in assay)/Kd value (1.6 nM)).
As a result, the compounds of the present invention exhibited good NMDA receptor affinity. The Ki value of the NMDA receptor affinity of some typical compounds of the present invention is shown in Table 1 below.

**Table 1**

| Compound | Ki(µM) | Compound | Ki (µM) |
|---|---|---|---|
| Example 1 | 1.4 | Example 85 | 6.2 |
| Example 6 | 0.88 | Example 91 | 1.1 |
| Example 9 | 0.93 | Example 98 | 8.4 |
| Example 15 | 0.86 | Example 101 | 2.1 |
| Example 28 | 1.0 | Example 108 | 5.6 |
| Example 32 | 0.44 | Example 113 | 0.9 |
| Example 34 | 1.8 | Example 131 | 4.8 |
| Example 36 | 1.9 | Example 139 | 5.0 |
| Example 44 | 0.93 | Example 143 | 0.7 |
| Example 53 | 4.1 | Example 145 | 8.4 |

### 2. Intracellular Calcium Concentration Determination Test by FLIPR (Fluorometric Imaging Plate Reader):

### 1) Preparation of Rat First-Generation Neurocytes:

Anesthetized with ether, Wistar rats (by Nippon SLC) of pregnancy 19 days was let die from loss of blood by breast incision. The abdomen was cut open, and the womb was taken out, and the fetus was taken out of it. The whole brain was taken out, then the hemicerebrum was isolated in Neurobasal medium (Glu, Asp-free) (by Gibco), and the meninx was removed. The hemicerebrum was recovered by centrifugation, and suspended in a cell-dispersing solution (0.36 mg/ml papain, 150U/ml DNase 1,0.02% L-cysteine monohydrochloride monohydrate, 0.02% bovine serum albumin, 0.5% glucose, Ca²⁺ Mg²⁺-free PBS), and processed at 37°C for 15 minutes. This was centrifuged at 400 g for 5 minutes, and the supernatant was removed by suction. This was suspended in a neurocyte culture medium (by Sumitomo Bakelite), and the cell masses were removed by filtration. The number of the living cells was counted, and 100,000 cells/well were incubated on a 96-well plate (Biocoat PDL96W black/clear, by Nippon Becton Dickinson) at 37°C in 5% CO₂.

### 2) Intracellular Calcium Concentration Determination by FLIPR (Fluorometric Imaging Plate Reader):

The culture of rat first-generation neurocytes (DIV7-9) was removed by suction, and the cells were washed once with an assay buffer (Hank's Balanced Salt Solution (Ca²⁺, Mg²⁺-free), 20 mM Hepes-NaOH (pH 7.4), 1 mM CaCl₂). 100 µl of the assay buffer containing Fluo3 (by Dojin Chemical) was added thereto, and incubated for 1 hour (37°C, 5% CO₂). The cells were washed three times with 100 µl of the assay buffer, and then a test compound solution dissolved in 1 µl of DMSO, and 100 µl of the assay buffer containing 2.5 µM (final concentration) tetrodotoxin were added to it and incubated for 30 minutes (37°C, 5% CO₂). The fluorescent intensity was measured at intervals of 2 seconds. Ten seconds after the measurement start, 50 µl of a ligand solution (Hank's Balanced Salt Solution (Ca²⁺, Mg²⁺-free), 20 mM Hepes-NaOH (pH 7.4), 1 mM CaCl₂, 9 µM NMDA, 30 µM glycine) containing the test compound solution dissolved in 0.5 µl of DMSO was added to it, and the fluorescent intensity of the system was measured for 120 seconds from the start of the measurement. The data measured for 120 seconds (60 times in total) were averaged. Based on the 10 µM MK-801 inhibition, 100%, of a control case of DMSO alone, the concentration of the compound for 50% inhibition, IC₅₀ was computed.
As a result, the compounds of the present invention exhibited good NMDA receptor antagonizing effect.

The pharmaceutical composition that contains, as the active ingredient thereof, one or more of the compounds of the present invention and their pharmaceutically-acceptable salts may be formulated, optionally along with carriers and vehicles for ordinary pharmaceutical application and other additives, as tablets, powders, infinitesimal grains, granules, capsules, pills, liquids, injections, suppositories, ointments, fomentations, and is administered to patients orally or non-orally.
The clinical dose to human of the compound of the present invention may be suitably determined, depending on the condition, the body weight, the age and the sex of the patient to whom the compound is applied. In general, it may be from 0.1 to 500 mg/adult/day for oral administration, and from 0.01 to 100 mg/adult/day for non-oral administration, and this may be administered all at once or in several times. The dose may vary under various conditions, and as the case may be, it may be smaller than the above-mentioned dose range.
The solid composition for oral administration of the compound of the present invention may be tablets, powders or granules. In the solid composition, one or more active substances may be mixed with at least one inert diluent, such as lactose, mannitol, glucose, hydroxypropyl cellulose, microcrystalline cellulose, starch, polyvinylpyrrolidone, magnesium metasilicate aluminate. According to an ordinary manner, the composition may contain any other additive than such an inert diluent, for example, lubricant such as magnesium silicate, disintegrator such as calcium cellulose glycolate, stabilizer such as lactose, solubilizer or dissolution promoter such as glutamic acid, aspartic acid. The tablets and pills may be coated with sugar or with gastric-coating or enteric-coating film.

The liquid composition for oral administration includes pharmaceutically-acceptable emulsion, solution, suspension, syrup and elixir, and contains an ordinary inert diluent such as pure water, ethyl alcohol. The composition may contain any other additive than such an inert diluent, for example, auxiliary agent such as solubilizer, dissolution promoter, wetting agent, suspending agent, as well as sweetener, flavoring, fragrance, and preservative. The injection for non-oral administration includes germ-free water-base or waterless solution, suspension and emulsion. The diluent for water-base solution and suspension include, for example, distilled water for injection and physiological saline water. The diluent for the waterless solution and suspension includes, for example, propylene glycol, polyethylene glycol, vegetable oil such as olive oil, alcohols such as ethyl alcohols, Polysorbate 80 (trade name).
The composition may further contain any other additive such as isotonizer, preservative, wetting agent, emulsifier, dispersant, stabilizer, solubilizer, dissolution promoter. These may be sterilized by filtration through a bacteria-trapping filter, or by addition of germicide, or through irradiation with light. As the case may be, a germ-free solid composition may be prepared, and it may be dissolved in germ-free water or germ-free solvent for injection to give the intended liquid composition before use.

### EXAMPLES

The compounds of the present invention are described with reference to the following Examples. The starting compounds for the compounds of the present invention include new compounds, and their production is illustrated as Reference Examples hereinunder.

### Reference Example 1:

### 3-(3-Bromo-4-fluorophenyl)propionic acid:

Piperidine (1 ml) was added to a pyridine (250 ml) solution of 3-bromo-4-fluorobenzaldehyde (20 g) and malonic acid (51 g), followed by heating under reflux for 3 hours. After completion of the reaction, the solvent was evaporated under reduced pressure, water was added, and this was neutralized with 1 N hydrochloric acid added thereto with stirring. The precipitated matter was collected by filtration, and the resulting (2E)-3-(3-bromo-4-fluorophenyl)acrylic acid was dissolved in THF (200 ml), followed by stirring with 5% Rh-C (3 g) in a hydrogen atmosphere at room temperature for 12 hours. The insoluble matter was removed by filtration, and the solvent was evaporated under reduced pressure to obtain the compound (15 g) of Reference Example 1.

In Reference Example 2, the compound shown in Table 2 was produced in the same manner as in Reference Example 1.

### Reference Example 3:

### 3-(2-Fluoro-5-methylphenyl)propionic acid:

Piperidine (0.3 ml) was added to a pyridine (50 ml) solution of 2-fluoro-5-methylbenzaldehyde (4.2 g) and malonic acid (16 g), followed by heating under reflux for 3 hours. After completion of the reaction, the solvent was evaporated under reduced pressure, water was added, and this was neutralized with 1 N hydrochloric acid added thereto with stirring. The precipitated matter was collected by filtration, and the resulting (2E)-3-(2-fluoro-5-methylphenyl)acrylic acid was dissolved in a mixed solvent (70 ml) of THF and EtOH, followed by stirring with 10% Pd-C (0.5 g) in a hydrogen atmosphere at room temperature for 12 hours. The insoluble matter was removed by filtration, and the solvent was evaporated under reduced pressure to obtain the compound (5.1 g) of Reference Example 3.

In Reference Examples 4 to 7, the compounds shown in Table 2 were produced in the same manner as in Reference Example 3.

### Reference Example 8:

### 3-(4-Bromo-2-fluoro-5-methylphenyl)propionic acid:

The compound (3.0 g) of Reference Example 3 was dissolved in a mixed solvent of trifluoroacetic acid (15 ml) and concentrated sulfuric acid (3 ml), and at room temperature, N-bromosuccinimide (hereinafter referred to as NBS) (3.5 g) was added thereto gradually, followed by stirring at that temperature for 1 hour. The reaction solution was poured into ice-water, followed by extraction with chloroform and drying over anhydrous sodium sulfate. The solvent was evaporated under reduced pressure to obtain the compound (3.7 g) of Reference Example 8.
In Reference Examples 9 to 12, the compounds shown in Table 2 were produced in the same manner as in Reference Example 8.

### Reference Example 13:

### Methyl 3-(3,4,5-trifluorophenyl)-2,2-dimethylpropionate:

At -20°C, a 1.6 M n-BuLi/hexane solution (100 ml) was added to a THF (200 ml) solution of i-Pr₂NH (25 ml), followed by stirring at that temperature for 30 minutes. This was cooled to -78°C, then methyl 3-(3,4,5-trifluorophenyl)propionate (24 g) was added thereto, followed by further stirring for 1 hour. Then, MeI (10 ml) was added, followed by further stirring for 1 hour. The reaction solution was heated to 0°C, then an aqueous saturated ammonium chloride solution was added, followed by extraction with diethyl ether, and then washing with 1 N hydrochloric acid, an aqueous saturated sodium hydrogencarbonate solution, water and saturated brine. After drying over anhydrous sodium sulfate, the solvent was evaporated under reduced pressure to obtain methyl 3-(3,4,5-trifluorophenyl)-2-methylpropionate.
Next, at -78°C, methyl 3-(3,4,5-trifluorophenyl)-2-methylpropionate produced in the above was added to LAD that had been prepared similarly from i-Pr₂NH (25 ml), followed by stirring at that temperature for 1 hour. Then, MeI (10 ml) was added, followed by furtherstirring for 1 hour. The reaction solution was heated to 0°C, an aqueous saturated ammonium chloride solution was added, followed by extraction with diethyl ether. This was washed with 1 N hydrochloric acid, an aqueous saturated sodium hydrogencarbonate solution, water and saturated brine, then dried over anhydrous sodium sulfate, and the solvent was evaporated under reduced pressure to obtain the compound (21 g) of Reference Example 13.

### Reference Example 14:

### 6,7-Dichloroindan-1-one:

3-(3,4-Dichlorophenyl)propionic acid (500 mg) was stirred in TfOH (1.5 ml) in an argon atmosphere at 100°C for 24 hours. The reaction solution was poured into ice-water, extracted with diethyl ether, washed with water, an aqueous saturated sodium hydrogencarbonate solution and saturated brine, then dried over anhydrous sodium sulfate. Then, the solvent was evaporated under reduced pressure. The residue was purified by silica gel column chromatography (eluent: n-hexane/ethyl acetate = 5/1) to obtain 5,6-dichloroindan-1-one (115 mg) and the compound (44 mg) of Reference Example 14.
In Reference Examples 15 to 22, the compounds shown in Tables 2 and 3 were produced in the same manner as in Reference Example 14.

### Reference Example 23:

### 4-Bromo-6-fluoro-7-methoxy-1-indanone:

The compound (20 g) of Reference Example 9 was dissolved in TfOH (200 ml), followed by stirring in an argon atmosphere at 50°C for 2 hours. The reaction solution was poured into ice-water, and with stirring and cooling on ice, this was neutralized with an aqueous saturated ammonia. The precipitated matter was collected by filtration, washed with water, and dried under reduced pressure to obtain the compound (13 g) of Reference Example 23 as a brown solid.
In Reference Examples 24 to 25, the compounds shown in Tables 2 and 3 were produced in the same manner as in Reference Example 14.
In Reference Example 26, the compound shown in Table 3 was obtained from the compound obtained in Reference Example 13 and shown in Table 2, in the same manner as in Reference Example 14.

### Reference Example 27:

### 6-Fluoro-2,2-dimethylindan-1-one:

MeI (0.354 ml) and 55% oily NaH (248 mg) were added to a THF (10 ml) solution of 6-fluoroindan-1-one (388 mg) in an argon atmosphere at room temperature, followed by stirring at that temperature for 30 minutes. An aqueous saturated ammonium chloride solution was added to the reaction liquid, followed by extraction with diethyl ether, washing with saturated brine, then drying over anhydrous sodium sulfate. Then, the solvent was evaporated under reduced pressure. The residue was purified by silica gel column chromatography (eluent: n-hexane/ethyl acetate =10/1) to obtain the compound (241 mg) of Reference Example 27.
In Reference Examples 28 to 55, the compounds shown in Tables 3 to 5 were produced in the same manner as in Reference Example 27.

### Reference Example 56 and Reference Example 57:

### 7-Chloro-6-fluoro-2,2-dimethylindan-1-one, and 5-Chloro-6-fluoro-2,2-dimethylindan-1-one:

3-(3-Chloro-4-fluorophenyl)propionic acid (9.8 g) was dissolved in TFOH (40 ml), followed by stirring in an argon atmosphere at 100°C for 24 hours. The reaction solution was poured into ice-water, extracted with diethyl ether, and washed with saturated sodium hydrogencarbonate, and saturated brine. Then, the solvent was evaporated under reduced pressure. The residue was purified by silica gel column chromatography (eluent: n-hexane/ethyl acetate = 10/1 to 3/1) to obtain a mixture (7.2 g) of 5-chloro-6-fluoroindan-1-one and 7-chloro-6-fluoroindan-1-one. Then, this was dissolved in THF (200 ml), MeI (6.1 ml) was added thereto, and with stirring at room temperature, 55% oily NaH (4.3 g) was added thereto gradually with taking 30 minutes. After the heat generation stopped, an aqueous saturated ammonium chloride solution was added to the reaction solution, followed by extraction with diethyl ether, and washing with saturated brine. This was dried over anhydrous magnesium sulfate, the solvent was evaporated under reduced pressure, and the residue was purified by silica gel column chromatography (eluent: n-hexane/ethyl acetate = 30/1 to 10/1) to obtain the compound (0.85 g) of Reference Example 56 and the compound (5.9 g) of Reference Example 57.
In Reference Examples 58 to 61, the compounds shown in Table 5 were produced in the same manner as in Reference Examples 56 and 57.

### Reference Example 62:

### 4,6,7-Tribromo-2,2-dimethylindan-1-one:

6-Bromo-2,2-dimethylindan-1-one (600 mg) was dissolved in a mixed solution of trifluoroacetic acid (5 ml) and concentrated sulfuric acid (4 ml), and at room temperature, NBS (1.34 g) was added thereto, followed by stirring at that temperature for 3 hours. The reaction solution was poured into ice-water and neutralized with an aqueous saturated ammonia. The precipitated matter was collected by filtration to obtain the compound (1.1 g) of Reference Example 62.
In Reference Example 63, the compound shown in Table 5 was produced in the same manner as in Reference Example 62.

### Reference Example 64:

### 6-Fluoro-7-methoxy-2,2-dimethylindan-1-one:

i-Pr₂NEt (8.9 ml) and 10% Pd-C (1 g) were added to an EtOH (300 ml) solution of the compound (15 g) of Reference Example 53, followed by stirring in a hydrogen atmosphere at room temperature for 1 hour. The insoluble matter was removed by filtration, the solvent was evaporated under reduced pressure, and water was added to the residue, followed by extraction with diethyl ether and washing with saturated brine. This was dried over anhydrous magnesium sulfate, and the solvent was evaporated under reduced pressure to obtain the compound (1.1 g) of Reference Example 64.
In Reference Examples 65 and 66, the compounds shown in Table 6 were produced in the same manner as in Reference Example 64.

### Reference Example 67:

### 7-Fluoro-3,3-dimethylquinoline-2,4(1H,3H)-dione:

P₂O₅ (10 g) was added to MsOH (100 ml), followed by stirring at 80°C for 30 minutes. 3-[(3-Fluorophenyl)amino]-2,2-dimethyl-3-oxopropionic acid (8 g) was added, followed by stirring at that temperature for 2 hours. The reaction solution was cooled to room temperature, poured into ice-water, and with stirring, an aqueous saturated ammonia was added thereto thereby making it basic. The precipitated matter was collected by filtration, washed with water, and dried under reduced pressure to obtain the compound (6 g) of Reference Example 67.

### Reference Example 68:

### Ethyl 4-(1-hydroxy-2,2,6-trimethyl-2,3-dihydro-1H-inden-1-yl)piperidin-1-carboxylate:

Et₃N (7 ml) and ethyl chloroformate (4 ml) were added to a toluene (50 ml) solution of a desalted product (1.5 g) of the compound of Example 6, followed by stirring at 100°C for 2 hours. The reaction solution was subjected to liquid-liquid separation with ethyl acetate/water, and the organic layer was washed with saturated brine, then dried over anhydrous sodium sulfate, and the solvent was evaporated under reduced pressure. The residue was purified by silica gel column chromatography (eluent: n-hexane/ethyl acetate = 5/1) to obtain the compound (1.04 g) of Reference Example 68.
In Reference Examples 69 and 70, the compounds shown in Table 6 were produced in the same manner as in Reference Example 68. In Reference Example 71, the compound shown in Table 6 was produced in the same manner as in Reference Example 27. In Reference Example 72, the compound shown in Table 7 was produced in the same manner as in Reference Example 68.

### Reference Example 73:

### 4-(2-Fluoro-5-methoxy)butanoic acid:

At room temperature, 2-fluoro-5-methoxybenzaldehyde (2.5 g) was added to a 1,2-dichloroethane (50 ml) solution of TiCl₄ (2.06 ml), followed by stirring at that temperature for 30 minutes. This was cooled to -78°C, then 1-ethoxy-1-[(trimethylsilyl)oxy]cyclopropane (3.76 ml) was added thereto, and with stirring, this was heated to 0°C, taking 3.5 hours. The reaction solution was poured into ice-water, extracted with chloroform, washed with saturated brine, then dried over anhydrous sodium sulfate, and the solvent was evaporated under reduced pressure. The resulting yellow oil was dissolved in ethanol (100 ml), and 10% Pd-C (450 mg) was added thereto, followed by stirring in a hydrogen atmosphere under normal pressure for 2 hours. The insoluble matter was removed by filtration using Celite, and the solvent was evaporated under reduced pressure to obtain an yellow oil. Then, this was dissolved in EtOH (50 ml), and an aqueous 1 N NaOH solution (17.5 ml) was added thereto, followed by stirring at room temperature for 4 days. The reaction solution was made acidic with diluted hydrochloric acid, then extracted with ethyl acetate, and washed with saturated brine. This was dried over anhydrous sodium sulfate, and the solvent was evaporated under reduced pressure to obtain the compound (3.45 g) of Reference Example 73 as a brown solid.
In Reference Example 74, in the same manner as in Reference Example 23; in Reference Example 75, in the same manner as in Reference Example 27; in Reference Example 76, in the same manner as in Reference Example 3; in Reference Example 77, in the same manner as in Reference Example 14; in Reference Example 78, in the same manner as in Reference Example 27; in Reference Example 79, in the same manner as in Reference Example 23; in Reference Example 80, in the same manner as in Reference Example 27; in Reference Example 81, in the same manner as in Reference Example 117; the compounds shown in Table 7 were produced.

### Reference Example 82:

### 6-Bromo-2,2-dimethyl-7-methoxyindan-1-one:

At room temperature, 55% oily NaH (760 mg) was added to a THF solution of the compound (989 mg) of Reference Example 81 and MeI (1.09 ml), followed by stirring at 60°C for 3 hours. An aqueous saturated ammonium chloride solution was added, followed by liquid-liquid separation with a mixed solvent (3/1) of ethyl acetate and THF. The organic layer was washed with saturated brine, dried with sodium sulfate, and the solvent was evaporated under reduced pressure. The residue was purified by silica gel chromatography (eluent; hexane/ethyl acetate = 6/1) to obtain 6-bromo-2,2-dimethyl-7-hydroxyindan-1-one (1.1 g). Then, MeI (0.298 ml) and K₂CO₃ (1.81 g) were added to its DMF solution in that order, followed by stirring at 60°C for 24 hours. After completion of the reaction, water was added, followed by liquid-liquid separation with ethyl acetate. The organic layer was washed with saturated brine, dried over anhydrous sodium sulfate, and the solvent was evaporated under reduced pressure. The residue was purified by silica gel column chromatography (eluent: hexane/ethyl acetate = 9/1 to 4/1) to obtain the compound (644 mg) of Reference Example 82 as a colorless powdery solid.

### Reference Example 83:

### 6-Fluoro-7-hydroxy-2,2-dimethylindan-1-one:

With cooling on ice, 1 M BBr₃/methylene chloride solution (60 ml) was gradually dropwise added to a methylene chloride solution of the compound (8.10 g) of Reference Example 64. This was stirred overnight at room temperature, and an aqueous saturated sodium hydrogencarbonate solution was added thereto, followed by extraction with methylene chloride. This was washed with saturated brine, dried over anhydrous sodium sulfate, and the solvent was evaporated under reduced pressure to obtain the compound (7.86 g) of Reference Example 83 as a brown solid.

### Reference Example 84:

### 7-Ethoxy-6-fluoro-2,2-dimethylindan-1-one:

At room temperature, EtI (5.0 ml) was added to a DMF solution of the compound (4.0 g) of Reference Example 83 and K₂CO₃ (8.54 g), followed by stirring overnight at that temperature. Water was added to the reaction solution, followed by extraction with ethyl acetate and washing with water and saturated brine. This was dried over anhydrous sodium sulfate, and the solvent was evaporated under reduced pressure to obtain the compound (4.47 g) of Reference Example 84 as a brown oil.
In Reference Example 83, in the same manner as in Reference Example 3; in Reference Example 86, in the same manner as in Reference Example 96; in Reference Example 87, in the same manner as in Reference Example 23 and then in the same manner as in Reference Example 27; in Reference Example 88, in the same manner as in Reference Example 3; in Reference Example 89, in the same manner as in Reference Example 67; in Reference Example 90, in the same manner as in Reference Example 27; in Reference Example 91, in the same manner as in Reference Example 3; the compounds shown in Tables 7 and 8 were produced.

### Reference Example 92:

### 4-Fluoro-6-hydroxy-2,2,7-trimethylindan-1-one:

In the same manner as in Reference Example 14, the compound (442 mg) of Reference Example 92, and a mixture (239 mg) of 4-fluoro-6-hydroxy-2,2,5-trimethylindan-1-one and 4-fluoro-6-hydroxy-2,2-dimethylindan-1-one were obtained each as a brown solid, from the compound of Reference Example 91.
In Reference Example 93, the compound shown in Table 8 was produced from the compound of Reference Example 92, in the same manner as in Reference Example 27.

### Reference Example 94:

### 1-(4-Fluoro-2-hydroxyphenyl)-2-methylpropan-1-one:

A mixture of AlCl₃ (150 g) and NaCl (150 g) was melted at 160°C with stirring. At the same temperature, 3-fluorophenyl 2-methylpropanoate (50 g) was added, followed by stirring for 1 hour. The reaction liquid was cooled, then with cooling on ice, water was gradually added thereto to break AlCl₃. Then, concentrated hydrochloric acid was added, followed by heating and stirring so as to dissolve the mass. When the mass became suspended, this was cooled, and extracted with diethyl ether, washed with saturated brine, dried over anhydrous sodium sulfate. The solvent was evaporated under reduced pressure to obtain the compound (49 g) of Reference Example 94 as a brown oil.

### Reference Example 95:

### 1-(3,5-Dibromo-4-fluoro-2-hydroxyphenyl)-2-methylpropan-1-one:

At -10°C, NBS (115 g) was added to a mixed solution of the compound (49 g) of Reference Example 94 in trifluoroacetic acid (200 ml) and concentrated sulfuric acid (40 ml), taking 1 hour. Then, this was stirred at -10°C to 0°C for 1 hour, and the reaction solution was poured into ice-water, followed by extraction with diethyl ether. The organic layer was concentrated under reduced pressure, the residue was subjected to liquid-liquid separation with an aqueous saturated sodium hydrogencarbonate solution and diethyl ether, and the organic layer was washed several times with an aqueous 5% sodium thiosulfate solution. This was further washed with saturated brine, dried over anhydrous sodium sulfate, and the solvent was evaporated under reduced pressure to obtain a brown oil. After allowing it as it is, partial crystallization was found. The crystallized part was collected by filtration to obtain the compound (91 g) of Reference Example 95 as a brown crystal.

### Reference Example 96:

### N'-[1-(3,5-dibromo-4-fluoro-2-hydroxyphenyl)-2-methylpropylidene]-1-methylpiperidine-4-carbohydrazide acetate:

Acetic acid (7.3 ml) was added to an i-PrOH solution of the compound (34 g) of Reference Example 95 and 1-methylpiperidine-4-carbohydrazide (20 g), followed by heating under reflux for 5 hours. Since an insoluble matter precipiated therein, the reaction solution was cooled to room temperature, and it was collected by filtration and washed with i-PrOH to obtain the compound (40 g) of Reference Example 96 as a crystal.

### Reference Example 97:

### 3,4-Difluoro-5-methoxybenzaldehyde:

1.58 M n-BuLi/hexane solution (1.4 ml) was added to a diethyl ether solution of 5-bromo-1,2-difluoro-3-methoxybenzene (497 mg) in an argon atmosphere at -70°C, followed by stirring at that temperature for 1 hour. DMF (0.259 ml) was added, followed by stirring for 30 minutes. After completion of the reaction, water was added, followed by extraction with diethyl ether, washing with saturated brine, and drying over anhydrous sodium sulfate. The solvent was evaporated under reduced pressure to obtain the compound (317 mg) of Reference Example 97.
In Reference Example 98, the compound in Table 8 was produced in the same manner as in Reference Example 84.

### Reference Example 99:

### 1-(2-Fluoro-6-methoxyphenyl)-2-methylpropan-1-ol:

1.58 M n-BuLi/hexane solution (152 ml) was gradually added to a THF solution of 3-fluoroanisole (28 g) and N,N,N',N',N"-pentamethyldiethylenetriamine (48 ml), in an argon atmosphere at -78°C, followed by stirring at that temperature for 1 hour. Isobutylaldehyde (22 ml) was gradually added to it, and further stirred for 15 minutes. This was gradually heated to room temperature, then water was added to it, and extracted with diisopropyl ether. This was washed with water and saturated brine, dried over anhydrous sodium sulfate, and the solvent was evaporated under reduced pressure to obtain the compound (47 g) of Reference Example 99 as an oil.
In Reference Example 100, in the same manner as in Reference Example 27; in Reference Example 101, in the same manner as in Reference Example 1; in Reference Example 102, in the same manner as in Reference Example 23; in Reference Example 103, in the same manner as in Reference Example 27; in Reference Example 104, in the same manner as in Reference Example 3; the compounds shown in Table 9 were produced.

### Reference Example 105:

### 4-Bromo-5,5-difluoro-7-methoxyindan-1-one:

A trifluoroacetic acid/concentrated sulfuric acid (5/1) mixed solution of the compound (4.4 g) of Reference Example 104 was cooled to 0°C, and NBS (3.8 g) was, as divided into 10 portions, separately added thereto, followed by stirring at 0°C for 1 hour. Water was added to the reaction solution, followed by extraction with ethyl acetate, washing with saturated brine, and drying over anhydrous sodium sulfate. The solvent was evaporated under reduced pressure to obtain 3-(2-bromo-3,4-difluoro-5-methoxyphenyl)propionic acid as a crude product. Then, this was dissolved in TfOH (8.8 ml), followed by stirring at room temperature for 30 minutes. The reaction solution was poured into ice-water, followed by extraction with ethyl acetate, washing with saturated brine, and drying over anhydrous sodium sulfate. The solvent was evaporated under reduced pressure, and the residue was purified by silica gel column chromatography (eluent: n-hexane/ethyl acetate = 4/1) to obtain the compound (2.84 g) of Reference Example 105.

### Reference Example 106:

### 5,5-Difluoro-7-methoxy-2,2-dimethylindan-1-one :

At room temperature, 55% oily NaH (978 mg) was added to a THF solution of the compound (2.8 g) of Reference Example 105 and MeI (1.4 ml), followed by stirring at that temperature for 1 hour. An aqueous saturated ammonium chloride solution was added, followed by extraction with ethyl acetate, washing with saturated brine, and drying over anhydrous sodium sulfate. The solvent was evaporated under reduced pressure to obtain 4-bromo-5,5-difluoro-7-methoxy-2,2-dimethylindan-1-one as a crude product. Then, this was dissolved in EtOH, 10% Pd-C was added, followed by stirring in a hydrogen atmosphere (1 atmospheric pressure) at room temperature for 20 hours. The insoluble matter was removed by filtration using Celite, the solvent was evaporated under reduced pressure, and the residue was purified by silica gel column chromatography (eluent: n-hexane/ethyl acetate = 9/1) to obtain the compound (550 mg) of Reference Example 106 as a colorless oil.

### Reference Example 107:

### 5,6-Difluoroindan-1-ol:

With cooling on ice, NaBH₄ (774 mg) was added to an MeOH solution of 5,6-difluoroindan-1-one (3.44 g), followed by stirring at that temperature for 1 hour. Acetone was added to the reaction solution, then the solvent was evaporated under reduced pressure, and the residue was subjected to liquid-liquid separation with saturated ammonium chloride and ethyl acetate. The organic layer was washed with saturated brine, dried over anhydrous sodium sulfate, and the solvent was evaporated under reduced pressure to obtain the compound (4.29 g) of Reference Example 107 as an oil.

### Reference Example 108:

### 5,6-Difluoro-1-{[2-(trimethylsilyl)ethoxy]methoxy} indane:

With cooling on ice, 55% oily NaH (1.31 g) and 1-(chloromethoxy)-2-(trimethylsilyl)ethane (5.0 g) were added to a DMF solution of the compound (4.29 g) of Reference Example 107, followed by stirring overnight at room temperature. Water was added to the reaction solution, extracted with ethyl acetate, washed with water and saturated brine, dried over anhydrous sodium sulfate, and the solvent was evaporated under reduced pressure. The residue was purified by silica gel column chromatography (eluent: n-hexane/ethyl acetate = 20/1) to obtain the compound (4.25 g) of Reference Example 108 as an oil.

### Reference Example 110:

### 5,6-Difluoro-7-hydroxymethyl-1-{[2-(trimethylsilyl)ethoxy]methoxy} indane:

1.58 M n-BuLi/hexane solution (3.6 ml) was added to a THF solution of the compound (1.46 g) of Reference Example 107 in an argon atmosphere at -78°C, followed by stirring at that temperature for 1 hour. DMF (0.49 ml) was added to it at that temperature, and gradually heated to room temperature, and then subjected to liquid-liquid separation with an aqueous saturated ammonium chloride solution and ethyl acetate, and the organic layer was washed with saturated brine. This was dried over anhydrous sodium sulfate, the solvent was evaporated under reduced pressure, and the residue was purified by silica gel column chromatography (eluent: n-hexane/ethyl acetate = 4/1) to obtain 5,6-difluoro-7-formyl-1-{[2-(trimethylsilyl)ethoxy]methoxy}indane (1.31 g) as an oil. With cooling on ice, NaBH₄ (227 mg) was added to its MeOH solution, followed by stirring at that temperature for 1 hour. Acetone was added to the reaction solution, then the solvent was evaporated under reduced pressure, and the residue was subjected to liquid-liquid separation with saturated ammonium chloride and ethyl acetate. The organic layer was washed with saturated brine, dried over anhydrous sodium sulfate, and the solvent was evaporated under reduced pressure to obtain the compound (929 mg) of Reference Example 110 as an oil.

### Reference Example 111:

### 5,6-Difluoro-7-methoxymethyl-1- {[2-(trimethylsilyl)ethoxy]methoxy} indane:

With cooling on ice, 55% oily NaH (147 mg) was added to a THF solution of the compound (929 mg) of Reference Example 110 and MeI (0.21 ml), followed by stirring overnight at room temperature. The reaction solution was subjected to liquid-liquid separation with an aqueous saturated ammonium chloride solution and ethyl acetate, and the organic layer was washed with saturated brine. This was dried over anhydrous sodium sulfate, the solvent was evaporated under reduced pressure, and the residue was purified by silica gel column chromatography (eluent: n-hexane/ethyl acetate = 20/1) to obtain the compound (905 mg) of Reference Example 111 as an oil.

### Reference Example 112:

### 5,6-Difluoro-7-methoxymethylindan-1-ol:

CsF (1.59 g) was added to a DMF solution of the compound (899 mg) of Reference Example 111, followed by stirring overnight under heat at 130°C. The reaction solution was cooled to room temperature, ethyl acetate was added thereto and stirred, and the insoluble matter was removed by filtration using Celite. The filtrate was concentrated under reduced pressure, and the residue was purified by silica gel column chromatography (eluent: n-hexane/ethyl acetate = 2/1) to obtain the compound (479 mg) of Reference Example 112 as an oil.

### Reference Example 113:

### 5,6-Difluoro-7-methoxymethylindan-1-one:

The compound (479 mg) of Reference Example 112, N-methylmorpholine (393 mg) and Pr₄N⁺RuO₄⁻ (79 mg) were added to a 1/1 mixed solvent of MeCN and methylene chloride, followed by stirring overnight at room temperature. The solvent was evaporated under reduced pressure, and the residue was purified by silica gel column chromatography (eluent: n-hexane/ethyl acetate = 2/1) to obtain the compound (450 mg) of Reference Example 113 as an oil.
In Reference Example 114, in the same manner as in Reference Example 27; in Reference Example 115, in the same manner as in Reference Example 8 but from the compound of Reference Example 71; the compounds shown in Table 9 were produced.

### Reference Example 116:

### 1-(2-Fluoro-6-hydroxyphenyl)-2-methylpropan-1-one:

A methylene chloride solution of the compound (43 g) of Reference Example 99 was added to 1 M BBr₃/methylene chloride solution (250 ml) in an argon atmosphere at - 78°C, stirred for 1 hour at a temperature falling between that temperature and -40°C, then gradually heated to 0°C. Water was added to it for liquid-liquid separation, and the organic layer was washed with saturated brine. This was dried over anhydrous sodium sulfate, and the solvent was evaporated under reduced pressure to obtain the compound (38 g) of Reference Example 116 as an oil.

### Reference Example 117:

### 1-(3-Bromo-6-fluoro-2-hydroxyphenyl)-2-methylpropan-1-one:

Bromine (60 g) was gradually dropwise added to a toluene solution of t-BuNH₂ (51 g) in an argon atmosphere at -30°C. This was stirred fro 30 minutes at that temperature, then cooled to -78°C, and a methylene chloride solution of the compound (70 g) of Reference Example 116 was gradually added to it. This was further stirred for 1 hour at that temperature, then gradually heated to 0°C, taking 4 hours. Water was added to the reaction solution, extracted with ethyl acetate, washed with water and saturated brine, then dried over anhydrous sodium sulfate, and the solvent was evaporated under reduced pressure to obtain the compound (88 g) of Reference Example 117 as an oil.

### Reference Example 118:

### N'-[1-(3-bromo-6-fluoro-2-hydroxyphenyl)-2-methylpropylidene]quinuclidine-3-carbohydrazide:

A mixture of methyl quinuclidine-3-carboxylate hydrochloride (1.0 g) and hydrazine monohydrate (3.0 ml) was stirred for 2 hours under heat at 100°C. Still under heat, this was concentrated under reduced pressure, and the residue was dissolved in i-PrOH. To it were added the compound (2.7 g) of Reference Example 117 and acetic acid (0.596 ml), followed by heating under reflux for 7 hours. After completion of the reaction, the solvent was evaporated under reduced pressure, and the residue was subjected to liquid-liquid separation with 0.5 N hydrochloric acid and diisopropyl ether. The aqueous layer was again washed with diisopropyl ether, then neutralized with sodium hydrogencarbonate, and extracted three times with chloroform. The chloroform layer was dried over anhydrous sodium sulfate, and the solvent was evaporated under reduced pressure to obtain the compound (710 mg) of Reference Example 118 as a colorless amorphous substance.

### Reference Example 119:

### 1-(2-Fluoro-6-methoxyphenyl)-2-methylpropan-1-one:

With cooling on ice, SO₃·pyridine (139 g) was gradually added to a DMSO solution of the compound (47 g) of Reference Example 99 and Et₃N (152 ml), followed by stirring overnight at room temperature. Water was added to the reaction solution, extracted with diisopropyl ether, washed with diluted hydrochloric acid, water and saturated brine, dried over anhydrous sodium sulfate, and the solvent was evaporated under reduced pressure to obtain the compound (43 g) of Reference Example 119 as an oil.

### Reference Example 120:

### 6-Fluoro-7-methoxy-2,2-dimethyl-1-trimethylsiloxayindane-1-carbonitrile:

At room temperature, (TMS)CN (930 mg) was added to a 1,2-dichloroethane solution of the compound (485 mg) of Reference Example 64 and ZnI₂ (37 mg), followed by stirring at that temperature for 2 hours. An aqueous saturated sodium hydrogencarbonate solution was added to the reaction solution, extracted with chloroform, washed with saturated brine, dried over anhydrous sodium sulfate, and the solvent was evaporated under reduced pressure to obtain the compound (710 mg) of Reference Example 120.

### Reference Example 121:

### Methyl (6-fluoro-1-hydroxy-7-methoxy-2,2-dimethyl-2,3-dihydro-1H-indene)acetate:

2.67 M n-BuLi/hexane solution (17 ml) was added to a THF solution of i-Pr₂NH (6.4 ml) in an argon atmosphere at -78°C, followed by stirring at that temperature for 30 minutes. Methyl acetate (3.6 ml) was added to it, and further stirred for 30 minutes. Then, a THF solution of the compound (1.9 g) of Reference Example 64 was added to it and stirred for 2 hours. Water was added to the reaction solution, extracted with ethyl acetate, washed with water and saturated brine, dried over anhydrous sodium sulfate, and the solvent was evaporated under reduced pressure to obtain the compound (2.8 g) of Reference Example 121.

### Reference Example 122:

### 6-Fluoro-1-(3-hydroxyethyl)-7-methoxy-2,2-dimethylindan-1-ol:

A THF solution of the compound (1.0 g) of Reference Example 121 was added to a THF suspension of LiAlH₄ (202 mg), followed by heating under reflux at 80°C for 2.5 hours. The reaction solution was cooled, diluted with THF, water (0.8 ml) and an aqueous 15% NaOH solution (0.2 ml) were added thereto and stirred, and the insoluble matter was removed by filtration using Celite. The solvent was evaporated under reduced pressure, and the residue was purified by silica gel column chromatography (eluent: n-hexane/ethyl acetate = 5/1 to 3/1) to obtain the compound (366 mg) of Reference Example 122.

### Reference Example 123:

### 1-(1-Hydroxy-2,2,6-trimethyl-2,3-dihydro-1H-inden-1-yl)acetone oxime:

1.58 M n-BuLi/hexane solution (86 ml) was added to a THF solution of acetone oxime (3.94 g) in an argon atmosphere at 0°C, followed by stirring at that temperature for 2 hours. Next, 2,2,6-trimethylindan-1-one (7.83 g) was added to it, and further stirred for 3 hours. An aqueous saturated ammonium chloride solution was added to the reaction solution, extracted with ethyl acetate, and washed with water and saturated brine. This was dried over anhydrous sodium sulfate, and the solvent was evaporated under reduced pressure to obtain the compound (12 g) of Reference Example 123.

### Reference Example 124:

### 2,2,3',6-Tetramethyl-2,3-dihydro-4'H-spiro[inden-1,5'-isoxazole]:

With cooling on ice, P₂O₅ (60 g) was gradually added to a chloroform solution of the compound (12 g) of Reference Example 123, followed by stirring at that temperature for 2 hours. The reaction solution was poured into ice-water, extracted with chloroform, dried over anhydrous sodium sulfate, and the solvent was evaporated under reduced pressure. The residue was purified by silica gel column chromatography (eluent: n-hexane/ethyl acetate = 10/1 to 4/1) to obtain the compound (5.7 g) of Reference Example 124:

### Reference Example 125:

### 2,2,3',6-Tetramethyl-2,3-dihydro-4'H-spiro[inden-1,5'-isoxazolidine]:

BF₃·Et₂O (0.76 ml) was added to a methylene chloride solution of the compound (1.15 g) of Reference Example 124 in an argon atmosphere at -78°C, followed by stirring at that temperature for 10 minutes. Then, 0.98 M MeLi/diethyl ether solution (6.12 ml) was added thereto, followed by stirring at that temperature for 2 hours. An aqueous saturated sodium hydrogencarbonate solution was added to the reaction solution, and extracted with chloroform. This was dried over anhydrous sodium sulfate, the solvent was evaporated under reduced pressure, and the residue was purified by silica gel column chromatography (eluent: chloroform/ethyl acetate = 2/1) to obtain the compound (421 mg) of Reference Example 125.

### Reference Example 126:

### 6-Fluoro-7-methoxy-2,2-dimethyl-1-(3-{[(t-butyl)dimethylsilyl]oxy}propyl)indan-1-ol:

In an argon atmosphere, (3-bromopropoxy)(t-butyl)dimethylsilane (3.3 ml) was dropwise added to a THF suspension of Mg (350 mg), followed by stirring overnight at room temperature. To this was added a THF solution of the compound (1.0 g) of Reference Example 64, followed by stirring under heat at 60°C for 1 hour. The reaction solution was cooled, an aqueous saturated ammonium chloride solution was added thereto, extracted with ethyl acetate, and dried over anhydrous sodium sulfate. The solvent was evaporated under reduced pressure, and the residue was purified by silica gel column chromatography (eluent: n-hexane/ethyl acetate = 10/1) to obtain the compound (865 mg) of Reference Example 126.

### Reference Example 127:

### 6-Fluoro-1-(3-hydoxypropyl)-7-methoxy-2,2-dimethylindan-1-ol:

1 M n-Bu₄NF/THF solution (4.4 ml) was added to a THF solution of the compound (827 mg) of Reference Example 126, followed by stirring at room temperature for 2 hours. Water was added to the reaction solution, extracted with ethyl acetate, dried over anhydrous sodium sulfate, and the solvent was evaporated under reduced pressure. The residue was purified by silica gel column chromatography (eluent: n-hexane/ethyl acetate = 5/1 to 1/1) to obtain the compound (384 mg) of Reference Example 127 as an oil.

### Reference Example 128:

### 6-Fluoro-7-methoxy-2,2-dimethyl-1-(pyridin-2-ylmethyl)indan-1-ol:

2.67 M n-BuLi/hexane solution was added to a THF solution of i-Pr₂NH (0.61 ml) in an argon atmosphere at -78°C, followed by stirring for 30 minutes at that temperature. To it was added 2-picoline (0.43 ml), then heated to room temperature, followed by stirring for 30 minutes. To it, further added was a THF solution of the compound (460 mg) of Reference Example 64, followed by stirring at that temperature for 2 hours. Water was added to the reaction solution, extracted with ethyl acetate, washed with water and saturated brine, and dried over anhydrous sodium sulfate. The solvent was evaporated under reduced pressure, and the residue was purified by silica gel column chromatography (eluent: n-hexane/ethyl acetate = 5/1) to obtain the compound (525 mg) of Reference Example 128 as an oil.

### Reference Example 129:

### 4-Fluoro-6,7-dihydroxy-2,2-dimethylindan-1-one:

With cooling on ice, 1 M BBr₃/methylene chloride solution (17 ml) was added to a 1,2-dichloroethane solution of the compound (1.3 g) of Reference Example 90, followed by stirring at that temperature for 1 hour. An aqueous saturated sodium hydrogencarbonate solution was added to the reaction solution, extracted with chloroform, dried over anhydrous sodium sulfate, and the solvent was evaporated under reduced pressure. The residue was purified by silica gel column chromatography (eluent: n-hexane/ethyl acetate = 5/1 to 1/1) to obtain the compound (735 mg) of Reference Example 129.

### Reference Example 130:

### 5-Fluoro-7,7-dimethyl-6,7-dihydro-8H-indano[4,5-d][1,3]dioxol-8-one:

KF (276 mg) and dibromomethane (0.074 ml) were added to a DMF solution of the compound (200 mg) of Reference Example 129, followed by stirring under heat at 100°C for 5 hours. The reaction solution was concentrated under reduced pressure, then subjected to liquid-liquid separation with ethyl acetate and an aqueous saturated sodium hydrogencarbonate solution, and the organic layer was dried over anhydrous sodium sulfate. The solvent was evaporated under reduced pressure, and the residue was purified by silica gel column chromatography (eluent: n-hexane/ethyl acetate =5/1) to obtain the compound (40 mg) of Reference Example 130.

### Reference Example 131:

### 6-Fluoro-8,8-dimethyl-2,3,7,8-tetrahydro-9H-indeno[4,5-b] [1,4]dioxin-9-one:

A DMF solution of the compound (251 mg) of Reference Example 129, 1,2-dibromoethane (0.11 ml) and K₂CO₃ (412 mg) was stirred under heat at 80°C for 4 hours. The reaction solution was concentrated under reduced pressure, then subjected to liquid-liquid separation with ethyl acetate and an aqueous saturated sodium hydrogencarbonate solution, the organic layer was dried over anhydrous sodium sulfate, and the solvent was evaporated under reduced pressure. The residue was purified by silica gel column chromatography (eluent: n-hexane/ethyl acetate = 3/1) to obtain the compound (68 mg) of Reference Example 131.
In Reference Example 132, the compound in Table 11 was produced in the same manner as in Reference Example 129 but from the compound of Reference Example 71.

### Reference Example 133:

### 4-Fluoro-7-hydroxy-6-iodo-2,2-dimethylindan-1-one:

The compound (4.26 g) of Reference Example 132 was dissolved in trifluoroacetic acid (169 ml) and concentrated sulfuric acid (35 ml), and N-iodosuccinimide (5.18 g) was added to it at 0°C. This was stirred at that temperature for 1 hour, then water was added thereto, and extracted with ethyl acetate. This was washed with saturated brine, dried over anhydrous sodium sulfate, and the solvent was evaporated under reduced pressure. The residue was purified by silica gel column chromatography (eluent: n-hexane/ethyl acetate = 10/1) to obtain the compound (6.46 g) of Reference Example 133.

### Reference Example 134:

### 5-Fluoro-7,7-dimethyl-2-(trimethylsilyl)-6,7-dihydro-8H-indene[5,4-b]furan-8-one:

Et₃N (43.5 ml), trimethylsilylacetylene (17.3ml), PdCl₂(PPh₃)₂ (548 mg) and CuI (595 mg) were added to an MeCN solution of the compound (5.0 g) of Reference Example 133, followed by stirring at 50°C for 2 days. The reaction solution was concentrated under reduced pressure, and the residue was purified by silica gel column chromatography (eluent: hexane/ethyl acetate = 5/2) to obtain the compound (4.09 g) of Reference Example 134.
In Reference Example 135, in the same manner as in Reference Example 3; in Reference Example 136, in the same manner as in Reference Example 23; in Reference Example 137, in the same manner as in Reference Example 27; the compounds shown in Table 11 were produced.

### Reference Example 138:

### 6-Fluoro-7-(2-hydroxyethoxy)-2,2-dimethylindan-1-one:

A DMF solution of the compound (500 mg) of Reference Example 83, 2-bromoethanol (0.55 ml) and K₂CO₃ (1.1 g) was stirred under heat at 80°C. After 6 hours, and after 8 hours, 2-bromoethanol (0.55 ml) was added thereto, followed by stirring overnight under heat at that temperature. Water was added to the reaction solution, extracted with ethyl acetate, and washed with water and saturated brine. This was dried over anhydrous sodium sulfate, the solvent was evaporated under reduced pressure, and the residue was purified by silica gel column chromatography (eluent; n-hexane/ethyl acetate = 5/1) to obtain the compound (578 mg) of Reference Example 138 as an oil.

### Reference Example 139:

### 1-(3-Buten-1-yl)-6-fluoro-7-methoxy-2,2-dimethylindan-1-ol:

In an argon atmosphere, 4-bromo-1-butene (1.5 ml) was dropwise added to a THF suspension of Mg (350 mg), followed by stirring overnight at room temperature. A THF solution of the compound (1.0 g) of Reference Example 64 was added to it at room temperature, followed by stirring under heat at 60°C for 1 hour. An aqueous saturated ammonium chloride solution was added to the reaction solution, extracted with ethyl acetate, and dried over anhydrous sodium sulfate. The solvent was evaporated under reduced pressure, and the residue was purified by silica gel column chromatography (eluent: n-hexane/ethyl acetate = 10/1) to obtain the compound (890 mg) of Reference Example 139.

### Reference Example 140:

### 6'-Fluoro-5-(iodomethyl)-7'-methoxy-2',2'-dimethyl-2',3',4,5-tetrahydro-3H-spiro[furan-2,1'-indene] :

An MeCN solution of the compound (888 mg) of Reference Example 139 was gradually dropwise added to an acetonitrile solution of iodine (1.7 g) and NaHCO₃ (1.5 g), at room temperature. This was stirred at that temperature for 1 hour, then an aqueous sodium thiosulfate solution was added to the reaction solution, extracted with ethyl acetate, washed with saturated brine, and dried over anhydrous sodium sulfate. The solvent was evaporated under reduced pressure, and the residue was purified by silica gel column chromatography (eluent: n-hexane/ethyl acetate = 20/1) to obtain the compound (985 mg) of Reference Example 140 as an oil.

### Reference Example 141:

### 2-(2-Hydroxyethyl)-6-methylindan-1-one:

A mixture of 3-(4-methylbenzyl)dihydrofuran-2(3H)-one (2.9 g) and TfOH (23 g) was stirred overnight at 80°C. This was restored to room temperature, then water was added thereto and extracted with chloroform. The organic layer was dried over anhydrous sodium sulfate, the solvent was evaporated under reduced pressure, and the residue was purified by silica gel column chromatography (eluent: n-hexane/ethyl acetate = 1/1) to obtain the compound (1.4 g) of Reference Example 141.

### Reference Example 142:

### 2-(2-Hydroxyethyl)-2,6-dimethylindan-1-one:

55% oily NaH (696 mg) was added to a THF solution of the compound (1.38 g) of Reference Example 141 at room temperature, followed by stirring for 10 minutes. To it was added MeI (1.04 ml), followed by stirring at room temperature for 2 hours. An aqueous saturated ammonium chloride solution was added to the reaction solution, and extracted with ethyl acetate. This was dried over anhydrous sodium sulfate, and the solvent was evaporated under reduced pressure to obtain a mixture of the compound of Reference Example 142 and 2-(2-methoxyethyl)-2,6-dimethylindan-1-one. This was dissolved in 1,2-dichloroethane, and 1 M BBr₃/dichloromethane solution (7.3 ml) was added thereto and stirred at room temperature for 1 hour. An aqueous saturated sodium hydrogencarbonate solution was added to the reaction solution, extracted with chloroform, dried over anhydrous sodium sulfate, and the solvent was evaporated under reduced pressure. The residue was purified by silica gel column chromatography (eluent: n-hexane/ethyl acetate = 5/2) to obtain the compound (540 mg) of Reference Example 142 and 2-(2-bromoethyl)-2,6-dimethylindan-1-one (1.25 g).

### Reference Example 143:

### N'-[1-(3-butyl-6-fluoro-2-hydroxyphenyl)-2-methylpropylidene]isonicotinohydrazide:

An EtOH suspension of the compound (88 g) of Reference Example 117 and isonicotinohydrazide (55 g) was heated under reflux for 6 hours. The solvent was evaporated, water and diethyl ether were added, followed by stirring. The precipitated matter was collected by filtration, washed with water and diethyl ether, and dried under reduced pressure to obtain the compound (92) of Reference Example 143 as a crystal.

### Reference Example 144:

### 4-Bromo-7-fluoro-3-hydroxy-2,2-dimethyl-3-(pyridin-4-yl)indan-1-one:

At room temperature, PhI(OAc)₂ (92 g) was added to a 1,2-dichloroethane solution of the compound (91 g) of Reference Example 143, followed by stirring at that temperature for 2 hours. An aqueous saturated ammonium hydrogencarbonate solution was added, followed by stirring. The precipitated matter was removed by filtration using Celite. The filtrate was subjected to liquid-liquid separation, the organic layer was dried over anhydrous sodium sulfate, and the solvent was evaporated under reduced pressure. The residue was dissolved in 1,2-dichloroethane, then 1,8-diazabicyclo[5,4,0]undec-7-ene (7.2 ml) was added thereto, followed by stirring under heat at 60°C for 1 hour. The reaction suspension was cooled to room temperature, the precipitated matter was collected by filtration and washed with a small amount of 1,2-dichloroethane to obtain the compound (65 g) of Reference Example 144 as a crystal.

### Example 1:

### 2,2-Dimethyl-1-(1-methylpiperidin-4-yl)indan-1-ol monofumarate:

With cooling on ice, a THF solution of (1-methylpiperidin-4-yl)magnesium chloride (2 equivalents) that had been separately prepared from 4-chloro-1-methylpiperidine, Mg (2 equivalents) and EtBr (catalytic amount) was added to a THF (20 ml) solution of 2,2-dimethylindan-1-one (961 mg), followed by stirring for 2 hours at a temperature falling between that temperature and room temperature. An aqueous saturated ammonium chloride solution was added to the reaction solution, and the aqueous layer was extracted with ethyl acetate, washed with saturated brine, and dried over anhydrous sodium sulfate. The solvent was evaporated under reduced pressure, and the residue was purified by silica gel column chromatography (eluent: chloroform/methanol/aqueous saturated ammonia = 10/1/0.1) to obtain a colorless crystal (1.46 g). This was formed into its salt with 1 equivalent of fumaric acid, and washed with diethyl ether to obtain the compound of Example 1 as a colorless crystal.
In Examples 2 to 5, the compounds shown in Table 12 were produced in the same manner as in Example 1.

### Example 6:

### 2,2,6-Trimethyl-1-(1-methylpipendin-4-yl)indan-1-ol monofumarate:

With cooling on ice, a THF solution of (1-methylpiperidin-4-yl)magnesium chloride (2 equivalents) that had been separately prepared from 4-chloro-1-methylpiperidine, Mg (2 equivalents) and EtBr (catalytic amount) was added to a THF (100 ml) solution of 2,2,6-trimethylindan-1-one (8.0 g), followed by stirring for 2 hours at a temperature falling between that temperature and room temperature. An aqueous saturated ammonium chloride solution was added to the reaction solution, and the aqueous layer was extracted with ethyl acetate, washed with saturated brine, and dried over anhydrous sodium sulfate. The solvent was evaporated under reduced pressure, and the residue was purified by silica gel column chromatography (eluent: chloroform/methanol/aqueous saturated ammonia = 10/1/0.1) to obtain a colorless crystal (9.2 g). This was formed into its salt with 1 equivalent of fumaric acid, and recrystallized from ethanol/ethyl acetate to obtain the compound of Example 6 as a colorless crystal.
In Examples 7 to 12, the compounds shown in Tables 13 and 14 were produced in the same manner as in Example 6.

### Example 13:

### 7-Bromo-2,2-dimethyl-1-(1-methylpiperidin-4-yl)indan-1-ol monofumarate:

The compound (1.93 g) of Example 48 was dissolved in a mixed solvent of THF (50 ml) and ethyl acetate (50 ml), and i-PrNEt (0.68 ml) and then 10% PD-C (1 g) were added, and in a hydrogen atmosphere (ordinary pressure), this was stirred at room temperature for 2 hours. The reaction solution was diluted with ethanol, the insoluble matter was removed by filtration, and the solvent was evaporated under reduced pressure. The residue was subjected to liquid-liquid separation with an aqueous saturated sodium hydrogencarbonate solution and chloroform, the organic layer was dried over anhydrous sodium sulfate, and the solvent was evaporated under reduced pressure. The residue was purified by silica gel column chromatography (eluent: chloroform/methanol/aqueous saturated ammonia = 20/1/0.1) to obtain a crystal (735 mg). This was formed into its salt with one equivalent of fumaric acid, and recrystallized from EtOH/acetone to obtain the compound of Example 13 as a colorless crystal.
In Examples 14 to 16, the compounds shown in Table 14 were produced in the same manner as in Example 6.

### Example 17;

### 6-Cyano-2,2-dimethyl-1-(1-methylpiperidin-4-yl)indan-1-ol monofumarate:

An N-methylpyrrolidone (5 ml) solution of a desalted product (500 mg) of the compound of Example 14, Zn(CN)₂ (191 mg), Pd(PPh₃)₄ (512 mg) and Ca(OH)₂ (121 mg) was stirred under heat at 100°C for 4 hours. The reaction solution was subjected to liquid-liquid separation with chloroform and an aqueous saturated sodium hydrogencarbonate solution. The organic layer was washed with water and saturated brine, and dried over anhydrous sodium sulfate. The solvent was evaporated under reduced pressure. The residue was purified by silica gel column chromatography (eluent: chloroform/methanol/aqueous saturated ammonia = 10/1/0.1) to obtain a crystal (330 mg). This was formed into its salt with one equivalent of fumaric acid, and washed with diethyl ether to obtain the compound of Example 17 as a colorless crystal.
In Examples 18 to 62, the compounds shown in Tables 15 to 23 were produced in the same manner as in Example 6.

### Example 63:

### 7-Fluoro-3,3-dimethyl-4-(1-methylpiperidin-4-yl)-1,2,3,4-tetrahydroquinolin-4-ol monofumarate:

With cooling on ice, LiAlH₄ (302 mg) was added to a THF (30 ml) solution of the compound (500 mg) of Example 64, followed by heating under reflux for 24 hours. The reaction solution was cooled with ice, Na₂SO₄·10H₂O and Celite were added thereto, followed by stirring. Then, the insoluble matter was removed by filtration. The solvent was evaporated under reduced pressure, and the residue was purified by silica gel column chromatography (eluent: chloroform/methanol/aqueous saturated ammonia = 10/1/0.1) to obtain an amorphous substance (450 mg). This was formed into its salt with one equivalent of fumaric acid, and washed with acetone to obtain the compound of Example 63 as a crystal.
In Examples 64 to 70, the compounds shown in Tables 23 and 24 were produced in the same manner as in Example 6.
In Example 71, the compound shown in Table 24 was produced in the same manner as in Example 72 given below.

### Example 72:

### 2,2,6-Trimethyl-1-(1-methylpyrrolidin-3-yl)indan-1-ol monofumarate:

At -10°C, 1.6 M n-BuLi/hexane solution (3.16 ml) was added to a THF (20 ml) solution of i-Pr₂NH (0.7 ml), followed by stirring at that temperature for 30 minutes. The reaction solution was cooled to -78°C, and N-methylpyrrolidone (0.386 ml) was added, followed by further stirring for 1 hour. A THF (5 ml) solution of 2,2,6-trimethylindan-1-one (450 ml) was added, followed by stirring for 30 minutes, and then heated to -30°C. Then, an aqueous saturated ammonium chloride solution was added, followed by extraction with diethyl ether. The organic layer was washed with saturated brine, dried over anhydrous sodium sulfate, and the solvent was evaporated under reduced pressure. The residue was dissolved in THF (50 ml), and LiAlH₄ (950 mg) was added thereto, followed by heating under reflux for 1 hour. With cooling on ice, Na₂SO_{4·}10H₂O and Celite were added to the reaction solution, followed by stirring. Then, the insoluble matter was removed by filtration. The solvent was evaporated under reduced pressure, and the residue was purified by silica gel column chromatography (eluent: chloroform/methanol/aqueous saturated ammonia = 10/1/0.1) to obtain an oil (435 mg). This was formed into its salt with one equivalent of fumaric acid, and washed with ethyl acetate to obtain the compound of Example 72 as a colorless crystal.

### Example 73:

### 2,2,6-Trimethyl-1-(piperidin-4-yl)indan-1-ol monofumarate:

The compound (1.04 g) of Reference Example 68 was dissolved in a mixed solvent of MeOH (10 ml), EtOH (10 ml) and water (10 ml), and KOH (15 g) was added, followed by stirring at 80°C for 2 hours. The reaction solution was extracted with ethyl acetate, washed with saturated brine, dried over anhydrous sodium sulfate, and the solvent was evaporated under reduced pressure. The residue was purified by silica gel column chromatography (eluent: chloroform/MeOH/aqueous saturated ammonia = 5/1/0.1) to obtain an amorphous substance (810 mg). This was formed into its salt with one equivalent of fumaric acid, and washed with diethyl ether to obtain the compound of Example 73 as a colorless crystal.
In Examples 74 and 75, the compounds shown in Table 25 were produced in the same manner as in Example 73.
In Examples 76 and 77, the compounds shown in Table 25 were produced in the same manner as in Example 78 given below.

### Example 78:

### 2,2,6-Trimethyl-1-(1-benzylpiperidin-4-yl)indan-1-ol monofumarate:

Acetic acid (0.44 ml) was added to a 1,2-dichloroethane (4 ml) solution of a desalted product (200 mg) of the compound of Example 73 and benzaldehyde (0.157 ml) at room temperature, followed by stirring for 15 minutes. Then, NaB(OAc)₃H (817 mg) was added, followed by further stirring for 1 hour. An aqueous saturated ammonia was added to the reaction solution, followed by extraction with ethyl acetate and washing with saturated brine. This was dried over anhydrous sodium sulfate, the solvent was evaporated under reduced pressure, and the residue was purified by silica gel column chromatography (eluent: chloroform/MeOH/aqueous saturated ammonia = 20/1/0.1). This was formed into its salt with one equivalent of fumaric acid, and washed with diethyl ether to obtain the compound (178 mg) of Example 78 as a crystal.
In Example 79, the compound shown in Table 26 was produced in the same manner as in Example 78.

### Example 80:

### 4-(1-Methoxy-2,2,6-trimethyl-2,3-dihydro-1H-inden-1-yl)-1-methylpiperidin monofumarate:

10-Camphorsulfonic acid (255 mg) was added to an MeOH (5 ml) solution of a desalted product (200 mg) of the compound of Example 6, followed by stirring at room temperature for 24 hours. The reaction liquid was subjected to liquid-liquid separation with an aqueous saturated sodium hydrogencarbonate solution and ethyl acetate. The organic layer was washed with saturated brine, dried over anhydrous sodium sulfate, and the solvent was evaporated under reduced pressure. The residue was purified by basic silica gel column chromatography (eluent: chloroform/MeOH/aqueous saturated ammonia = 10/1/0.1), then formed into its salt with one equivalent of fumaric acid, and recrystallized from MeOH/diethyl ether to obtain the compound (151 mg) of Example 80 as a colorless crystal.

### Example 81:

### [6-Fluoro-2,2-dimethyl-1-(1-methylpiperidin-4-yl)-2,3-dihydro-1H-inden-1-yl]amine monofumarate:

10% Pd-C (0.5 g) was added to an MeOH solution of a desalted product (2.29 g) of the compound of Example 82, and in a hydrogen atmosphere (normal pressure), this was stirred at room temperature for 20 hours. The insoluble matter was removed by filtration. The solvent was evaporated under reduced pressure, the residue was purified by silica gel column chromatography (eluent: chloroform/MeOH/aqueous saturated ammonia = 10/1/0.1), then formed into its salt with one equivalent of fumaric acid, and washed with diethyl ether to obtain the compound (1.81 g) of Example 81 as a colorless crystal.

### Example 82:

### 4-(1-Azido-6-fluoro-2,2-dimethyl-2,3-dihydro-1H-inden-1-yl)-1-methylpiperidine monofumarate:

With cooling on ice, a chloroform solution of trifluoroacetic acid (4.03 ml) and a desalted product (2.4 g) of the compound of Example 9 was added to a chloroform (15 ml) solution of NaN₃ (1.36 g), followed by stirring at room temperature for 4 hours. An aqueous saturated ammonia was added to the reaction solution, followed by extraction with chloroform, washing with water, and drying over anhydrous sodium sulfate. The solvent was evaporated under reduced pressure, and the residue was purified by silica gel column chromatography (eluent: chloroform/MeOH/aqueous saturated ammonia = 5/1/0.1) to obtain an oil (2.48 g). This was formed into its salt with one equivalent of fumaric acid, and washed with diethyl ether to obtain the compound of Example 82 as an amorphous substance.
In Examples 83, 84 and 85, the compounds shown in Tables 26 and 27 were produced in the same manner as in Examples 81, 82 and 6, respectively.
In Example 86, in the same manner as in Example 73; and in Example 87, in the same manner as in Example 6, the compounds shown in Table 27 were produced.

### Example 88:

### 2,2-Dimethyl-1-(-methylpiperidin-4-yl)-6-(piperidin-1-ylcarbonyl)indan-1-ol:

An aqueous 10% NaOH solution (17 ml) was added to an MeOH solution of a desalted product (345 mg) of the compound of Example 17, followed by stirring under heat at 60°C for 13 hours. After completion of the reaction, this was neutralized with 1N hydrochloric acid to have a pH of from 5 to 6, and the solvent was evaporated under reduced pressure. Next, the residue was dissolved in DMF, and then piperidine (0.6 ml), 1-hydroxybenzotriazole (164 mg), 1-ethyl-3-(3-dimethylaminopropyl)carbodiimide hydrochloride (232 mg) were added, followed by stirring for 1 day. Water and chloroform were added to the reaction solution for liquid-liquid separation, the organic layer was washed with saturated brine, and followed by drying over anhydrous sodium sulfate. The solvent was evaporated under reduced pressure, and the residue was purified by silica gel column chromatography (eluent: chloroform/methanol/aqueous saturated ammonia = 20/1/0.1 to 10/1/0.1) to obtain the compound (12 mg) of Example 88 as a colorless amorphous substance.

### Example 89:

### Benzyl 3-hydroxy-2,2-dimethyl-3-(1-methylpiperidin-4-yl)indane-5-carboxylate:

An aqueous 10% NaOH solution (8.4 ml) was added to an MeOH solution of a desalted product (600 mg) of the compound of Example 17, followed by stirring at 70°C for 21 hours. The reaction solution was subjected to liquid-liquid separation with water and chloroform, the aqueous layer was neutralized with 1 N hydrochloric acid to have a pH of 6 or so, and water was evaporated under heat under reduced pressure. The residue was dissolved in acetonitrile, and with cooling on ice, benzyl alcohol (2.2 ml), N,N-dimethylpyridine-4-amine (258 mg) and 1-ethyl-3-(3-dimethylaminopropyl)carbodiimide hydrochloride (445 mg) were added thereto, followed by stirring at room temperature for 18 hours. The reaction solution was subjected to liquid-liquid separation with chloroform and water, and the organic layer was washed with saturated brine, and dried over anhydrous sodium sulfate. The solvent was evaporated under reduced pressure, and the residue was purified by silica gel column chromatography (eluent: chloroform/MeOH/aqueous saturated ammonia = 10/1/0.1) to obtain the compound (8 mg) of Example 89 as a colorless amorphous substance.
In Example 90, the compound shown in Table 27 was produced in the same manner as in Example 6.
In Examples 91 and 100, the compounds shown in Tables 28 and 29 were produced in the same manner as in Example 6 but from the mixture of 4-fluoro-6-hydroxy-2,2,5-trimethylindan-1-one and 4-fluoro-6-hydroxy-2,2-dimethylindan-1-one obtained in Reference Example 92.
In Examples 92 to 94, in the same manner as in Example 6; and in Example 95, in the same manner as in Example 119, the compounds shown in Table 28 were produced.

### Example 96:

### 7-Ethyl-5-fluoro-2,2-dimethyl-1-(1-methylpiperidin-4-yl)indan-1-ol monofumarate:

In MeOH in the presence of 10% Pd-C in a hydrogen atmosphere having one atmospheric pressure, a desalted product (27 mg) of the compound of Example 116 was stirred at room temperature for 5 hours. The insoluble matter was removed by filtration using Celite. The solvent was evaporated under reduced pressure, and the residue was formed into its fumarate with one equivalent fumaric acid, and then washed with diethyl ether to obtain the compound (25 mg) of Example 96 as a colorless powder.
In Example 97, in the same manner as in Example 6 but from the compound of Reference Example 82; and in Examples 98, 99, 101 and 102, in the same manner as in Example 6, the compounds shown in Tables 29 and 30 were produced.

### Example 103:

### 5,7-Dibromo-6-fluoro-2,2-dimethyl-1-(1-methylpiperidin-4-yl)indan-1-ol:

With cooling on ice, SOCl₂ (0.89 ml) was gradually dropwise added to an MeCN suspension of the compound (1.1 g) of Example 153. This was stirred at that temperature for 1 hour, then the solvent was evaporated under reduced pressure with cooling with water. The residue was dissolved in acetic acid, and Zn powder (0.8 g) was added, followed by stirring under heat at 60°C for 2 hours. Zn was removed by filtration using Celite, acetic acid was evaporated under reduced pressure, and the residue was subjected liquid-liquid separation with a diluted aqueous ammonia and chloroform. The chloroform layer was dried over anhydrous sodium sulfate, and the solvent was evaporated under reduced pressure. The residue was purified by silica gel column chromatography (eluent: chloroform/MeOH/aqueous saturated ammonia = 20/1/0.1) to obtain the compound (436 mg) of Example 103 as a colorless amorphous substance.
In Examples 104 to 106, in the same manner as in Example 6; in Example 107, in the same manner as in Example 119; and in Examples 108 and 109, in the same manner as in Example 6, the compounds shown in Tables 30 and 31 were produced.

### Example 110:

### 4-Fluoro-6-hydroxynlethyl-7-methoxy-2,2-dimethyl-1-(1-methylpiperidin-4-yl)indan-1-ol:

A THF solution of a desalted product (207 mg) of the compound of Example 113 was cooled to -70°C, and 1 M i-Bu₂AlH/toluene solution (3.1 ml) was dropwise added, followed by stirring at -70°C for 1 hour. Then, it was heated to room temperature, and further heated for 4 hours. Na₂SO₄•10H₂O was added to the reaction solution and stirred, then the insoluble matter was removed by filtration using Celite, and the solvent was evaporated under reduced pressure. The residue was dissolved in MeOH, and with cooling on ice, NaBH₄ (24 mg) was added thereto, followed by stirring at that temperature for 30 minutes. The reaction solution was concentrated under reduced pressure, and the residue was purified by silica gel column chromatography (eluent: chloroform/MeOH/aqueous saturated ammonia = 10/1/0.1) to obtain the compound (59 mg) of Example 110 as an amorphous substance.

### Example 111:

### 4-Fluoro-7-methoxy-2,2-dimethyl-1-(1-methylpiperidin-4-yl)-6-(morpholin-4-ylmethyl)indan-1-ol:

Morpholine (0.07 ml) and i-Pr₂NEt (0.01 ml) were added to a THF suspension of the compound (30 mg) of Example 112, followed by stirring at room temperature for 7 hours. The reaction solution was concentrated under reduced pressure, and the residue was purified by silica gel column chromatography (eluent: chloroform/MeOH/aqueous saturated ammonia = 10/1/0.1) to obtain the compound (12 mg) of Example 111 as a colorless amorphous substance.

### Example 112:

### 6-Chloromethyl-4-fluoro-7-methoxy-2,2-dimethyl-1-(1-methylpiperidin-4-yl)indan-1-ol hydrochloride:

An MeCN solution of the compound (52 mg) of Example 110 was cooled at 0°C, and then SOCl₂ (0.06 ml) was gradually added, followed by stirring for 1.5 hours. After completion of the reaction, the solvent was evaporated under reduced pressure to obtain the compound (61 mg) of Example 112 as an amorphous substance.
In Example 113, in the same manner as in Example 119; in Example 114, in the same manner as in Example 6; and in Example 115, as the side product of Example 114, the compounds shown in Table 32 were produced.

### Example 116:

### 4-Fluoro-2,2-dimethyl-1-(1-methylpiperidin-4-yl)-7-vinylindan-1-ol fumarate:

Water (1 ml) was added to a toluene (2 ml) suspension of the compound (200 mg) of Example 36, 2-vinyl-4,4,5,5-tetramethyl-1,3,2-dioxaborolan (108 mg), Na₂CO₃ (180 mg) and Pd(PPh₃)₄ (58 mg), followed by heating overnight under reflux. The reaction solution was subjected to liquid-liquid separation with chloroform and an aqueous saturated sodium hydrogencarbonate solution, the organic layer was dried over anhydrous sodium sulfate, and the solvent was evaporated under reduced pressure. The residue was purified by silica gel column chromatography (eluent: chloroform/MeOH/aqueous saturated ammonia = 10/1/0.1), then formed into its fumarate with one equivalent of fumaric acid, and washed with diethyl ether to obtain the compound of Example 116 as a colorless powder.

### Example 117:

### 4-Fluoro-2,2-dimethyl-1-(1-methylpiperidin-4-yl)-7-phenylindan-1-ol fumarate:

Water (0.5 ml) was added to a toluene (1 ml) suspension of the compound (102 mg) of Example 36, PhB(OH)₂ (43 mg), Na₂CO₃ (95 mg) and Pd(PPh₃)₄ (35 mg), followed by heating under reflux for one day. The reaction solution was subjected to liquid-liquid separation with chloroform and an aqueous saturated sodium hydrogencarbonate solution, the organic layer was dried over anhydrous sodium sulfate, and the solvent was evaporated under reduced pressure. The residue was purified by silica gel column chromatography (eluent: chloroform/MeOH/aqueous saturated ammonia = 10/1/0.1), then formed into its fumarate with one equivalent of fumaric acid, and recrystallized from isopropanol to obtain the compound (38 mg) of Example 117 as a colorless crystal.
In Example 118, the compound shown in Table 23 was produced in the same manner as in Example 117.

### Example 119:

### 7-Cyano-4-fluoro-2,2-dimethyl-1-(1-methylpiperidin-4-yl)indan-1-ol fumarate:

A 1-methyl-2-pyrrolidinone solution of the compound (107 mg) of Example 36, Zn(CN)₂ (47 mg), Pd(PPh₃)₄ (69 mg) and Ca(OH)₂ (30 mg) was stirred under heat at 180°C for 8 hours. The reaction liquid was cooled, and then subjected to liquid-liquid separation with chloroform and saturated sodium hydrogencarbonate. The organic layer was dried over anhydrous sodium sulfate. The solvent was evaporated under reduced pressure, and the residue was purified by silica gel column chromatography (eluent: chloroform/MeOH/aqueous saturated ammonia =10/1/0.1), then formed into its fumarate with one equivalent of fumaric acid, and washed under heat with MeCN to obtain the compound (19 mg) of Example 119 as a colorless crystal.
In Examples 120 to 122, in the same manner as in Example 117; in Example 123, as a side product in Example 124; and in Example 124, in the same manner as in Example 103, the compounds shown in Table 34 were produced.

### Example 125:

### 1-(Ambomethyl)-4-fluoro-7-methoxy-2,2-dimethylindan-1-ol fumarate:

A THF solution of the compound (700 mg) of Reference Example 120 was added to a THF suspension of LiAlH₄ (175 mg), followed by stirring under heat at 60°C for 2 hours. The reaction solution was diluted with THF, then water (0.7 ml) and an aqueous 15% sodium hydroxide (0.175 ml) solution were added, followed by stirring. Then, the insoluble matter was removed by filtration using Celite. The solvent was evaporated under reduced pressure, the residue was dissolved in THF, and then water (2 ml) and NaF (200 mg) were added, followed by stirring overnight at room temperature. An aqueous saturated sodium hydrogencarbonate solution was added to the reaction liquid, followed by extraction with ethyl acetate, washing with saturated brine, and drying over anhydrous sodium sulfate. The solvent was evaporated under reduced pressure, and the residue was purified by silica gel column chromatography (eluent: chloroform/MeOH/aqueous saturated ammonia = 20/1/0.1), then formed into its fumarate with one equivalent of fumaric acid, and washed with ethyl acetate/MeCN to obtain the compound (72 mg) of Example 125 as a colorless crystal.

### Example 126:

### 4-Fluoro-7-methoxy-2,2-dimethyl-1-(dimethylamino)indan-1-ol fumarate:

Formalin (0.4 ml) and formic acid (0.2 ml) were added to a THF solution of a desalted product (97 mg) of the compound of Example 125, followed by stirring under heat at 80°C for 7 hours. The reaction solution was subjected to liquid-liquid separation with an aqueous saturated sodium hydrogencarbonate solution and ethyl acetate, the organic layer was dried over anhydrous sodium sulfate, and the solvent was evaporated under reduced pressure. The residue was purified by silica gel column chromatography (eluent: chloroform/MeOH/aqueous saturated ammonia = 20/1/0.1), then formed into its fumarate with one equivalent of fumaric acid, and washed with ethyl acetate/acetone to obtain the compound (72 mg) of Example 126 as a colorless crystal.
In Examples 127 to 130, the compounds shown in Tables 34 and 35 were produced in the same manner as in Example 132 but from the compound of Reference Example 122.

### Example 131:

### 1-(2-Amino-2-methylpropyl)-2,2,6-trimethylindan-1-ol fumarate:

With cooling on ice, a THF solution of the compound (400 mg) of Reference Example 125 was added to a THF suspension of LiA1H₄ (186 mg), followed by stirring at room temperature for 1 hour. Na₂SO₄·10H₂O was added to the reaction solution, followed by stirring. Then, the insoluble matter was removed by filtration using Celite, and the solvent was evaporated under reduced pressure. The residue was purified by silica gel column chromatography (eluent: chloroform/MeOH/aqueous saturated ammonia = 50/1/0.1 to 10/1/0.1), then formed into its fumarate with one equivalent of fumaric acid, and recrystallized from ethyl acetate/acetone to obtain the compound (128 mg) of Example 131 as a colorless crystal.

### Example 132:

### 6-Fluoro-7-methoxy-2,2-dimethyl-1-[3-(piperidin-1-yl)propyl]indan-1-ol fumarate:

MsCl (0.11 ml) was added to a THF solution of the compound (120 mg) of Reference Example 127 and i-Pr₂NEt (0.4 ml) at room temperature, followed by stirring at that temperature for 1 hour. The reaction solution was dried into solid under reduced pressure, then the residue was dissolved in DMF. Piperidine (0.25 ml) was added, followed by stirring under heat at 80°C for 3 hours. The reaction solution was subjected liquid-liquid separation with water and ethyl acetate, and the organic layer was washed with saturated brine, and dried over anhydrous sodium sulfate. The solvent was evaporated under reduced pressure, and the residue was purified by silica gel column chromatography (eluent: chloroform/MeOH/aqueous saturated ammonia = 20/1/0.1), and then formed into its fumarate with one equivalent of fumaric acid to obtain the compound (24 mg) of Example 132 as an amorphous substance.
In Examples 133 and 134, the compounds shown in Tables 35 and 36 were produced in the same manner as in Example 132.

### Example 135:

### 6-Fluoro-7-methoxy-2,2-dimethyl-1-[(1-methylpiperidin-2-yl)methyl]indan-1-ol fumarate:

A THF solution of the compound (355 mg) of Reference Example 128 and MeI (1 ml) was heated under reflux for 1.5 hours. The solvent was evaporated under reduced pressure, the residue was dissolved in ethanol, then NaBH₄ (267 mg) was added, followed by stirring at 60°C for 2 hours. The reaction solution was cooled, an aqueous sodium hydrogencarbonate solution was added thereto, followed by extraction with ethyl acetate and drying over anhydrous sodium sulfate. The solvent was evaporated under reduced pressure, and the residue was purified by silica gel column chromatography (eluent: chloroform/MeOH/aqueous saturated ammonia = 20/1/0.2). Then, the resulting product was dissolved in EtOH, followed by stirring overnight in a hydrogen atmosphere (one atmospheric pressure) in the presence of 10% Pd-C at room temperature. The insoluble matter was removed by filtration using Celite, and the solvent was evaporated under reduced pressure to obtain a product. This was formed into its fumarate with one equivalent of fumaric acid to obtain the compound (212 mg) of Example 135 as an amorphous substance.

### Example 136:

### 6-Fluoro-7-methoxy-2,2-dimethyl-1-{5-[(dimethylamino)methyl]-2-thienyl}-indan-1-ol fumarate:

1.6 M n-BuLi/hexane solution (0.9 ml) was added to a THF solution of 2-(dimethylaminomethyl)thiophene (203 mg) in an argon atmosphere at -78°C, followed by stirring at that temperature for 1 hour. To this was added a THF solution of the compound (100 mg) of Reference Example 64, followed by further stirring for 1 hour. Water was added to the reaction solution, followed by extraction with ethyl acetate and drying over anhydrous sodium sulfate. The solvent was evaporated under reduced pressure, and the residue was purified by silica gel column chromatography (eluent: chloroform/MeOH/aqueous saturated ammonia = 20/1/0.1), and then formed into its fumarate with one equivalent of fumaric acid to obtain the compound (121 mg) of Example 136 as an amorphous substance.
In Examples 137 and 138, the compounds shown in Table 36 were produced in the same manner as in Example 6.

### Example 139:

### 5-Fluoro-7,7-dimethyl-8-(1-methylpiperidin-4-yl)-2,6,7,8-tetrahydro-2H-indeno [4,5-b]furan-8-ol fumarate:

10% Pd-C was added to an MeOH solution of a desalted product (500 mg) of the compound of Example 140, followed by stirring in a hydrogen atmosphere (1 atmospheric pressure) at room temperature for 5 hours. The insoluble matter was removed by filtration using Celite, and the solvent was evaporated under reduced pressure. This was formed into its fumarate with one equivalent of fumaric acid, and then crystallized from THF to obtain the compound (536 mg) of Example 139 as a colorless crystal.

### Example 140:

### 5-Fluoro-7,7-dimethyl-8-(1-methylpiperidin-4-yl)-7,8-dihydro-6H-indeno[4,5-b]furan-8-ol fumarate:

A THF solution (20 ml) of 0.7 M (N-methylpiperidin-4-yl)magnesium chloride was added to a THF solution of the compound (2.0 g) of Reference Example 134, followed by stirring at room temperature for 1 hour. A THF solution (24 ml) of 1 M (n-Bu)₄NF was added to it, followed by further stirring at room temperature for 1 hour. An aqueous saturated ammonium chloride solution was added, followed by extraction with ethyl acetate and drying over anhydrous sodium sulfate. The solvent was evaporated under reduced pressure, and the resulting crude product was purified by recrystallization from ethyl acetate, formed into its fumarate with one equivalent of fumaric acid, and recrystallized from THF to obtain the compound of Example 140 as a colorless crystal.
In Examples 141 and 142, the compounds shown in Table 37 were produced in the same manner as in Example 6.

### Example 143:

### 4-(9-Fluoro-5,5-dimethyl-2,3,5,6-tetrahydro-4aH-indeno[1,7-ef][1,4]dioxepin-4a-yl-1-methylpiperidine fumarate:

3N hydrochloric acid (9 ml) and concentrated hydrochloric acid (2.3 ml) were added to a THF solution of the compound (910 mg) of Example 142, followed by stirring under heat at 60°C for 3 days. The reaction solution was neutralized with an aqueous NaOH solution, followed by extraction with ethyl acetate and drying over anhydrous sodium sulfate. The solvent was evaporated under reduced pressure, and the residue was purified by silica gel column chromatography (eluent: chloroform/MeOH/aqueous saturated ammonia = 20/1/0.1), then formed into its fumarate with one equivalent of fumaric acid, and recrystallized from acetone to obtain the compound (320 mg) of Example 143 as a colorless crystal.

### Example 144:

### 1-(6'-fluoro-7'-methoxy-2',2'-dimethyl-2',3',4,5-tetrahydro-3H-spiro[furan-2,1'-inden]-5-yl)-N,N-dimethylmethanamine fumarate:

An aqueous 50% Me₂NH solution (1 ml) was added to a DMF solution of the compound (200 g) of Reference Example 140, followed by stirring under heat at 80°C for 3 hours. The reaction solution was subjected to liquid-liquid separation with water and ethyl acetate, and the organic layer was washed with saturated brine and dried over anhydrous sodium sulfate. The solvent was evaporated under reduced pressure, and the residue was purified by silica gel column chromatography (eluent: chloroform/MeOH/aqueous saturated ammonia = 20/1/0.1), and formed into its fumarate with one equivalent of fumaric acid to obtain the compound (182 mg) of Example 144 as an amorphous substance.
In Example 145, in the same manner as in Example 144; and in Example 146, in the same manner as in Example 6, the compounds shown in Table 38 were produced.

### Example 147:

### 1-Methyl-4-(7-methyl-2,3,3 a,4-tetrahydro-8bH-indeno[1,2-b]furan-8b-yl)piperidine fumarate:

An aqueous 3N HCl solution (15 ml) was added to a THF solution of the compound (1.59 g) of Example 146, and vigorously stirred for 2 hours. An aqueous 1N NaOH solution was added to the reaction solution so as to make it alkaline, followed by extraction with ethyl acetate and drying over anhydrous sodium sulfate. The solvent was evaporated under reduced pressure, and this was formed into its fumarate with one equivalent of fumaric acid, and recrystallized from MeOH/diethyl ether to obtain the compound (1.41 g) of Example 147 as a colorless crystal.
In Example 148, the compound shown in Table 38 was produced in the same manner as in Reference Example 149.

### Reference Example 149:

### 4,6-Dibromo-5-fluoro-3-hydroxy-2,2-dimethyl-3-(1-methylpiperidin-4-yl)indan-1-one:

A 1,2-dichloroethane suspension of the compound (39 g) of Reference Example 96 and PhI(OAc)₂ (47 g) was stirred at 40°C for 3 hours. After it was confirmed that the generation of nitrogen ended completely, saturated sodium hydrogencarbonate and an aqueous 5% sodium thiosulfate solution were added, followed by stirring for liquid-liquid separation. The organic layer was dried over anhydrous sodium sulfate, and the solvent was evaporated under reduced pressure. Then, the residue was dissolved in THF, and t-BuOK (9.7 g) was added thereto and stirred at 70°C for 3 hours. After completion of the reaction, the reaction solution was concentrated under reduced pressure, and subjected to liquid-liquid separation with ethyl acetate and saturated brine. The organic layer was washed with saturated brine, then dried over anhydrous sodium sulfate, and the solvent was evaporated under reduced pressure. Ethyl acetate (200 ml) and n-hexane (500 ml) were added to the residue, followed by stirring. Then, the insoluble matter was collected by filtration, and washed with diethyl ether to obtain the compound (20 g) of Reference Example 149 as a pale brown powder.
In Example 150, in the same manner as in Example 149; and in Example 151, in the same manner as in Example 153, the compounds shown in Tables 38 and 39 were produced.

### Example 152:

### Cis-7-bromo-4-fluoro-2,2-dimethyl-1-(1-methylpiperidin-4-yl)indane-1,3-diol fumarate:

The compound (6.5 g) of Example 11 was dissolved in 2 N hydrochloric acid (100 ml), followed by heating under reflux for 5 hours. With cooling on ice, this was neutralized with an aqueous ammonia added thereto, followed by extraction with chloroform and drying over anhydrous sodium sulfate. Then, the solvent was evaporated under reduced pressure and isopropanol was added to a mixture of the residue and trans-2,3-bis(benzoyloxy)succinic acid (6.3 g), followed by stirring. The starting trans-diol precipitated out as a salt, and the precipitate was removed by filtration. The filtrate was concentrated under reduced pressure, subjected to liquid-liquid separation with diluted an aqueous ammonia and chloroform, the chloroform layer was dried over anhydrous sodium sulfate, and the solvent was evaporated under reduced pressure. The residue was formed into its fumarate with fumaric acid (1.4 g), and crystallized from acetone to obtain the compound (4.25 g) of Example 152 as a colorless crystal.

### Example 153:

### Trans-5,7-dibromo-6-fluoro-2,2-dimethyl-1-(1-methylpiperidin-4-yl)indane-1,3-diol:

A THF (200 ml) suspension of LiAlH₄ was cooled to -78°C, and with stirring, a THF (50 ml) solution of the compound (20 g) of Example 149 was dropwise added thereto, taking 15 minutes. After the addition, the cooling bath was removed, and the reaction liquid was warmed to 0°C, taking 1 hour. With cooling on ice, Na₂SO₄•10H₂O was added, followed by stirring. Then, the insoluble matter was removed by filtration using Celite. The filtrate was distilled under reduced pressure, and the residue was purified by silica gel column chromatography (eluent: chloroform/MeOH/aqueous saturated ammonia = 20/1/0.1 to 5/1/0.1) to obtain the compound (3.2 g) of Example 153 as an amorphous substance.

### Example 154:

### Trans-7-bromo-4-fluoro-2,2-dimethyl-1-(1-methyl-1,2,3,6-tetrahydropyridin-4-yl)indane-1,3-diol:

MeI (0.52 ml) was added to a THF solution of the compound (1.04 g) of Reference Example 144, followed by heating under reflux for 6 hours. The solvent was evaporated under reduced pressure to obtain 4-(7-bromo-4-floro-1-hydroxy-2,2-dimethyl-3-oxo-2,3-dihydro-1H-inden-1-yl)-1-methylpyridinium iodide (1.46 g) as an amorphous substance. This was dissolved in MeOH, and at -10°C, NaBH₄ (545 mg) was added, followed by stirring at that temperature for 1 hour. The reaction solution was diluted with water, and the precipitated matter was collected by filtration to obtain the compound (640 mg) of Example 154 as a colorless crystal.

### Example 155:

### Cis-7-bromo-4-fluoro-2,2-dimethyl-1-(1-methyl-1,2,3,6-tetrahydropyridin-4-yl)indane-1,3-diol:

A few drops of water were added to a methanol solution of 4-(7-bromo-4-fluoro-1-hydroxy-2,2-dimethyl-3-oxo-2,3-dihydro-1H-inden-1-yl)-1-methylpyridinium iodide (285 mg) obtained in the same manner as in Reference Example 154 and CeCl₃ (858 mg), followed by stirring, and at -10°C, NaBH₄ (132 mg) was added to it and stirred for 3 hours. Water was added to the reaction solution, followed by extraction with chloroform and drying over anhydrous sodium sulfate. The solvent was evaporated under reduced pressure to obtain the compound of Example 155 as a colorless amorphous substance (70 mg).
In Example 156, the compound shown in Table 40 was produced in the same manner as in Example 153.

### Example 157:

### 3-Hydroxy-2,2-dimethyl-3-(1-methylpiperidin-4-yl)indane-5-carboxylic acid:

An EtOH solution of the compound (31 mg) of Example 89 and 1,4-cyclohexadiene (0.6 ml) was stirred in an argon atmosphere in the presence of 10% Pd-C at room temperature for 1 hour. After completion of the reaction, this was filtered using Celite to remove the insoluble matter, and the solvent was evaporated under reduced pressure to obtain the compound (24 mg) of Example 157 as a colorless powder.

### Example 158:

### 7-Methoxy-2,2-dimethyl-1-(1-methylpiperidin-4-yl)-6-nitro-1,2,3,4-tetrahydronaphthalen-1-ol fumarate:

An Ac₂O (1.0 ml) solution of the compound (100 mg) of Example 54 was cooled to 0°C, and an Ac₂O (1.0 ml) solution of fuming nitric acid (16 mg) was gradually added thereto. This was stirred for 30 minutes, then fuming nitric acid (30 mg) was further added thereto, followed by stirring at that temperature for 30 minutes. Ice-water was added to the reaction solution, followed by stirring. Then, it was subjected to liquid-liquid separation with chloroform and an aqueous saturated sodium hydrogencarbonate solution, and the organic layer was washed with saturated brine. This was dried over anhydrous sodium sulfate, and the solvent was evaporated under reduced pressure to obtain a mixture of 7-methoxy-2,2-dimethyl-1-(1-methylpiperidin-4-yl)-6-nitro-1,2,3,4-tetrahydronaphthalen-1-ol and 7-methoxy-2,2-dimethyl-1-(1-methylpiperidin-4-yl)-8-nitro-1,2,3,4-tetrahydronaphthalen-1-ol. This was formed into its fumarate with one equivalent of fumaric acid, and recrystallized from EtOB to obtain the compound of Example 158 as a colorless crystal.

### Example 159:

### 7-Amino-6-methoxy-2,2-dimethyl-1-(1-methylpiperidin-4-yl)-1,2,3,4-tetrahydronaphthalen-1-ol, and

### Example 160:

### N-[5-hydroxy-2-methoxy-6,6-dimethyl-5-(1-methylpiperidin-4-yl)-5,6,7, 8-tetrahydronaphthalen-1-yl]acetamide:

10% Pd-C was added to an EtOH/ethyl acetate mixed solution of a mixture (779 mg) 6-methoxy-2,2-dimethyl-1-(1-methylpiperidin-4-yl)-7-nitro-1,2,3,4-tetrahydronaphthalen-1-ol and 6-methoxy-2,2-dimethyl-1-(1-methylpiperidin-4-yl)-5-nitro-1,2,3,4-tetrahydronaphthalen-1-ol that had been obtained through nitration of the compound of Example 55 in the same manner as in Example 158, followed by stirring in a hydrogen atmosphere (1 atmospheric pressure) at room temperature for 6 hours. After completion of the reaction, the insoluble matter was removed by filtration using Celite, and the solvent was evaporated under reduced pressure. The residue was purified by silica gel column chromatography (eluent: chloroform/MeOH/aqueous saturated ammonia = 10/1/0.1 to obtain the compound (167 mg) of Example 159 and the compound (230 mg) of Example 160 as amorphous substances. (The formation of the compound of Example 160 would result from the residual acetic anhydride in the starting compound.)
In Example 161, the compound shown in Table 40 was produced in the same manner as in Example 126 but from the compound of Example 159.

The structural formulae and the physicochemical data of the compounds of the above Reference Examples and the compounds of the above Examples are shown in the following Tables 2 to 40. The compounds shown in Tables 41 to 45 may be readily produced similarly to the above Examples or Production Methods or according to the modifications apparent to one skilled in the art. The symbols in the Tables have the following meanings.
- Rf.: Reference Example
- Ex.: Example
- STRUCTURE: structural formula
- DATA: physicochemical data
- SALT: salt
- Bn: benzyl group
- Me: methyl group
- Et: ethyl group
- NMR: nuclear magnetic resonance spectrum (TMS internal standard)
- MS: mass spectrum
- FA: fumaric acid

**[Table 2]**

| Rf. | STRUCTURE | DATA | Rf. | STRUCTURE | DATA |
|---|---|---|---|---|---|
| 1 | 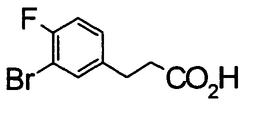 | FAB-MS (m/z); 245, 247 [(M-1)⁺] | 2 | 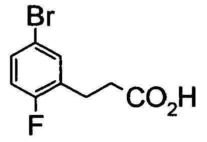 | FAB-MS (m/z); 245, 247 [(M-1)⁺] |
| 3 | 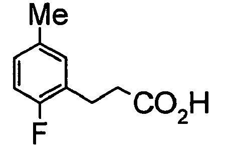 | FAB-MS (m/z); 181 [(M-1)⁺] | 4 | 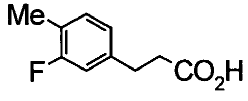 | FAB-MS (m/z); 181 [(M-1)⁺] |
| 5 | 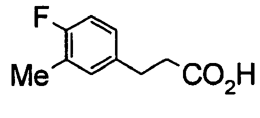 | FAB-MS (m/z); 181 [(M-1)⁺] | 6 | 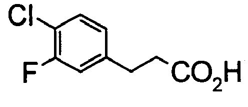 | ¹H-NMR (CDCl₃); 2.67(2H, t) 2.93(2H, t) |
| 7 | 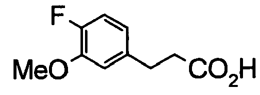 | FAB-MS (m/z); 197 [(M-1)⁺] | 8 | 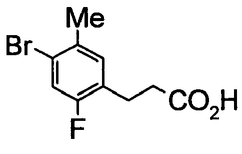 | FAB-MS (m/z); 259, 261 [(M-1)⁺] |
| 9 | 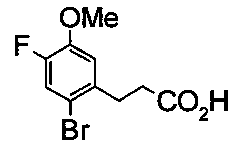 | FAB-MS (m/z); 275, 277 [(M-1)⁺] | 10 | 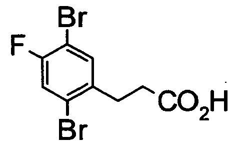 | ¹H-NMR (CDCl₃); 2.69(2H, t) 3.02(2H, t) |
| 11 | 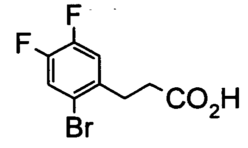 | FAB-MS (m/z); 263, 265 [(M-1)⁺] | 12 | 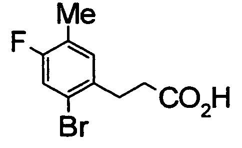 | FAB-MS (m/z); 259, 261 [(M-1)⁺] |
| 13 | 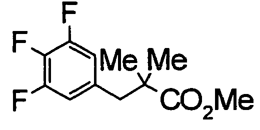 | EI-MS (m/z); 246 [M⁺] | 14 | 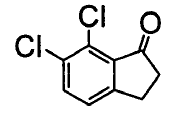 | FAB-MS (m/z); 201 [(M+1)⁺] |
| 15 | 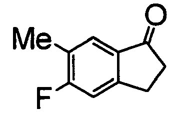 | EI-MS (m/z); 164 [M⁺] | 16 | 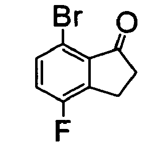 | EI-MS (m/z); 228, 230 [M⁺] |

**[Table 3]**

| Rf. | STRUCTURE | DATA | Rf. | STRUCTURE | DATA |
|---|---|---|---|---|---|
| 17 | 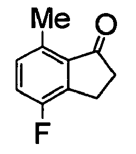 | EI-MS (m/z); 164 [M⁺] | 18 | 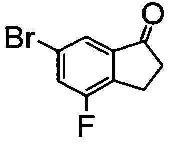 | EI-MS (m/z); 228, 230 [M⁺] |
| 19 | 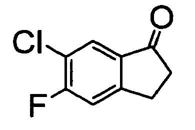 | EI-MS (m/z); 184 [M⁺] | 20 | 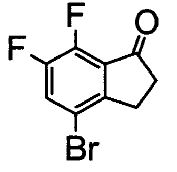 | EI-MS (m/z); 246, 248 [M⁺] |
| 21 | 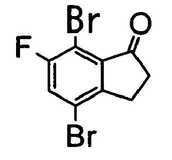 | EI-MS (m/z); 306, 308, 310 [M⁺] | 22 | 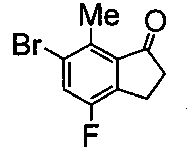 | EI-MS (m/z); 242, 244 [M⁺] |
| 23 | 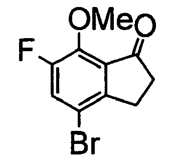 | EI-MS (m/z); 258, 260 [M⁺] | 24 | 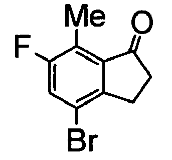 | EI-MS (m/z); 242, 244 [M⁺] |
| 25 | 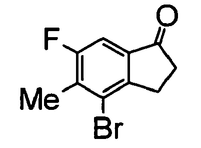 | EI-MS (m/z); 242, 244 [M⁺] | 26 | 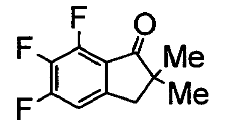 | EI-MS (m/z); 214 [M⁺] |
| 27 | 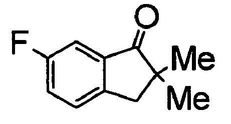 | EI-MS (m/z); 178 [M⁺] | 28 | 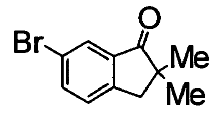 | EI-MS (m/z); 238, 240 [M⁺] |
| 29 | 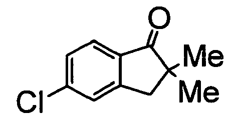 | FAB-MS (m/z); 195 [(M+1)⁺] | 30 | 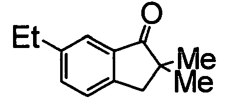 | FAB-MS (m/z); 189 [(M+1)⁺] |
| 31 | 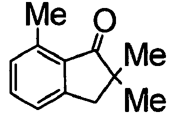 | FAB-MS (m/z); 175 [(M+1)⁺] | 32 | 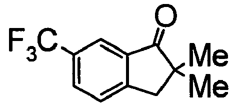 | EI-MS (m/z); 228 [M⁺] |

**[Table 4]**

| Rf. | STRUCTURE | DATA | Rf. | STRUCTURE | DATA |
|---|---|---|---|---|---|
| 33 | 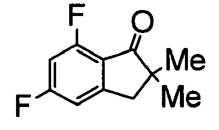 | FAB-MS (m/z); 197 [(M+1)⁺] | 34 | 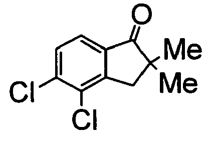 | FAB-MS (m/z); 229, 231 [(M+1)⁺] |
| 35 | 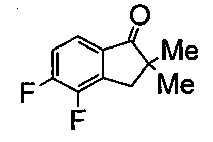 | EI-MS (m/z); 196 [M⁺] | 36 | 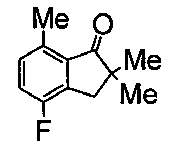 | EI-MS (m/z); 192 [M⁺] |
| 37 | 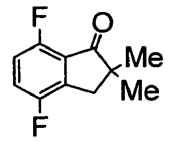 | ¹H-NMR (CDCl₃); 1.26(6H,s),2 .99(2H,s) | 38 | 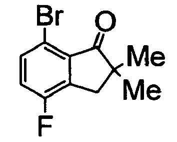 | EI-MS (m/z); 256, 258 [M⁺] |
| 39 | 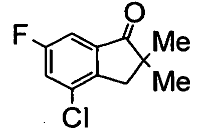 | EI-MS (m/z); 212 [M⁺] | 40 | 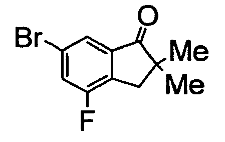 | EI-MS (m/z); 256, 258 [M⁺] |
| 41 | 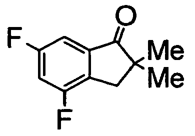 | EI-MS (m/z); 196 [M⁺] | 42 | 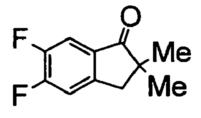 | EI-MS (m/z); 196 [M⁺] |
| 43 | 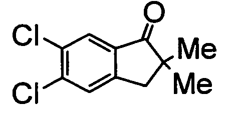 | FAB-MS (m/z); 229, 231 [(M+1)⁺] | 44 | 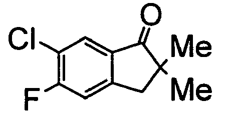 | EI-MS (m/z); 212 [M⁺] |
| 45 | 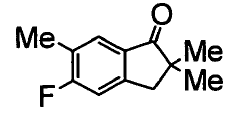 | EI-MS (m/z); 192 [M⁺] | 46 | 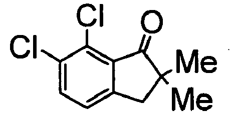 | EI-MS (m/z); 229, 231 [M⁺] |
| 47 | 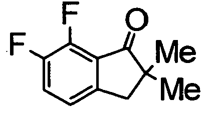 | ¹H-NMR (CDCl₃); 1.25(6H,s),2 .96(2H,s) | 48 | 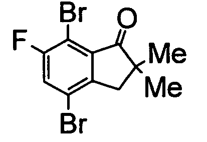 | FAB-MS (m/z); 335, 337, 339 [(M+1)⁺] |

**[Table 5]**

| Rf. | STRUCTURE | DATA | Rf. | STRUCTURE | DATA |
|---|---|---|---|---|---|
| 49 | 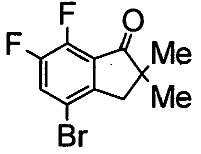 | ¹H-NMR (CDCl₃); 1.27(6H,s),2 .89(2H,d) | 50 | 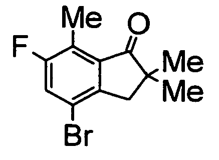 | FAB-MS (m/z); 271, 273 [(M+1)⁺] |
| 51 | 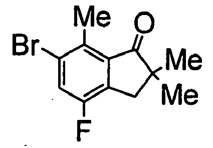 | EI-MS (m/z); 270, 272 [M⁺] | 52 | 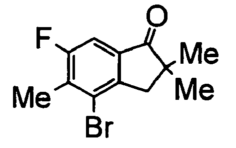 | ¹H-NMR (CDCl₃); 1.25(6H,s), 2.44(3H,d), 2.90(2H,s) |
| 53 | 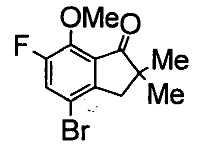 | FAB-MS (m/z); 287, 289 [(M+1)⁺] | 54 | 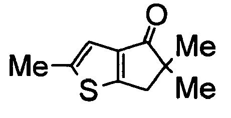 | EI-MS (m/z); 180 [M⁺] |
| 55 | 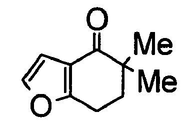 | EI-MS (m/z); 164 [M⁺] | 56 | 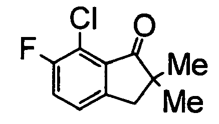 | EI-MS (m/z); 212 [M⁺] |
| 57 | 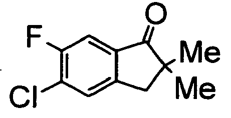 | EI-MS (m/z); 212 [M⁺] | 58 | 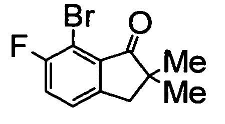 | EI-MS (m/z); 256, 258 [M⁺] |
| 59 | 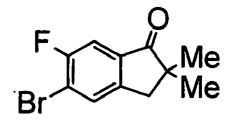 | EI-MS(m/z); 256, 258 [M⁺] | 60 | 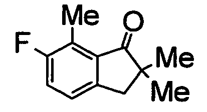 | EI-MS (m/z); 192 [M⁺] |
| 61 | 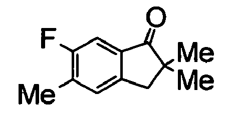 | EI-MS(m/z); 192 [M⁺] | 62 | 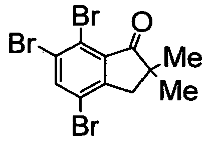 | FAB-MS (m/z); 397, 399 [(M+1)⁺] |
| 63 | 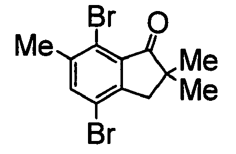 | FAB-MS (m/z); 331, 333, 335 [(M+1)⁺] | 64 | 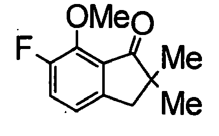 | EI-MS(m/z); 208 [M⁺] |

**[Table 6]**

| Rf. | STRUCTURE | DATA | Rf. | STRUCTURE | DATA |
|---|---|---|---|---|---|
| 65 | 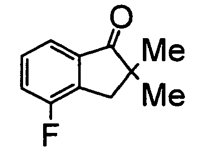 | EI-MS(m/z); 178 [M⁺] | 66 | 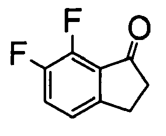 | ¹H-NMR (CDCl₃); 2.74-2.79 (2H, m), 3.10-3.15 (2H, m) |
| 67 | 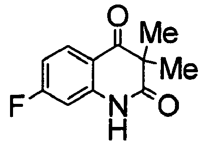 | FAB-MS(m/z); 208 [(M+1)⁺] | 68 | 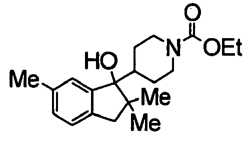 | FAB-MS(m/z); 332 [(M+1)⁺] |
| 69 | 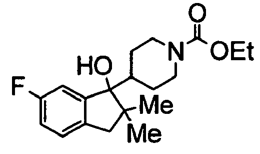 | FAB-MS(m/z); 336 [(M+1)⁺] | 70 | 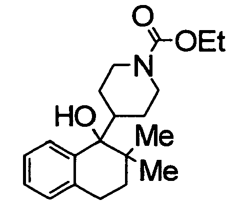 | FAB-MS(m/z); 332 [(M+1)⁺] |
| 71 | 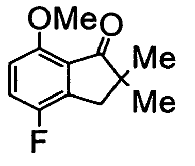 | EI-MS(m/z); 208 [M⁺] | | | |

**[Table 7]**

| Rf. | STRUCTURE | DATA | Rf. | STRUCTURE | DATA |
|---|---|---|---|---|---|
| 72 | 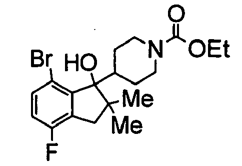 | FAB-MS(m/z); 414,416 [(M+1)⁺] | 73 | 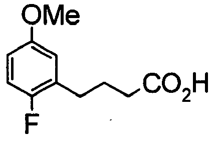 | FAB-MS(m/z); 211 [(M-1)⁺] |
| 74 | 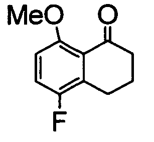 | FAB-MS(m/z); 195 [(M+1)⁺] | 75 | 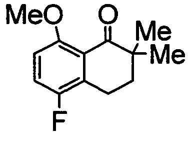 | FAB-MS (m/z); 223 [(M+1)⁺] |
| 76 | 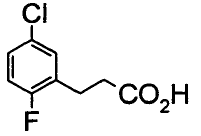 | FAB-MS(m/z); 201 [(M-1)⁺] | 77 | 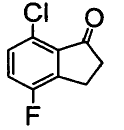 | EI-MS(m/z); 184 [M⁺] |
| 78 | 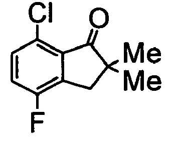 | EI-MS(m/z); 212 [M⁺] | 79 | 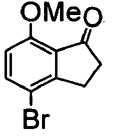 | EI-MS(m/z); 240, 242 [M⁺] |
| 80 | 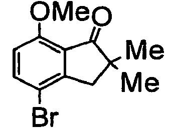 | FAB-MS(m/z); 269, 271 [(M+1)⁺] | 81 | 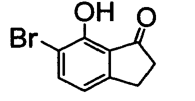 | FAB-MS(m/z); 227, 229 [(M+1)⁺] |
| 82 | 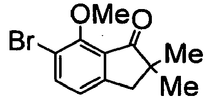 | ESI-MS(m/z); 269, 271 [(M+1)⁺] | 83 | 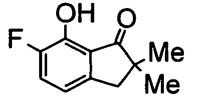 | FAB-MS(m/z); 195 [(M+1)⁺] |
| 84 | 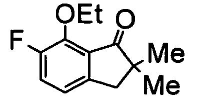 | EI-MS(m/z); 222 [M⁺] | 85 | 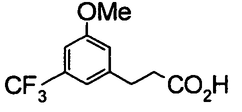 | EI-MS(m/z); 248 [M⁺] |

**[Table 8]**

| Rf. | STRUCTURE | DATA | Rf. | STRUCTURE | DATA |
|---|---|---|---|---|---|
| 86 | 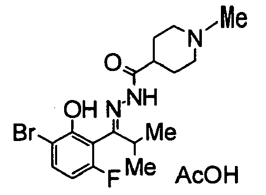 | FAB-MS(m/z); 400, 402 [(M+1)⁺] | 87 | 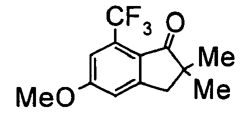 | FAB-MS(m/z); 259 [(M+1)⁺] |
| 88 | 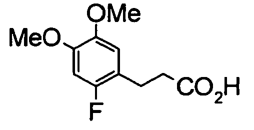 | ESI-MS(m/z); 227 [(M-1)⁺] | 89 | 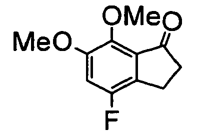 | ESI-MS(m/z); 211 [(M+1)⁺] |
| 90 | 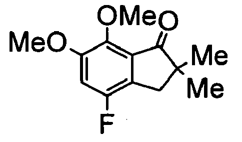 | FAB-MS(m/z); 239 [(M+1)⁺] | 91 | 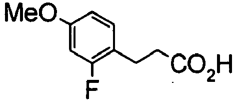 | FAB-MS(m/z); 197 [(M-1)⁺] |
| 92 | 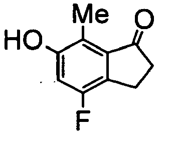 | FAB-MS(m/z); 181 [(M+1)⁺] | 93 | 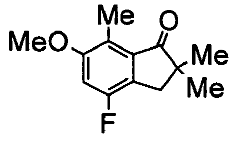 | EI-MS(m/z); 222 [M⁺] |
| 94 | 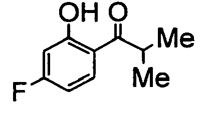 | EI-MS(m/z); 182 [M⁺] | 95 | 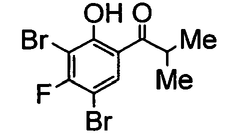 | FAB-MS(m/z); 337, 339, 341 [(M-1)⁺] |
| 96 | 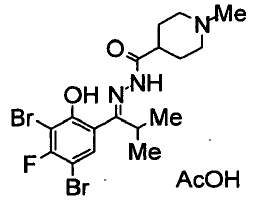 | FAB-MS(m/z); 478, 480, 482 [(M+1)⁺] | 97 | 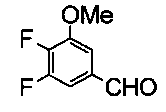 | EI-MS(m/z); 172 [M⁺] |
| 98 | 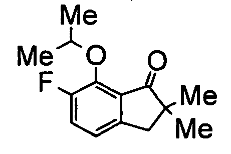 | EI-MS(m/z); 236 [M⁺] | 99 | 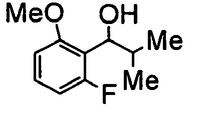 | EI-MS(m/z); 198 [M⁺] |

**[Table 9]**

| Rf. | STRUCTURE | DATA | Rf. | STRUCTURE | DATA |
|---|---|---|---|---|---|
| 100 | 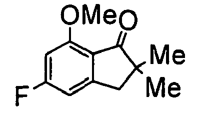 | EI-MS(m/z); 208 [M⁺] | 101 | 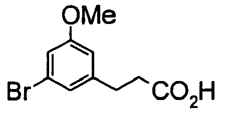 | ¹H-NMR (DMSO-d₃); 3.75(3H, s) |
| 102 | 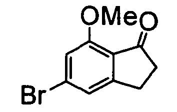 | EI-MS(m/z); 240, 242 [M⁺] | 103 | 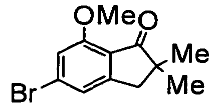 | FAB-MS (m/z); 369, 371 [(M+1)⁺] |
| 104 | 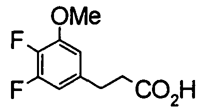 | FAB-MS(m/z); 215 [(M-1)⁺] | 105 | 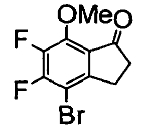 | EI-MS(m/z); 276, 278 [M⁺] |
| 106 | 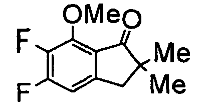 | ¹H-NMR (CDCl₃); 1.21 (6H, s) | 107 | 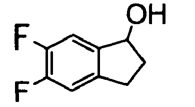 | EI-MS(m/z); 170 [M⁺] |
| 108 | 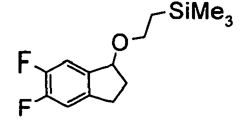 | ¹H-NMR (CDCl₃); 0.01 (9H, s) | | | |
| 110 | 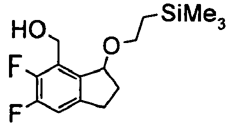 | ¹H-NMR (CDCl₃); 0.04(9H, s) | 111 | 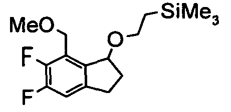 | FAB-MS(m/z); 345 [(M+1)⁺] |
| 112 | 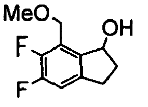 | EI-MS(m/z); 214 [M⁺] | 113 | 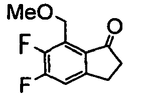 | FAB-MS(m/z); 213 [(M+1)⁺] |
| 114 | 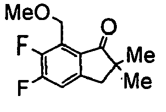 | EI-MS(m/z); 240 [M⁺] | 115 | 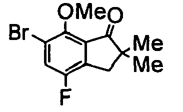 | EI-MS(m/z); 286, 288 [M⁺] |

**[Table 10]**

| Rf. | STRUCTURE | DATA | Rf. | STRUCTURE | DATA |
|---|---|---|---|---|---|
| 116 | 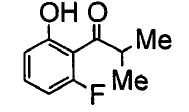 | EI-MS(m/z); 182 [M⁺] | 117 | 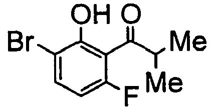 | FAB-MS(m/z); 261, 263 [(M+1)⁺] |
| 118 | 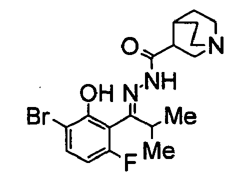 | FAB-MS(m/z); 412, 414 [(M+1)⁺] | 119 | 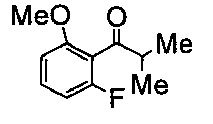 | FAB-MS(m/z); 197 [(M+1)⁺] |
| 120 | 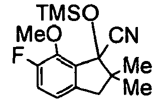 | ESI-MS(m/z); 330 [(M+Na)⁺] | 121 | 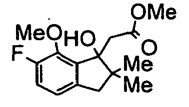 | ESI-MS(m/z); 305 [(M+Na)⁺] |
| 122 | 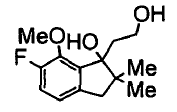 | ESI-MS(m/z); 277 [(M+Na)⁺] | 123 | 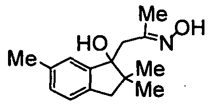 | FAB-MS(m/z); 248 [(M+1)⁺] |
| 124 | 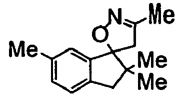 | FAB-MS(m/z); 230 [(M+1)⁺] | 125 | 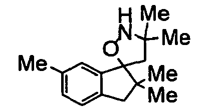 | FAB-MS(m/z); 246 [(M+1)⁺] |
| 126 | 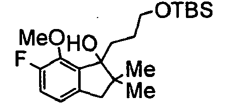 | ESI-MS(m/z); 405 [(M+Na)⁺] | 127 | 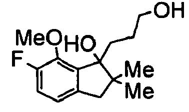 | ESI-MS(m/z); 291 [(M+Na)⁺] |
| 128 | 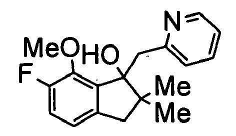 | FAB-MS(m/z); 302 [(M+1)⁺] | 129 | 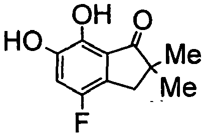 | ESI-MS(m/z); 209 [(M-1)⁺] |
| 130 | 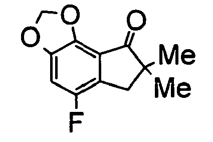 | ESI-MS(m/z); 223 [(M+1)⁺] | 131 | 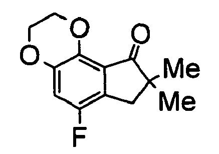 | ESI-MS(m/z); 237 [(M+1)⁺] |

**[Table 11]**

| Rf. | STRUCTURE | DATA | Rf. | STRUCTURE | DATA |
|---|---|---|---|---|---|
| 132 | 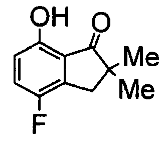 | ESI-MS(m/z); 195 [(M+1)⁺] | 133 | 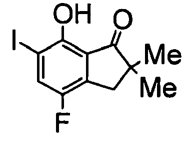 | ESI-MS(m/z); 319 [(M-1)⁺] |
| 134 | 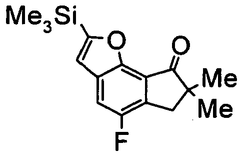 | ESI-MS(m/z); 291 [(M+1)⁺] | 135 | 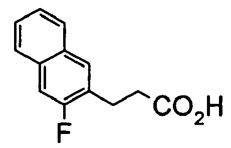 | API-MS(m/z); 217 [(M-1)⁺] |
| 136 | 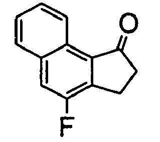 | ESI-MS(m/z); 201 [(M+1)⁺] | 137 | 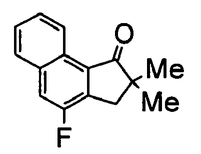 | ESI-MS(m/z); 229 [(M+1)⁺] |
| 138 | 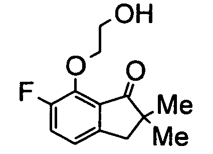 | ESI-MS(m/z); 239 [(M+1)⁺] | 139 | 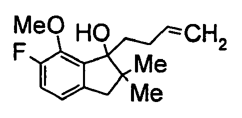 | ESI-MS(m/z); 287 [(M+Na)⁺] |
| 140 | 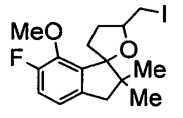 | ESI-MS(m/z); 413 [(M+Na)⁺] | 141 | 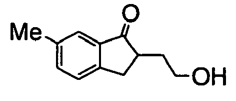 | ESI-MS(m/z); 191 [(M+1)⁺] |
| 142 | 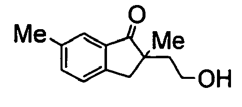 | ESI-MS(m/z); 227 [(M+Na)⁺] | 143 | 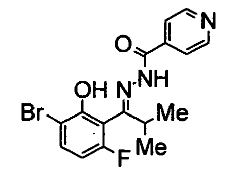 | FAB-MS(m/z); 380, 382 [(M+1)⁺] |
| 144 | 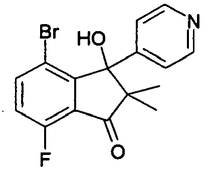 | FAB-MS(m/z); 350, 352 [(M+1)⁺] | | | |

**[Table 12]**

| Ex. | STRUCTURE | SALT | DATA |
|---|---|---|---|
| 1 | 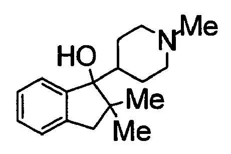 | 1FA | FAB-MS(m/z); 260 [M+1] ¹H-NMR(DMSO-d₃); 0.65-0.80(1H, m), 0.84(3H, s), 1.17(3H, s), 1.45-1.70(2H, m), 1.70-1.85(1H, m), 2.00-2.10(1H, m), 2.25-2.45(2H, m), 2.36(3H, s), 2.50(1H, d, J=16.2 Hz), 2.77(1H, d, J=16.2 Hz), 2.92(1H, brd, J=11.7 Hz), 3.14(1H, brd, J=11.7 Hz), 4.72(br), 6.44(2H, s), 7.07-7.20(4H, m). |
| 2 | 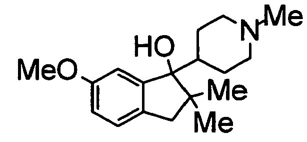 | 1FA | FAB-MS(m/z); 290 [(M+1)⁺] |
| 3 | 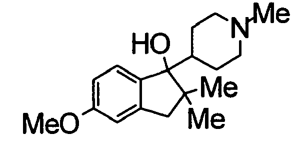 | 1FA | FAB-MS(m/z); 290 [(M+1)⁺] |
| 4 | 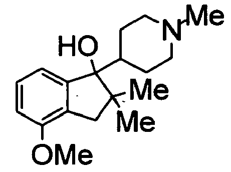 | 1FA | FAB-MS(m/z); 290 [(M+1)⁺] |
| 5 | 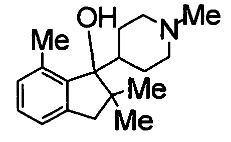 | 1FA | FAB-MS(m/z); 274 [(M+1)⁺] |
| 6 | 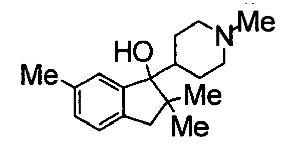 | 1FA | FAB-MS(m/z); 274[(M+1)⁺] ¹H-NMR(DMSO-d₃); 0.62-0.76(1H, m), 0.84(3H, s), 1.16(3H, s), 1.46-1.59(1H, m), 1.60-1.68(1H, m), 1.71-1.81(1H, m), 2.01-2.09(1H, m), 2.28(3H, s), 2.33(3H, s), 2.23-2.38(2H, m), 2.45(1H, d, 15.6 Hz), 2.73(1H, d, J=15.6 Hz), 2.87(1H, brd, J=11.7 Hz), 3.11(1H, brd, J=11.7 Hz), 6.47(2H, s), 6.97(1H, d, J=8.3 Hz), 6.98(1H, s), 7.01(1H, d, J=8.3 Hz) |

**[Table 13]**

| Ex. | STRUCTURE | SALT | DATA |
|---|---|---|---|
| 7 | 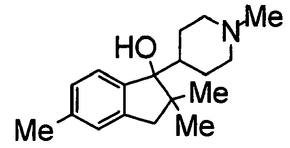 | 1FA | FAB-MS(m/z); 274 [(M+1)⁺] |
| 8 | 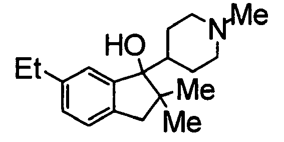 | 1FA | FAB-MS(m/z); 288 [(M+1)⁺] |
| 9 | 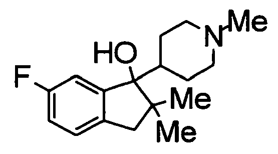 | 1FA | FAB-MS(m/z); 278 [(M+1)⁺] ¹H-NMR(DMSO-d₃); 0.67-0.82(1H, m), 0.85(3H, s), 1.17(3H, s), 1.44-1.59(1H, m), 1.65(1H, brd, J=13.6 Hz), 1.74-1.85(1H, m), 2.04(1H, brd, J=13.6 Hz), 2.26-2.43(2H, m), 2.36(3H, s), 2.48(1H, d, J=15.7 Hz), 2.74(1H, d, J=15.7 Hz), 2.92(1H, brd, J=11.7 Hz), 3.14(1H, brd, J=11.7 Hz), 6.46(2H, s), 6.90(1H, dd, J=3.0, 9.3 Hz), 6.93-7.01(1H, m), 7.15(1H, dd, J=5.4, 8.3 Hz). |
| 10 | 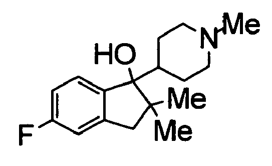 | 1FA | FAB-MS(m/z); 278 [(M+1)⁺] ¹H-NMR(DMSO-d₃); 0.65-0.83(1H, m), 0.85(3H, s), 1.17(3H, s), 1.45-1.60(1H, m), 1.64(1H, brd, J=13.6 Hz), 1.73-1.85(1H, m), 2.04(1H, brd, J=13.2 Hz), 2.28-2.46(2H, m), 2.37(3H, s), 2.51(1H, d, J=16.1 Hz), 2.77(1H, d, J=16.1 Hz), 2.94(1H, brd, J=11.7 Hz), 3.15(1H, brd, J=11.7 Hz), 5.01(br), 6.46(2H, s), 6.90-7.00(2H, m), 7.12-7.20(1H, m). |
| 11 | 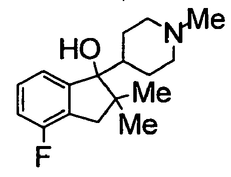 | 1FA | FAB-MS(m/z); 278 [(M+1)⁺] ¹H-NMR(DMSO-d₃); 0.64-0.80(1H, m), 0.87(3H, s), 1.20(3H, s), 1.42-1.60(1H, m), 1.65(1H, brd, J=13.7 Hz), 1.72-1.88(1H, m), 2.05(1H, brd, J=13.7 Hz), 2.26-2.46(2H, m), 2.36(3H, s), 2.59(1H, d, J=16.1 Hz), 2.72(1H, d, J=16.1 Hz), 2.93(1H, brd, J=10.2 Hz), 3.14(1H, brd, J=10.7 Hz), 4.82(br), 6.45(2H, s), 6.94-7.06(2H, m), 7.18-7.26(1H, m). |

**[Table 14]**

| Ex. | STRUCTURE | SALT | DATA |
|---|---|---|---|
| 12 | 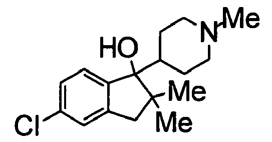 | 1FA | FAB-MS(m/z); 294 [(M+1)⁺] ¹H-NMR(DMSO-d₃); 0.65-0.83(1H, m), 0.85(3H, s), 1.17(3H, s), 1.44-1.58(1H, m), 1.64(1H, brd, 13.2 Hz), 1.73-1.85(1H, m), 2.04(1H, brd, J=13.2 Hz), 2.29-2.44(2H, m), 2.37(3H, s), 2.51(1H, d, J=16.1 Hz), 2.78(1H, d, J=16.1 Hz), 2.93(1H, brd, J=11.2 Hz), 3.14(1H, brd, J=11.2 Hz), 6.47(2H, s), 7.14-7.23(3H, m). |
| 13 | 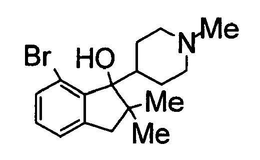 | 1FA | FAB-MS(m/z); 338, 340 [(M+1)⁺] ¹H-NMR(DMSO-d₃); 0.86(3H, s), 1.19(3H, s), 1.26-1.44(1H, m), 1.58-1.82(3H, m), 1.88-2.02(1H, m), 2.10-2.30(2H, m), 2.33(3H, s), 2.46(1H, d, J=16.2 Hz), 2.80(1H, d, J=16.2 Hz), 2.97(1H, brd, J=11.8 Hz), 3.05(1H, brd, J=11.8 Hz), 3.45(br), 4.61(1H, br), 6.50(2H, s), 7.09(1H, t, J=7.6 Hz), 7.16(1H, J=7.3Hz), 7.34(1H, d, J=7.8 Hz). |
| 14 | 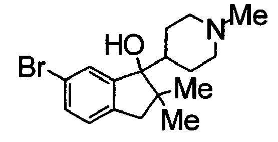 | 1FA | FAB-MS(m/z); 338, 340 [(M+1)⁺] |
| 15 | 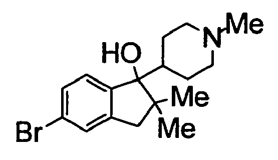 | 1FA | FAB-MS(m/z); 338, 340 [(M+1)⁺] ¹H-NMR(DMSO-d₃); 0.62-0.76(1H, m), 0.84(3H, s), 1.17(3H, s), 1.38-1.54(1H, m), 1,56-1.68(1H, m), 1.70-1.82(1H, m), 1.96-2.08(1H, m), 2.17-2.37(5H, m), 2.46-2.55(1H, m), 2,78(1H, d, J=16.2 Hz), 2.82-2.94(1H, m), 3.02-3.14(1H, m), 6.49(2H, s), 7.11(1H, d, J=8.8 Hz), 7.30-7.38(2H, m). |
| 16 | 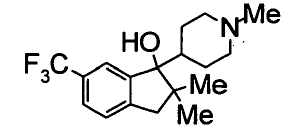 | 1FA | FAB-MS(m/z); 328 [(M+1)⁺] |

**[Table 15]**

| Ex. | STRUCTURE | SALT | DATA |
|---|---|---|---|
| 17 | 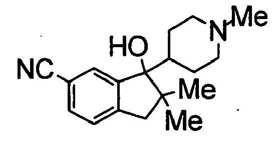 | 1FA | FAB-MS(m/z); 285 [(M+1)⁺] |
| 18 | 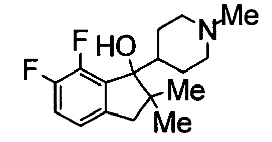 | 1FA | FAB-MS(m/z); 296 [(M+1)⁺] |
| 19 | 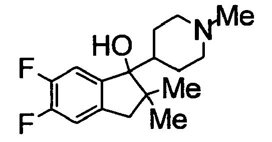 | 1FA | FAB-MS(m/z); 296 [(M+1)⁺] |
| 20 | 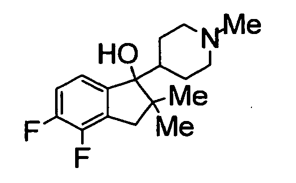 | 1FA | FAB-MS(m/z); 296 [(M+1)⁺] ¹H-NMR(DMSO-d₃); 0.65-0.83(1H, m), 0.87(3H, s), 1.19(3H, s), 1.42-1.57(1H, m), 1.65(1H, brd, J=13.2 Hz), 1.74-1.86(1H, m), 2.03(1H, brd, J=13.6 Hz), 2.28-2.44(2H, m), 2.36(3H, s), 2.63(1H, d, J=16.1 Hz), 2.75(1H, d, J=16.1 Hz), 2.93(1H, brd, J=11.2 Hz), 3.13(1H, brd, J=11.8 Hz), 6.46(2H, s), 6.98(1H, dd, J=4.4, 7.8 Hz), 7.15-7.25(1H, m). |
| 21 | 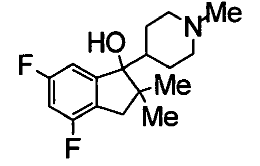 | 1FA | FAB-MS(m/z); 296 [(M+1)⁺] |
| 22 | 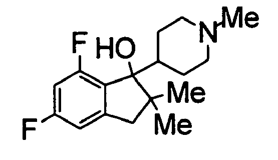 | 1FA | FAB-MS(m/z);296 [(M+1)⁺] ¹H-NMR(DMSO-d₃); 0.90(3H, s), 0.90-1.05(1H, m), 1.15(3H, m), 1.44-1.62(1H, m), 1.62-1.76(1H, m), 1.78-1.94(1H, m), 1.98-2.16(1H, m), 2.30-2.46(2H, m), 2.40(3H, s), 2.56(1H, d, J=16.1 Hz), 2.78(1H, d, J=16.1 Hz), 2.99(1H, brd, J=10.8 Hz), 3.16(1H, brd, J=10.8 Hz), 4.90(br), 6.46(2H, s), 6.80-6.94(2H, m). |

**[Table 16]**

| Ex. | STRUCTURE | SALT | DATA |
|---|---|---|---|
| 23 | 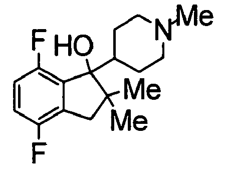 | 1FA | FAB-MS(m/z); 296 [(M+1)⁺] |
| 24 | 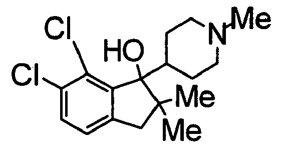 | 1FA | FAB-MS(m/z); 328, 330 [(M+1)⁺] |
| 25 | 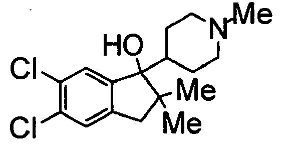 | 1FA | FAB-MS(m/z); 328, 330 [(M+1)⁺] |
| 26 | 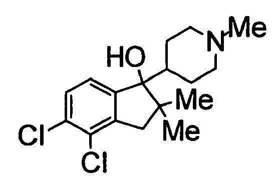 | 1FA | FAB-MS(m/z); 328, 330 [(M+1) ⁺] ¹H-NMR(DMSO-d₃); 0.60-0.78(1H, m), 0.87(3H, s), 1.20(3H, s), 1.40-1.55(1H, m), 1.63(1H, brd, J=13.2 Hz), 1.75-1.85(1H, m), 2.02(1H, brd, J=13.6 Hz), 2.20-2.42(2H, m), 2.34(3H, s), 2.60(1H, d, J=16.6 Hz), 2.81(1H, d, J=16.6 Hz), 2.92(1H, brd), 3.12(1H, brd), 5.02(br), 6.48(2H, s), 7.14(1H, d, J=7.8 Hz), 7.45(1H, d, J=7.8 Hz). |
| 27 | 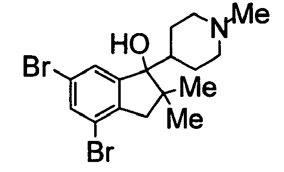 | - | FAB-MS(m/z); 416, 418, 420 [(M+1)⁺] |

**[Table 17]**

| Ex. | STRUCTURE | SALT | DATA |
|---|---|---|---|
| 28 | 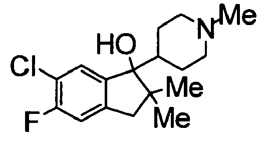 | 1FA | FAB-MS(m/z); 312 [(M+1)⁺] ¹H-NMR(DMSO-d₃); 0.66-0.83(1H, m), 0.85(3H, m), 1.17(3H, m), 1.42-1.58(1H, m), 1.66(1H, brd, J=13.2 Hz), 1.74-1.86(1H, m), 2.03(1H, brd, J=13.7 Hz), 2.29-2.45(2H, m), 2.37(3H, s), 2.51(1H, d, J=16.1 Hz), 2.77(1H, d, J=16.1 Hz), 2.94(1H, brd, J=11,8 Hz), 3.16(1H, brd, J=11.8 Hz), 6.47(2H, s), 7.22(1H, d, J=9.7 Hz), 7.23(1H, d, J=7.3 Hz). |
| 29 | 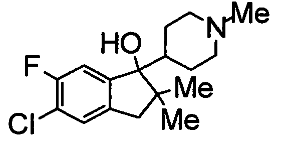 | 1FA | FAB-MS(m/z); 312 [(M+1)⁺] |
| 30 | 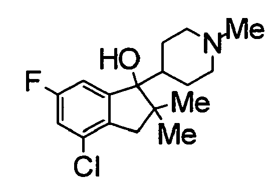 | 1FA | FAB-MS(m/z); 312 [(M+1)⁺] ¹H-NNM(DMSO-d₃); 0.65-0.80(1H, m), 0.87(3H, s), 1.19(3H, s), 1.44-1.56(1H, m), 1.65(1H, brd, J=13.2 Hz), 1.76-1.87(1H, m), 2.02(1H, brd, J=13.2 Hz), 2.28-2.44(2H, m), 2.37(3H, s), 2.54(1H, d, J=16.1 Hz), 2.72(1H, d, J=16.1 Hz), 2.94(1H, brd, J=11.2 Hz), 3.14(1H, brd, J=11.7 Hz), 6.46(2H, s), 6.93(1H, dd, J=2.4, 8.8 Hz), 7.22(1H, dd, J=2.4, 9.3 Hz). |
| 31 | 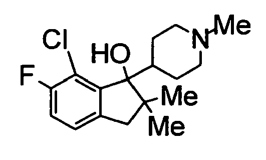 | 1FA | FAB-MS(m/z); 312 [(M+1)⁺] ¹H-NMR(DMSO-d₃); 0.87(3H, s), 1.17(3H, s), 1.18-1.32(1H, m), 1.60-1.74(2H, m), 1.83-2.00(2H, m), 2.20-2.31(2H, m), 2.35(3H, s), 2.45(1H, d, J=15.6 Hz), 2.77(1H, d, J=15.6 Hz), 2.97(1H, brd, J=11.5 Hz), 3.08(1H, brd, J=11.5 Hz), 4.85(br), 6.49(2H, s), 7.11-7.21(2H, m). |
| 32 | 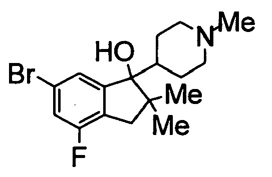 | 1FA | FAB-MS(m/z); 356, 358 [(M+1)⁺] ¹H-NMR(DMSO-d₃); 0.65-0.79(1H, m), 0.86(3H, s), 1.19(3H, s), 1.38-1.54(1H, m), 1.60-1.73(1H, m), 1.73-1.85(1H, m), 1.98-2.08(1H, m), 2.25-2.43(2H, m), 2.35(3H, s), 2.56(1H, d, J=16.2 Hz), 2.68(1H, d, J=16.2 Hz), 2.92(1H, brd, J=11.2 Hz), 3.13(1H, brd, J=11.2 Hz), 6.48(2H, s), 7.15(1H, d, J=2.0 Hz), 7.34(1H, dd, J=2.0, 8.8 Hz). |

**[Table 18]**

| Ex. | STRUCTURE | SALT | DATA |
|---|---|---|---|
| 33 | 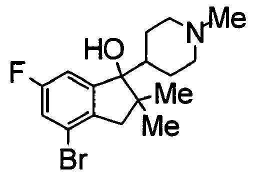 | 1FA | FAB-MS(m/z); 356, 358 [(M+1)⁺] |
| 34 | 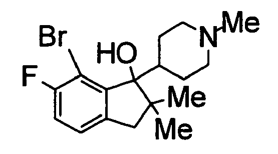 | 1FA | FAB-MS(m/z); 356, 358 [(M+1)⁺] ¹H-NMR(DMSO-d₃); 0.86(3H, s), 1.18(3H, s), 1.25-1.45(1H, m), 1.60-1.85(3H, m), 1.92-2.04(1H, m), 2.15-2.30(2H, m), 2.34(3H, s), 2.43(1H, d, J=15.7 Hz), 2.78(1H, d, J=15.7 Hz), 2.99(1H, brd, J=11.4 Hz), 3.07(1H, brd, J=11.4 Hz), 4.78(1H, br), 6.49(2H, s), 7.10-7.22(2H, m). |
| 35 | 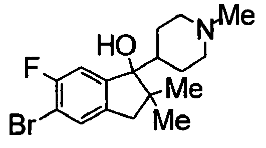 | 1FA | FAB-MS(m/z); 356, 358 [(M+1)⁺] |
| 36 | 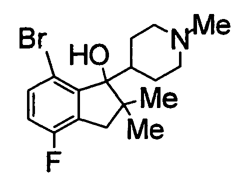 | 1FA | FAB-MS(m/z); 356, 358 [(M+1)⁺] ¹H-NMR(DMSO-d₃); 0.87(3H, s), 1.20(3H, s), 1.21-1.36(1H, m), 1.57-1.73(2H, m), 1.73-1.84(1H, m), 1.87-1.99(1H, m), 2.07-2.21(2H, m), 2.31(3H, s), 2.52(1H, d, J=16.1 Hz), 2.73(1H, d, J=16.1 Hz), 2.94(1H, brd, J=11.2 Hz), 3.03(1H, brd, J=11.2 Hz), 3.34(br), 4.79(1H, br), 6.51(2H, s), 7.00(1H, t, J=8.3 Hz), 7.39(1H, dd, J=8.8, 4.8 Hz).. |
| 37 | 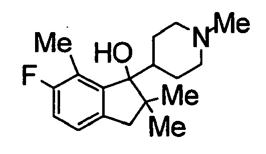 | 1FA | FAB-MS(m/z); 292 [(M+1)⁺] ¹H-NMR(DMSO-d₃); 0.85(3H, s), 1.00-1.16(1H, m), 1.17(3H, s), 1.46-1.67(2H, m), 1.78-1.98(2H, m), 2.31(3H, d, J=2.4 Hz), 2.38(3H, s), 2.28-2.42(3H, m), 2.73(1H, d, J=15.7 Hz), 2.98(1H, brd, J=11.2 Hz), 3.14(1H, brd, J=11.2 Hz), 4.77(br), 6.47(2H, s), 6.86-6.98(2H, m). |
| 38 | 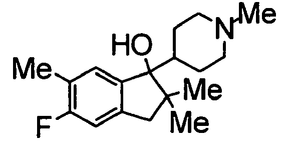 | 1FA | FAB-MS(m/z); 292 [(M+1)⁺] |

**[Table 19]**

| Ex. | STRUCTURE | SALT | DATA |
|---|---|---|---|
| 39 | 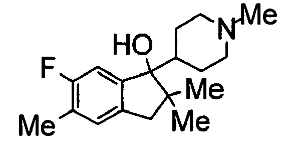 | 1FA | FAB-MS(m/z); 292 [(M+1)⁺] |
| 40 | 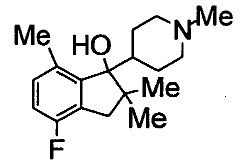 | 1FA | FAB-MS(m/z); 292 [(M+1)⁺] |
| 41 | 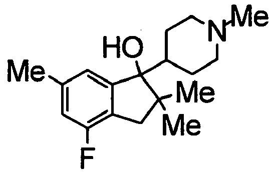 | 1FA | FAB-MS(m/z); 292 [(M+1)⁺] |
| 42 | 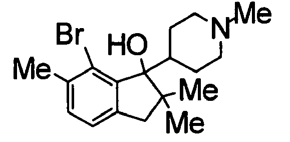 | 1FA | FAB-MS(m/z); 352, 354 [(M+1)⁺] |
| 43 | 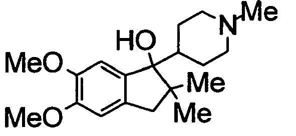 | 1FA | FAB-MS(m/z); 320 [(M+1)⁺] |
| 44 | 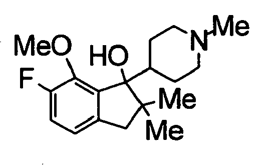 | 1FA | FAB-MS(m/z); 308 [(M+1)⁺] ¹H-NMR(DMSO-d₃); 0.92(3H, s), 1.00-1.20(1H, m), 1.14(3H, s), 1.57-1.72(2H, m), 1.82-1.98(2H, m), 2.25-2.36(2H, m), 2.37(3H, s), 2.47(1H, d, J=15.6 Hz), 2.68(1H, d, J=15.6 Hz), 2.97(1H, brd, J=11.7 Hz), 3.11(1H, brd, J=11.7 Hz), 3.82(3H, d, J=2.0 Hz), 4.47(1H, br), 6.48(2H, s), 6.85(1H, dd, J=8.3, 4.4 Hz), 7.04(1H, dd, J=11.7, 8.3 Hz). |

**[Table 20]**

| Ex. | STRUCTURE | SALT | DATA |
|---|---|---|---|
| 45 | 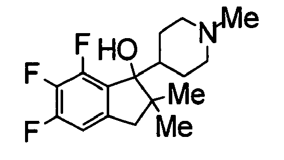 | 1FA | FAB-MS(m/z); 314 [(M+1)⁺] ¹H-NMR(DMSO-d₃); 0.84-1.00(1H, m), 0.89(3H, s), 1.15(3H, s), 1.38-1.56(1H, m), 1.60-1.74(1H, m), 1.76-1.90(1H, m), 2.04-2.14(1H, m), 2.18-2.38(2H, m), 2.32(3H, s), 2.53(1H, d, J=16.1 Hz), 2.78(1H, d, J=16.1 Hz), 2.90(1H, brd, J=10.3 Hz), 3.09(1H, brd, J=10.3 Hz), 3.35(br), 5.03(1H, br), 6.49(2H, s), 7.13(1H, dd, J=9.3, 6.8 Hz). |
| 46 | 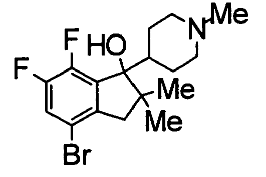 | 1FA | FAB-MS(m/z); 374, 376 [(M+1)⁺] |
| 47 | 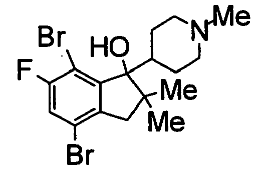 | - | FAB-MS(m/z); 434, 436, 438[(M+1)⁺] |
| 48 | 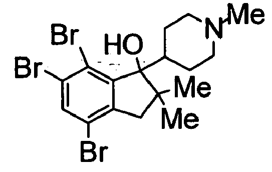 | 1FA | FAB-MS(m/z); 494, 496, 498, 500 [(M+1)⁺] |
| 49 | 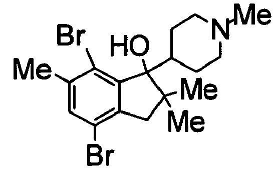 | 1FA | FAB-MS(m/z); 430, 432, 434 [(M+1)⁺] |
| 50 | 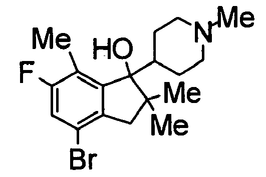 | 1FA | FAB-MS(m/z); 370, 372 [(M+1)⁺] |

**[Table 21]**

| Ex. | STRUCTURE | SALT | DATA |
|---|---|---|---|
| 51 | 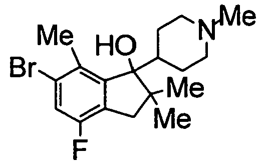 | 1FA | FAB-MS(m/z); 370, 372 [(M+1)⁺] |
| 52 | 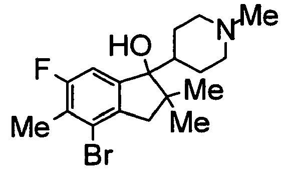 | 1FA | FAB-MS(m/z); 370, 372 [(M+1)⁺] |
| 53 | 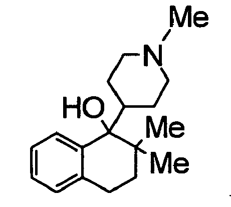 | - | FAB-MS(m/z); 274 [(M+1)⁺] |
| 54 | 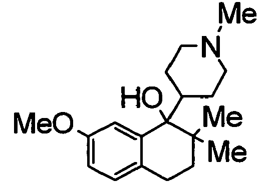 | 1FA | FAB-MS(m/z); 304 [(M+1)⁺] |
| 55 | 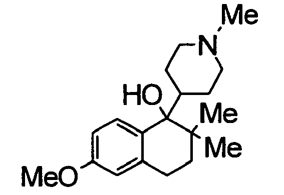 | 1FA | FAB-MS(m/z); 304 [(M+1)⁺] |

**[Table 22]**

| Ex. | STRUCTURE | SALT | DATA |
|---|---|---|---|
| 56 | 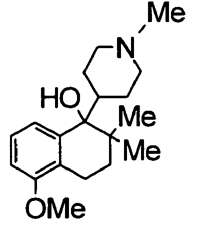 | 1FA | FAB-MS(m/z); 304 [(M+1)⁺] |
| 57 | 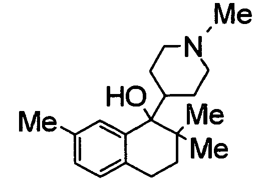 | 1FA | FAB-MS(m/z); 288 [(M+1)⁺] |
| 58 | 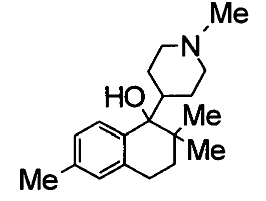 | 1FA | FAB-MS(m/z); 288 [(M+1)⁺] |
| 59 | 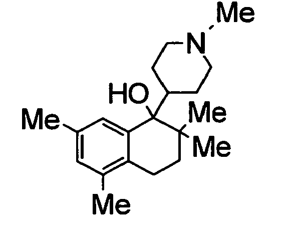 | 1FA | FAB-MS(m/z); 302 [(M+1)⁺] |
| 60 | 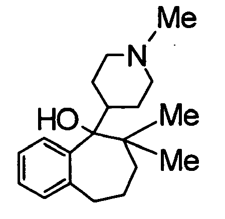 | 1FA | FAB-MS(m/z); 288 [(M+1)⁺] |

**[Table 23]**

| Ex. | STRUCTURE | SALT | DATA |
|---|---|---|---|
| 61 | 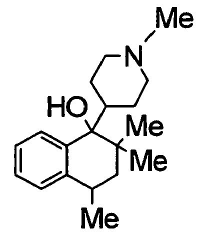 | 1FA | FAB-MS(m/z); 288 [(M+1)⁺] |
| 62 | 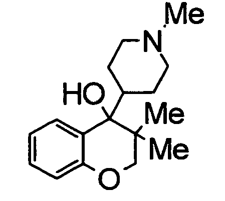 | 1FA | FAB-MS(m/z); 276 [(M+1)⁺] |
| 63 | 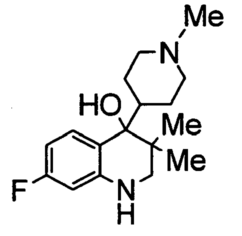 | 1FA | FAB-MS(m/z); 293 [(M+1)⁺] |
| 64 | 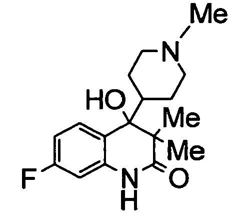 | 1FA | FAB-MS(m/z); 307 [(M+1)⁺] |
| 65 | 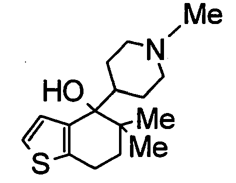 | 1FA | FAB-MS(m/z); 280 [(M+1)⁺] |

**[Table 24]**

| Ex. | STRUCTURE | SALT | DATA |
|---|---|---|---|
| 66 | 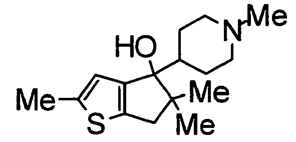 | 1FA | FAB-MS(m/z); 280 [(M+1)⁺] |
| 67 | 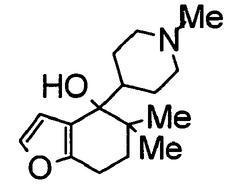 | 1FA | FAB-MS(m/z); 264 [(M+1)⁺] |
| 68 | 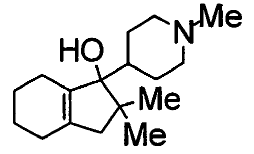 | 1FA | FAB-MS(m/z); 264 [(M+1)⁺] |
| 69 | 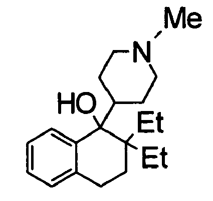 | 1FA | FAB-MS(m/z); 302 [(M+1)⁺] |
| 70 | 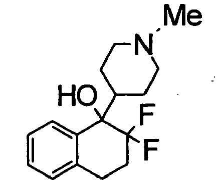 | 1FA | FAB-MS(m/z); 282 [(M+1)⁺] |
| 71 | 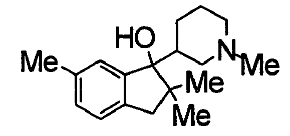 | 1FA | FAB-MS(m/z); 274 [(M+1)⁺] |

**[Table 25]**

| Ex. | STRUCTURE | SALT | DATA |
|---|---|---|---|
| 72 | 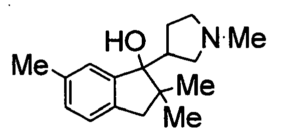 | 1FA | FAB-MS(m/z); 260 [(M+1)⁺] |
| 73 | 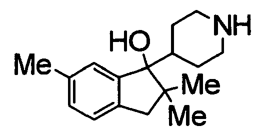 | 1FA | FAB-MS(m/z);260 [(M+1)⁺] |
| 74 | 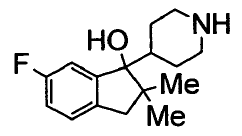 | 1FA | FAB-MS(m/z); 264 [(M+1)⁺] |
| 75 | 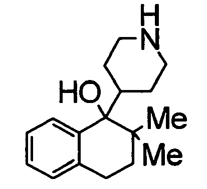 | 1FA | FAB-MS(m/z); 260 [(M+1)⁺] |
| 76 | 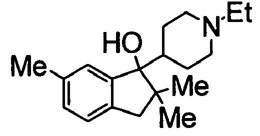 | 1FA | FAB-MS(m/z); 288 [(M+1)⁺] |
| 77 | 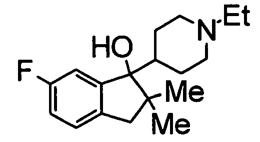 | 1FA | FAB-MS(m/z); 292 [(M+1)⁺] |
| 78 | 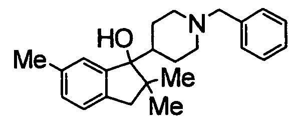 | 1FA | FAB-MS(m/z); 350 [(M+1)⁺] |

**[Table 26]**

| Ex. | STRUCTURE | SALT | DATA |
|---|---|---|---|
| 79 | 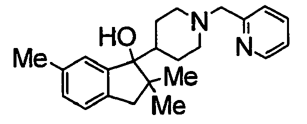 | 1FA | FAB-MS(m/z); 351 [(M+1)⁺] |
| 80 | 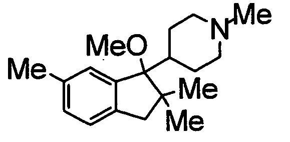 | 1FA | FAB-MS(m/z); 288 [(M+1)⁺] |
| 81 | 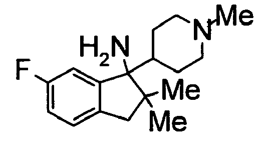 | 1FA | FAB-MS(m/z); 277 [(M+1)⁺] |
| 82 | 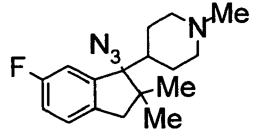 | 1FA | FAB-MS(m/z); 303 [(M+1)⁺] |
| 83 | 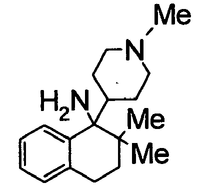 | 1FA | FAB-MS(m/z); 273 [(M+1)⁺] |
| 84 | 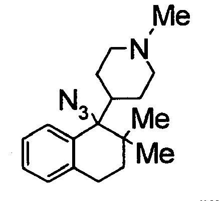 | 1FA | FAB-MS(m/z); 299 [(M+1)⁺] |

**[Table 27]**

| Ex. | STRUCTURE | SALT | DATA |
|---|---|---|---|
| 85 | 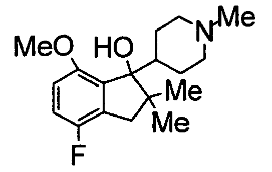 | 1 HCl | FAB-MS(m/z); 308 [(M+1)⁺] ¹H-NMR(DMSO-d₃); 0.95(3H, s), 1.16(3H, s), 1.28-1.44(1H, m), 1.68-2.16(4H, m), 2.55-3.50(9H, m), 3.75(3H, s), 6.84(1H, dd, J=9.2, 4.0 Hz), 7.00(1H, t, J=8.7 Hz), 10.11(1H, br). |
| 86 | 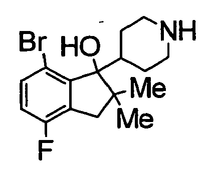 | 0.5FA | FAB-MS(m/z); 342, 344 [(M+1)⁺] ¹H-NMR(DMSO-d₃); 0.87(3H, s), 1.21(3H, s), 1.10-1.35(1H, m), 1.50-1.70(2H, m), 1.75-1.85(1H, m), 2.00-2.10(1H, m), 2.45-2.60(3H, m), 2.73(1H, d, J=16.4 Hz), 3.02(1H, brd, J=12.4 Hz), 3.11(1H, brd, J=12.4 Hz), 3.34(br), 4.78(1H, brs), 6.32(1H, s), 7.00(1H, t, J=8.4 Hz), 7.39(1H, dd, J=4.8, 8.4 Hz). |
| 87 | 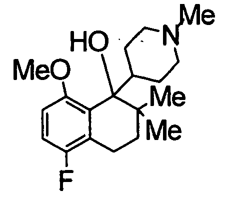 | 1 FA | FAB-MS(m/z); 322 [(M+1)⁺] |
| 88 | 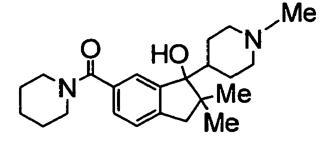 | - | FAB-MS(m/z); 371 [(M+1)⁺] |
| 89 | 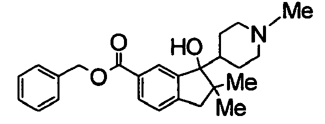 | - | ESI-MS(m/z); 394 [(M+1)⁺] |
| 90 | 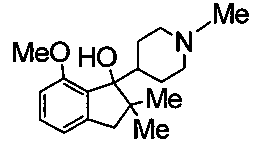 | 1 FA | FAB-MS(m/z); 290 [(M+1)⁺] |

**[Table 28]**

| Ex. | STRUCTURE | SALT | DATA |
|---|---|---|---|
| 91 | 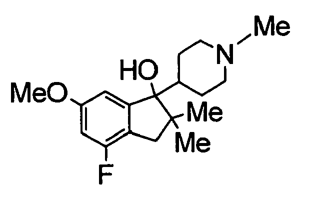 | 1 FA | FAB-MS(m/z); 308 [(M+1)⁺] ¹H-NMR(DMSO-d₃); 0.58-0.72(1H, m), 0.86(3H, s), 1.17(3H, s), 1.39-1.53(1H, m), 1.57-1.67(1H, m), 1.67-1.79(1H, m), 1.96-2.06(1H, m), 2.06-2.35(2H, m), 2.26(3H, s), 2.44-2.55(1H), 2.63(1H, d, J=15.3 Hz), 2.82(1H, brd, J=11.4 Hz), 3.03(1H, brd, J=11.4 Hz), 3.74(3H, s), 4.79(1H, br), 6.50(2H, s), 6.58(1H, d, J=2.1 Hz), 6.63(1H, dd, J=2.1, 10.8 Hz). |
| 92 | 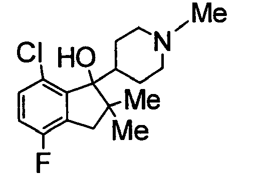 | 1 FA | FAB-MS(m/z); 312 [(M+1)⁺] ¹H-NMR(DMSO-d₃); 0.88(3H, s), 1.19(3H, s), 1.12-1.31(1H, m), 1.58-1.76(2H, m), 1.83-2.00(2H, m), 2.18-2.32(2H, m), 2.35(3H, s), 2.55(1H, d, J=16.9 Hz), 2.73(1H, d, J=16.9 Hz), 2.98(1H, brd, J=11.6 Hz), 3.09(1H, brd, J=11.6 Hz), 4.87(br), 6.49(2H, s), 7.07(1H, t, J=8.4 Hz), 7.22(1H, dd, J=4.4, 8.4 Hz). |
| 93 | 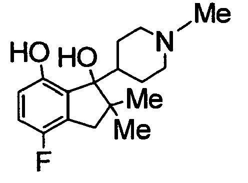 | - | FAB-MS(m/z); 294 [(M+1)⁺] |
| 94 | 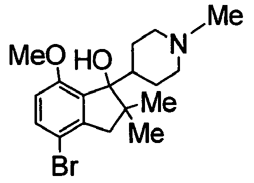 | 1 FA | FAB-MS(m/z); 368, 370 [(M+1)⁺] |
| 95 | 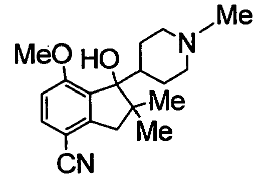 | 1 FA | FAB-MS(m/z); 315 [(M+1)⁺] |

**[Table 29]**

| Ex. | STRUCTURE | SALT | DATA |
|---|---|---|---|
| 96 | 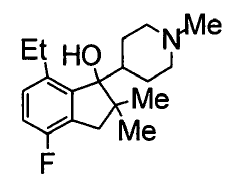 | 1 FA | FAB-MS(m/z); 306 [(M+1)⁺] ¹H-NMR(DMSO-d₃); 0.87(3H, s), 0.95-1.15(1H, m), 1.12(3H, t, J=7.6 Hz), 1.20(3H, s), 1.40-1.68(2H, m), 1.70-1.94(2H, m), 2.18-2.36(2H, m), 2.33(3H, s), 2.44-2.54(1H), 2.58-2.72(2H, m), 2.86-3.14(3H, m), 4.65(l H, br), 6.48(2H, s), 6.91(1H, t, J=8.4 Hz), 7.05(1H, dd, J=4.8, 8.4 Hz). |
| 97 | 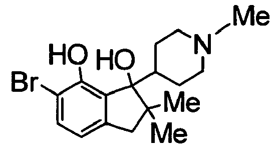 | 1FA | FAB-MS(m/z); 354, 356 [(M+1)⁺] |
| 98 | 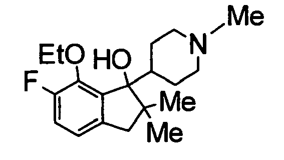 | 1FA | FAB-MS(m/z); 322 [(M+1)⁺] ¹H-NMR(DMSO-d₃); 0.93(3H, s), 1.14(3H, s), 1.04-1.17(1H, m), 1.30(3H, t, J=7.0 Hz), 1.64-1.77(2H, m), 1.82-2.02(2H, m), 2.39(3H, s), 2.29-2.42(2H, m), 2.49(1H, d, J=15.7 Hz), 2.68(1H, d, J=15.7 Hz), 3.01(1H, brd, J=11.4 Hz), 3.15(1H, brd, J=11.4 Hz), 3.97-4.13(2H, m), 4.42(br), 6.47(2H, s), 6.84 (1H, dd, J=4.3, 8.0 Hz), 7.03(1H, dd, J=8.0, 12.0 Hz). |
| 99 | 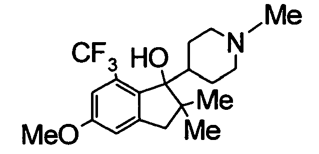 | 1FA | FAB-MS(m/z); 358 [(M+1)⁺] |
| 100 | 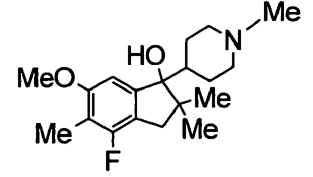 | 1FA | FAB-MS(m/z); 322 [(M+1)⁺] |

**[Table 30]**

| Ex. | STRUCTURE | SALT | DATA |
|---|---|---|---|
| 101 | 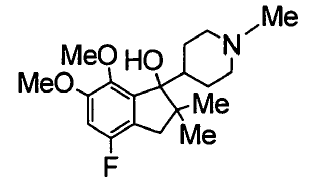 | 1FA | FAB-MS(m/z); 338 [(M+1)⁺] ¹H-NMR(DMSO-d₃); 0.93(3H, s), 1.05-1.19(1H, m), 1.15(3H, s), 1.59-1.73(2H, m), 1.83-1.95(2H, m), 2.28-2.43(2H, m), 2.38(3H, s), 2.49(1H, d, J=15.7 Hz), 2.58(1H, d, J=15.7 Hz), 3.00(1H, brd, J=11.8 Hz), 3.12(1H, brd, J=11.8 Hz), 3.69(3H, s), 3.77(3H, s), 6.47(2H, s), 6.78(1H, d, J=10.0 Hz). |
| 102 | 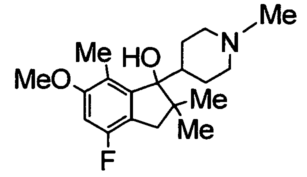 | 1FA | FAB-MS(m/z); 322 [(M+1⁺)] |
| 103 | 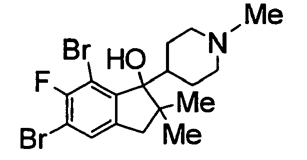 | - | FAB-MS(m/z); 434, 436, 438 [(M+1)⁺] |
| 104 | 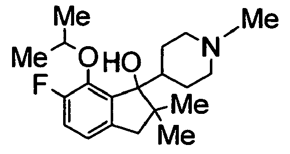 | 1FA | FAB-MS(m/z); 336 [(M+1)⁺] |
| 105 | 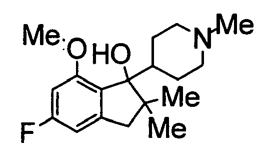 | 1FA | FAB-MS(m/z); 308 [(M+1)⁺] ¹H-NMR(DMSO-d₃); 0.92(3H, s), 1.12(3H, s), 1.06-1.28(1H, m), 1.57-1.74(2H, m), 1.82-1.98(2H, m), 2.30-2.46(2H, m), 2.41(3H, s), 2.50(1H, d,1=16.1 Hz), 2.67(1H, d, J=16.1 Hz), 3.03(1H, brd, J=11.6 Hz), 3.15(1H, brd, J=11.6 Hz), 3.74(3H, s), 6.48(2H, s), 6.57(1H, dd, J=8.5, 1.8 Hz), 6.66(1H, dd, J=11.7, 1.8 Hz). |
| 106 | 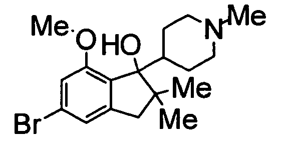 | 1FA | FAB-MS(m/z); 368, 370 [(M+1)⁺] |

**[Table 31]**

| Ex. | STRUCTURE | SALT | DATA |
|---|---|---|---|
| 107 | 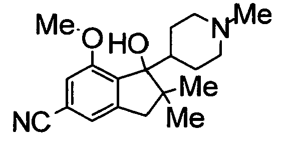 | 1FA | FAB-MS(m/z); 315 [(M+1)⁺] |
| 108 | 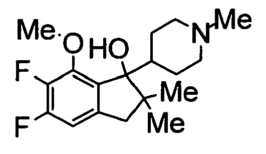 | 1FA | FAB-MS(m/z); 326 [(M+1)⁺] ¹H-NMR(DMSO-d₃); 0.91(3H, s), 1.01-1.16(1H, m), 1.13(3H, s), 1.55-1.70(2H, m), 1.82-1.97(2H, m), 2.23-2.40(2H, m), 2.37(3H, s), 2.48(1H, d, J=15.8 Hz), 2.69(1H, d, J=15.8 Hz), 2.97(1H, brd, J=11.4 Hz), 3.11(1H, brd, J=11.4 Hz), 3.87(3H, d, J=1.8 Hz), 6.48(2H, s), 6.94(1H, dd, J=6.5, 9.6 Hz). |
| 109 | 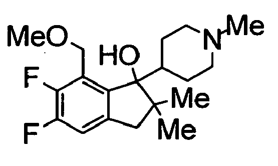 | 0.5FA | FAB-MS(m/z); 340 [(M+1)⁺] ¹H-NMR(DMSO-d₃); 0.71-0.95(1H, m), 0.85(3H, s), 1.17(3H, s), 1.34-1.49(1H, m), 1.53-1.65(1H, m), 1.68-1.89(2H, m), 1.94-2.14(2H, m), 2.21(3H, s), 2.38-2.55(1H, m), 2.72-2.85(2H, m), 2.90-3.01(1H, m), 3.27(3H, s), 4.63(1H, d, J=10.3 Hz), 4.73(1H, d, J=10.3 Hz), 4.82(br), 6.47(1H, s), 7.17(1H, t, J=8.1 Hz). |
| 110 | 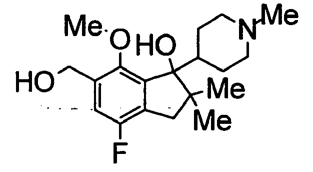 | - | FAB-MS(m/z); 338 [(M+1)⁺] |
| 111 | 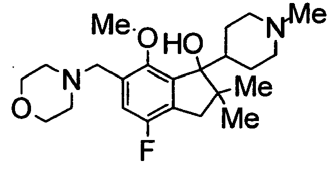 | - | FAB-MS(m/z); 407 [(M+1)⁺] |
| 112 | 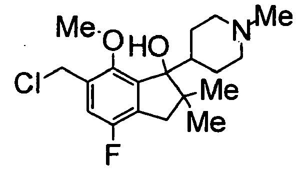 | HCl | ESI-MS(m/z); 356 [(M+1)⁺] |

**[Table 32]**

| Ex. | STRUCTURE | SALT | DATA |
|---|---|---|---|
| 113 | 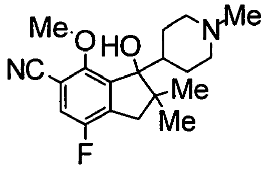 | 1FA | FAB-MS(m/z); 333 [(M+1)⁺] ¹H-NMR(DMSO-d₃); 0.91-1.09(1H, m), 0.94(3H, s), 1.18(3H, s), 1.56-1.73(2H, m), 1.87-2.01(2H, m), 2.27-2.47(2H, m), 2.38(3H, s), 2.65(1H, d, J=17.2 Hz), 2.76(1H, d, J=17.2 Hz), 2.98(1H, brd, J=11.7 Hz), 3.13(1H, brd, J=11.7 Hz), 3.90(3H, s), 6.48(2H, s), 7.64(1H, d, J=7.8 Hz). |
| 114 | 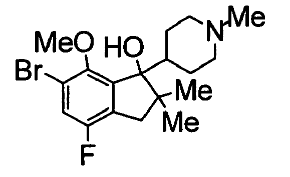 | 1FA | FAB-MS(m/z); 386, 388 [(M+1)⁺] |
| 115 | 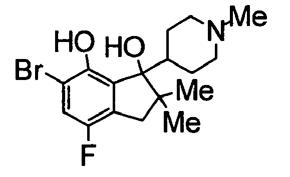 | 1FA | FAB-MS(m/z); 372, 374 [(M+1)⁺] |
| 116 | 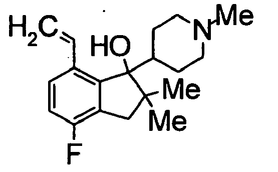 | 1FA | FAB-MS(m/z); 304 [(M+1)⁺] |
| 117 | 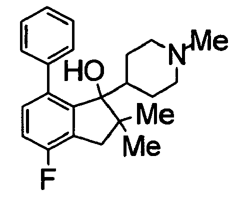 | 1FA | FAB-MS(m/z); 354 [(M+1)⁺] ¹H-NMR(DMSO-d₃); 0.94(3H, s), 1.00(3H, s), 0.85-2.00(7H, m), 2.18(3H, s), 2.66(1H, d, J=16.0 Hz), 2.74(1H, d, J=16.0 Hz), 2.65-2.75(1H), 2.86(1H, brd, J=11.2 Hz), 3.35(br), 4.65(1H, br), 6.51(2H, s), 6.98(1H, dd, J=5.6, 8.4 Hz), 7.07(1H, t, J=8.4 Hz), 7.25-7.36(3H, m), 7.40-7.50(2H, m). |

**[Table 33]**

| Ex. | STRUCTURE | SALT | DATA |
|---|---|---|---|
| 118 | 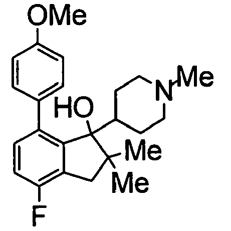 | 1FA | FAB-MS(m/z); 384 [(M+1)⁺] |
| 119 | 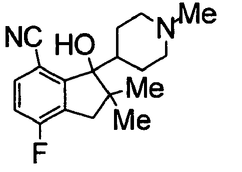 | 1FA | FAB-MS(m/z); 303 [(M+1)⁺] ¹H-NMR(DMSO-d₃); 0.85(3H, s), 0.70-1.00(1H, m), 1.21(3H, s), 1.40-2.40(6H, m), 2.29(3H, s), 2.58(1H, d, J=16.8 Hz), 2.80(1H, d, J=16.8 Hz), 2.84(1H, brd, J=10.8 Hz), 3.08(1H, brd, J=10.8 Hz), 5.24(1H, br), 6.50(2H, s), 7.26(1H, brt, J=8.4 Hz), 7.72(1H, brs). |
| 120 | 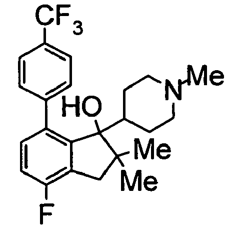 | 1FA | FAB-MS(m/z); 422 [(M+1)⁺] |
| 121 | 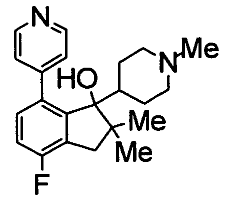 | 2 FA | FAB-MS(m/z); 355 [(M+1)⁺] |

**[Table 34]**

| Ex. | STRUCTURE | SALT | DATA |
|---|---|---|---|
| 122 | 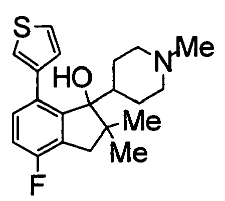 | 1FA | FAB-MS(m/z); 360 [(M+1)⁺] ¹H-NMR(DMSO-d₃); 0.80-1.35(3H, m), 1.06(3H, s), 1.13(3H, s), 1.60-1.80(3H, m), 1.90-2.10(1H, m), 2.22(3H, s), 2.65(1H, d, J=16.4 Hz), 2.71(1H, d, J=16.4 Hz), 2.74(1H, brd, J=10.8 Hz), 2.87(1H, brd, J=10.8 Hz), 4.73(1H, br), 6.51(2H, s), 7.00-7.14(2H, m), 7.31(1H, d, J=4.8 Hz), 7.46(1H, dd, J=2.8, 4.8 Hz), 7.55(1H, d, J=2.8 Hz). |
| 123 | 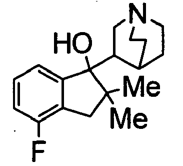 | HCl | FAB-MS(m/z); 290 [(M+1)⁺] |
| 124 | 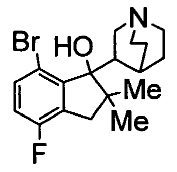 | HCl | FAB-MS(m/z); 368, 370 [(M+1)⁺] |
| 125 | 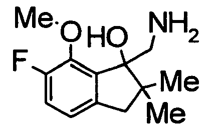 | 1FA | FAB-MS(m/z); 240 [(M+1)⁺] |
| 126 | 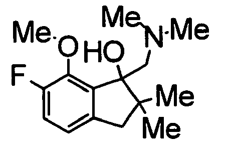 | 1FA | FAB-MS(m/z); 268 [(M+1)⁺] |
| 127 | 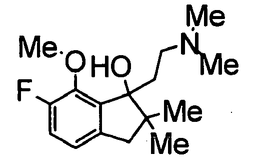 | 1FA | FAB-MS(m/z); 282 [(M+1)⁺] |

**[Table 35]**

| Ex. | STRUCTURE | SALT | DATA |
|---|---|---|---|
| 128 | 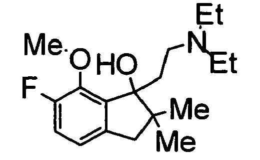 | 1FA | FAB-MS(m/z); 310 [(M+1)⁺] |
| 129 | 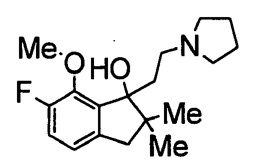 | 1FA | FAB-MS(m/z); 308 [(M+1)⁺] |
| 130 | 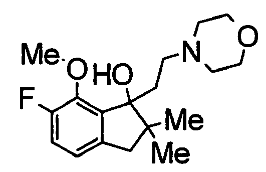 | 1FA | FAB-MS(m/z); 324 [(M+1)⁺] |
| 131 | 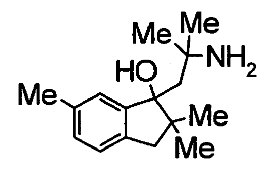 | 0.5FA | FAB-MS(m/z); 248 [(M+1)⁺] ¹H-NMR(DMSO-d₃); 0.85(3H, s), 0.95(3H, s), 1.14(3H, s), 1.23(1H, s), 1.70(1H, d, J=15.2 Hz), 1.96(1H, d, J=15.2 Hz), 2.29(3H, s), 2.51(1H, d, J=14.8 Hz), 2.56(1H, d, J=14.8 Hz), 3.31(br), 6.37(1H, s), 6.95(1H, d, J=7.6 Hz), 7.01(1H, d, J=7.6 Hz), 7.29(1H, s). |
| 132 | 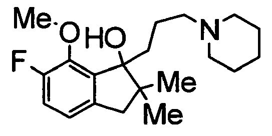 | 1FA | FAB-MS(m/z); 336 [(M+1)⁺] |
| 133 | 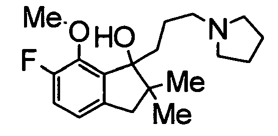 | 1FA | FAB-MS(m/z); 322 [(M+1)⁺] |

**[Table 36]**

| Ex. | STRUCTURE | SAL T | DATA |
|---|---|---|---|
| 134 | 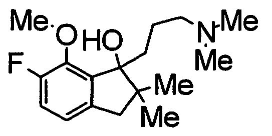 | 1FA | FAB-MS(m/z); 296 [(M+1)⁺] |
| 135 | 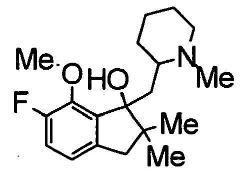 | 1FA | FAB-MS(m/z); 322 [(M+1)⁺] |
| 136 | 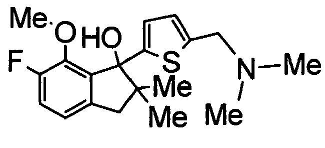 | 1FA | FAB-MS(m/z); 350 [(M+1)⁺] |
| 137 | 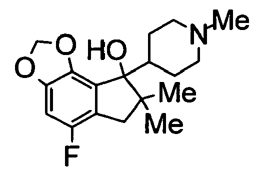 | 1FA | ESI-MS(m/z); 322 [(M+1)⁺] ¹H-NMR(DMSO-d₃); 0.91(3H, s), 0.86-1.01(1H, m), 1.14(3H, s), 1.50-1.87(3H, m), 2.01-2.11(1H, m), 2.14-2.35(2H, m), 2.32(3H, s), 2.53(1H, d, J=15.7 Hz), 2.60(1H, d, J=15.7 Hz), 2.90(1H, brd, J=11.5 Hz), 3.06(1H, brd, J=11.5 Hz), 3.33(br), 5.97(2H, d, 9.9 Hz), 6.49(2H, s), 6.73(1H, d, J=8.4 Hz). |
| 138 | 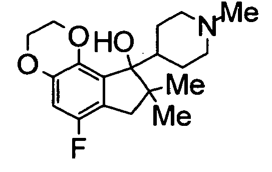 | 1FA | FAB-MS(m/z); 336 [(M+1)⁺] |

**[Table 37]**

| Ex. | STRUCTURE | SALT | DATA |
|---|---|---|---|
| 139 | 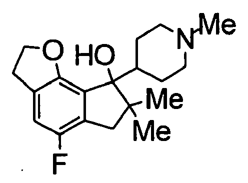 | 1FA | FAB-MS(m/z); 320 [(M+1)⁺] ¹H-NMR(DMSO-d₃); 0.91(3H, s), 0.96-1.17(1H, m), 1.12(3H, s), 1.62-1.78(2H, m), 1.82-1.92(1H, m), 1.97-2.07(1H, m), 2.29-2.44(2H, m), 2.39(3H, s), 2.54(1H, d, J=16.1 Hz), 2.59(1H, d, J=16.1 Hz), 2.99(1H, brd, J=11.3 Hz), 3.08(2H, t, J=8.3 Hz), 3.14(1H, brd, J=11.3 Hz), 4.42-4.53(2H, m), 6.45(2H, s), 6.89(1H, d, J=8.4 Hz). |
| 140 | 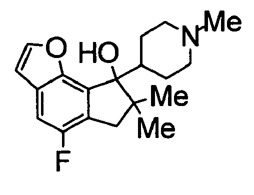 | 1FA | FAB-MS(m/z); 318 [(M+1)⁺] ¹H-NMR(DMSO-d₃); 0.83-1.00(1H, m), 0.95(3H, s), 1.21(3H, s), 1.57-1.79(2H, m), 1.92-2.04(1H, m), 2.17-2.28(1H, m), 2.35(3H, s), 2.30-2.46(2H, m), 2.70(1H, d, J=15.9 Hz), 2.80(1H, d, J=15.9 Hz), 2.93(1H, brd, J=11.5 Hz), 3.14(1H, brd, J=11.5 Hz), 6.41(2H, s), 6.88(1H, d, J=2.3 Hz), 7.28(1H, d, J=8.5 Hz), 7.96(1H, d, J=2.3 Hz). |
| 141 | 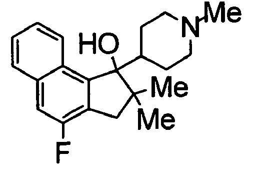 | 1FA | FAB-MS(m/z); 328 [(M+1)⁺] |
| 142 | 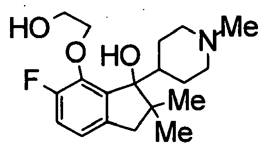 | - | ESI-MS(m/z); 338 [(M+1)⁺] |
| 143 | 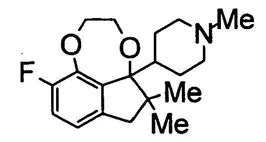 | 1FA | FAB-MS(m/z); 320 [(M+1)⁺] ¹H-NMR(DMSO-d₃); 0.97(3H, s), 1.19(3H, s), 1.10-1.28(1H, m), 1.49-1.62(1H, m), 1.68-1.89(2H, m), 1.93-2.05(1H, m), 2.25-2.43(2H, m), 2.40(3H, s), 2.46(1H, d, J=15.8 Hz), 2.76(1H, d, J=15.8 Hz), 3.05(1H, brd, J=11.8 Hz), 3.11(1H, brd, J=11.8 Hz), , 3.89-3.99(2H, m), 4.06-4.16(1H, m), 4.42-4.50(1H, m), 6.51(2H, s), 6.82(1H, dd, J=4.1, 8.0 Hz), 7.06(1H, dd, J=8.0, 11.1 Hz). |
| 144 | 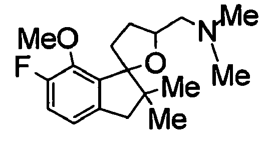 | 1FA | FAB-MS(m/z); 308 [(M+1)⁺] |

**[Table 38]**

| Ex. | STRUCTURE | SALT | DATA |
|---|---|---|---|
| 145 | 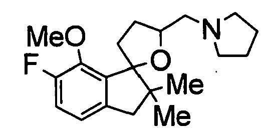 | 1FA | FAB-MS(m/z); 334 [(M+1)⁺] |
| 146 | 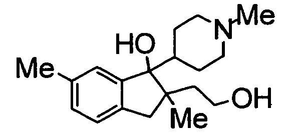 | - | ESI-MS(m/z); 304 [(M+1)⁺] |
| 147 | 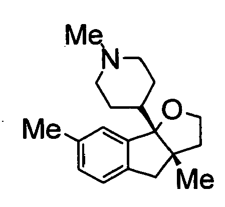 | 1FA | FAB-MS(m/z); 286 [(M+1)⁺] ¹H-NMR(DMSO-d₃); 0.56-0.69(1H, m), 1.25(3H, s), 1.45-1.54(1H, m), 1.58-1.68(!H, m), 1.75-1.86(3H, m), 1.93-2.03(1H, m), 2.28(3H, s), 2.35(3H, s), 2.30-2.45(2H, m), 2.70(1H, d, J=15.7 Hz), 2.83(1H, d, J=15.7 Hz), 2.89(1H, brd, J=11.8 Hz), 3.13(1H, brd, J=11.8 Hz), 3.32-3.40(1H, m), 3.76(1H, q, J=8.1 Hz), 6.47(2H, s), 7.01(1H, s), 7.03(1H, d, J=7.8 Hz), 7.08(1H, d, J=7.8 Hz). |
| 148 | 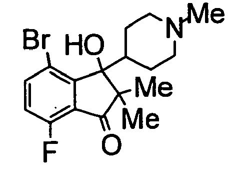 | - | FAB-MS(m/z); 370, 372 [(M+1)⁺] |
| 149 | 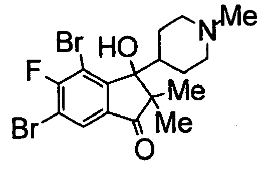 | - | FAB-MS(m/z); 448, 450, 452 [(M+1)⁺] |
| 150 | 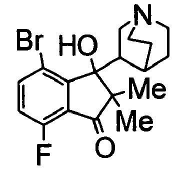 | - | FAB-MS(m/z); 382, 384 [(M+1)⁺] |

**[Table 39]**

| Ex. | STRUCTURE | SALT | DATA |
|---|---|---|---|
| 151 | 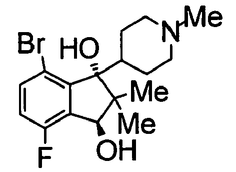 | - | FAB-MS(m/z); 372, 374 [(M+1)⁺] ¹H-NMR(DMSO-d₃); 0.78(3H, s), 1.11(3H, s), 1.18-1.25(1H, m), 1.55-1.76(5H, m), 2.07(3H, s), 2.02-2.16(1H, m), 2.65-2.80(2H, m), 4.44(1H, s), 4.50(1H, d, J=5.3 Hz), 5.56(1H, d, J=5.3 Hz), 6.99(1H, t, J=8.5 Hz), 7.46(1H, dd, J=4.6, 8.5 Hz). |
| 152 | 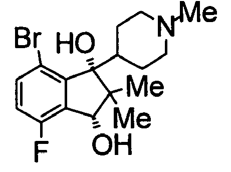 | - | FAB-MS(m/z); 372, 374 [(M+1)⁺] |
| 153 | 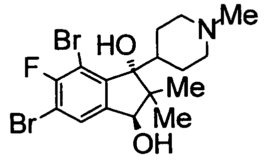 | - | FAB-MS(m/z); 450, 452, 454 [(M+1)⁺] |
| 154 | 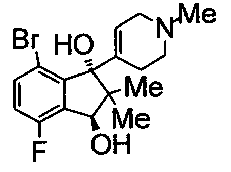 | - | FAB-MS(m/z); 370, 372 [(M+1)⁺] ¹H-NMR(DMSO-d₃); 0.87(3H, s), 0.93(3H, s), 1.50-1.68(1H, m), 2.17(3H, s), 2.26-2.39(3H, m), 2.76-2.91(2H, m), 4.60(1H, s), 4.71(1H, d, J=4.8 Hz), 5.46(1H, d, J=4.8 Hz), 5.40-5.52(1H, br), 7.05(1H, t, J=8.8 Hz), 7.48(1H, dd, J=4.4, 8.8 Hz). |
| 155 | 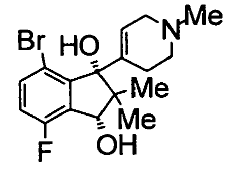 | - | EI-MS(m/z); 369, 371 [(M+1)⁺] |

**[Table 40]**

| Ex. | STRUCTURE | SALT | DATA |
|---|---|---|---|
| 156 | 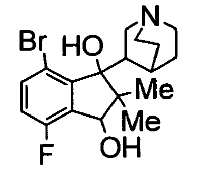 | - | ESI-MS(m/z); 384, 386 [(M+1)⁺] |
| 157 | 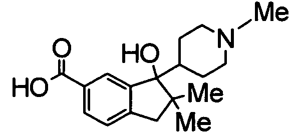 | - | FAB-MS(m/z); 304 [(M+1)⁺] |
| 158 | 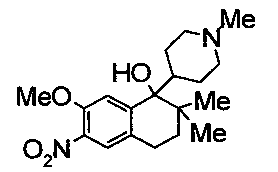 | 1 FA | ESI-MS(m/z); 349[(M+1)⁺] |
| 159 | 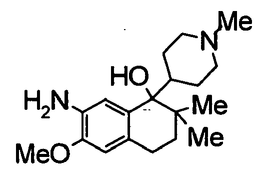 | - | ESI-MS(m/z); 319[(M+1)⁺] |
| 160 | 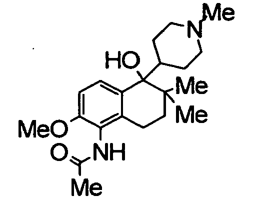 | - | FAB-MS(m/z); 361[(M+1)⁺] |
| 161 | 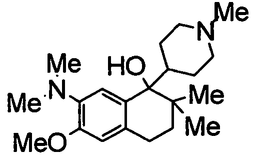 | - | FAB-MS(m/z); 347 [(M+1)⁺] |

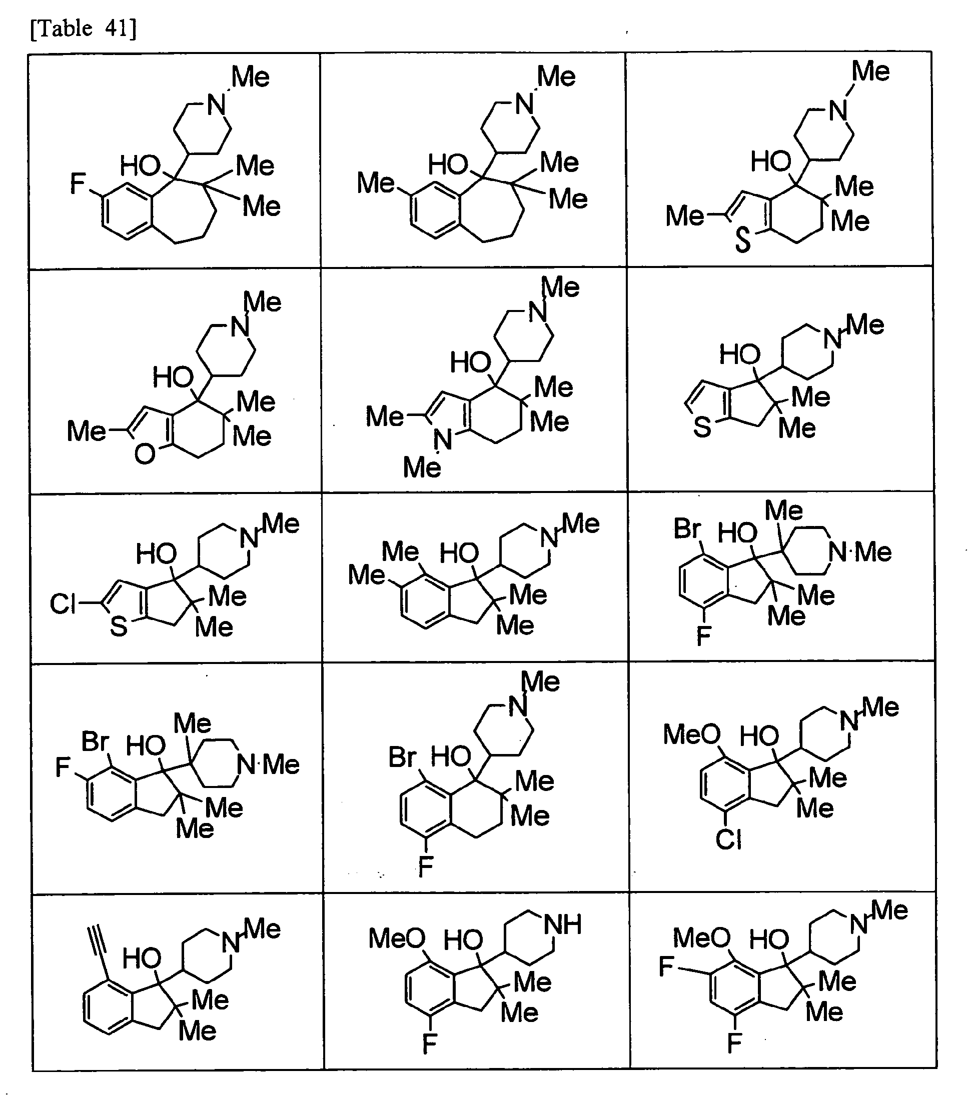

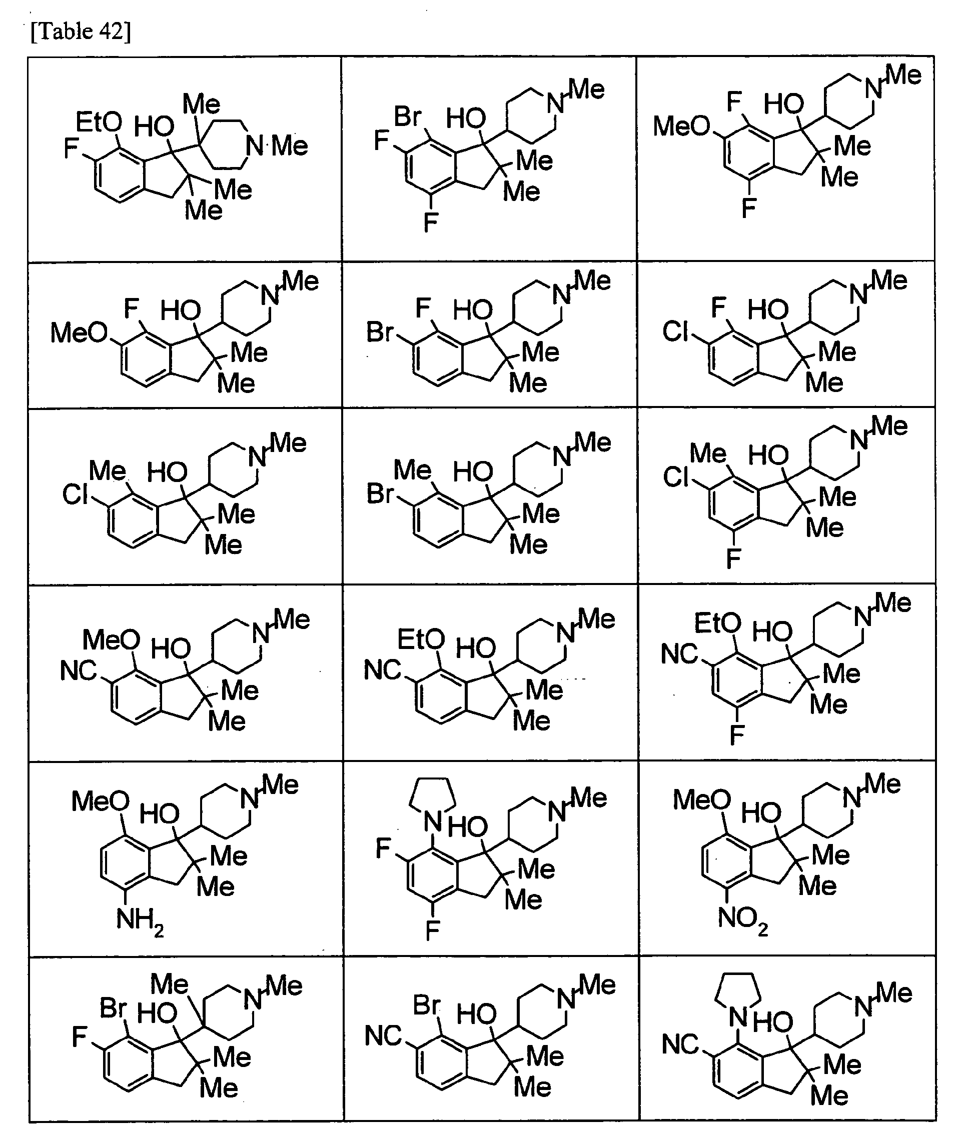

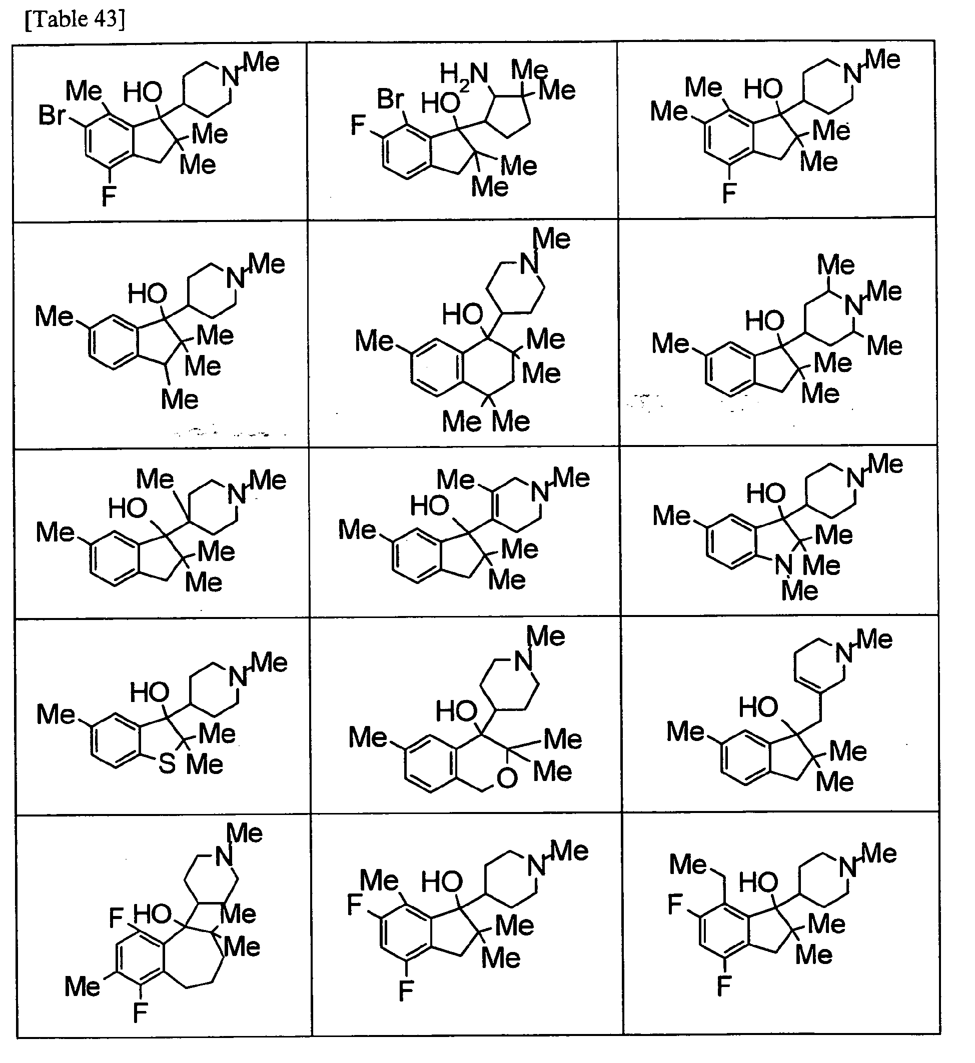

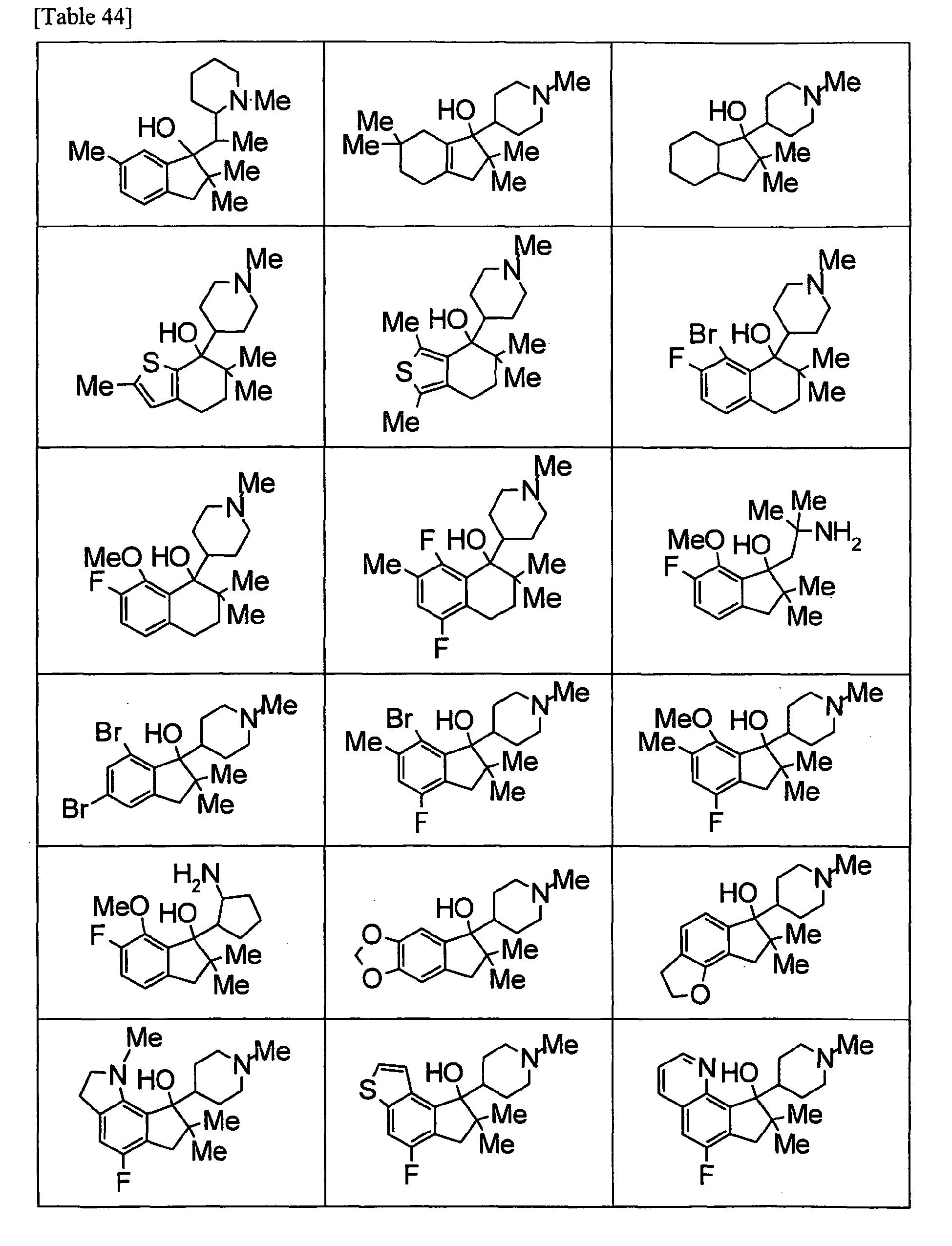

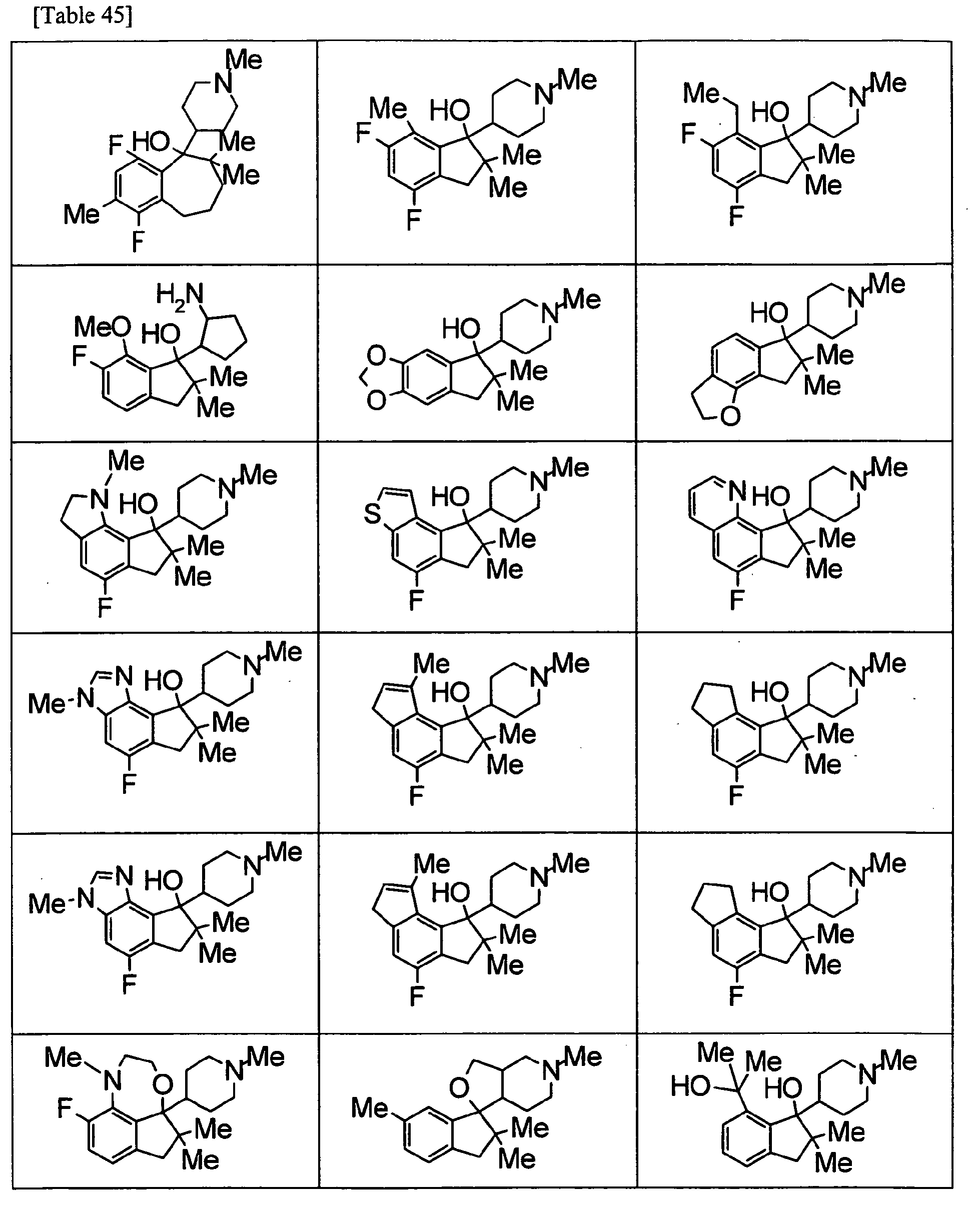

### INDUSTRIAL APPLICABILITY

As described hereinabove, the compounds of the present invention have an NMDA receptor antagonistic activity, and are useful for treatment and prevention of Alzheimer's disease, cerebrovascular dementia, Parkinson's disease, ischemic apoplexy, pain, etc., and they have many industrial applications.

## Claims

1. A cyclic amine and its salt of the following formula (I): 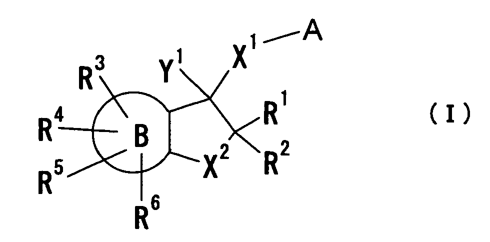 (wherein the symbols have the following meanings:
A: a 5- to 8-membered cyclic amine optionally having a double bond, optionally having a bridge structure and optionally having a substituent of R⁷ to R¹¹ in the ring, or -NH₂, -NH(lower alkyl), or -N(lower alkyl)₂;
Ring B: benzene, thiophene, furan, pyrrole; a 5- to 7-membered cycloalkane, or a 5- to 7-membered cycloalkene;
X¹: a bond, a lower alkylene, or -L³-D-L⁴-;
L³ and L⁴ are the same or different, each representing a bond or a lower alkylene;
D: a 5- or 6-membered carbon ring or 5- or 6-membered hetero ring, optionally having a substituent;
X²: -(CR¹²R¹³)n-, -N(R¹⁴)-, -N(R¹⁴)CO-, -CON(R¹⁴)-, -O-, -S-, -CO-, -CH(OH)-, -N(R¹⁴)-(CR¹²R¹³)n-, -(CR¹²R¹³)n-N(R¹⁴)-, -CON(R¹⁴)-(CR¹²R¹³)n-, -N(R¹⁴)CO-(CR¹²R¹³)n-, -(CR¹²R¹³)n-N(R¹⁴)CO-, -(CR¹²R¹³)n-CON(R¹⁴)-, -CO-(CR¹²R¹³)n-, -(CR¹²R¹³)n-CO-, -O-(CR¹²R¹³)n-, -(CR¹²R¹³)n-O-, -S-(CR¹²R¹³)n-, or -(CR¹²R¹³)n-S-;
Y¹: -OH, -O-lower alkyl, -NH₂, or -N₃;
R¹ and R²: the same or different, and a halogen atom, a lower alkyl, or a lower alkylene-OH;
R³ to R⁶: the same or different, and a hydrogen atom, a halogen atom, a lower alkyl, a lower alkenyl, a lower alkynyl, -O-lower alkyl, -OH, -NH₂, -NH(lower alkyl), -N(lower alkyl)₂, -NH-CO-lower alkyl, -N(lower alkyl)-CO-lower alkyl, -NH-CO-O-lower alkyl, -N(lower alkyl)-CO-O-lower alkyl, -CN-, -NO₂, -CF₃, -lower alkylene-OH, -lower alkylene-halogen atom, -O-lower alkylene-OH, -lower alkylene-O-lower alkyl, -CO-N(lower alkyl)₂, -CO-NH-(lower alkyl), -O-CO-N(lower alkyl)₂, -O-CO-NH-(lower alkyl), a 5- to 8-membered cyclic amine, -CO-5- to 8-membered cyclic amine, -COOH, -COO-lower alkyl, -COO-lower alkylene-aryl, a 5- or 6-membered hetero ring, -lower alkylene-5- or 6-membered hetero ring, an aryl optionally having a substituent, or an aryl optionally having -lower alkylene-substituent;
R⁷: a hydrogen atom, a lower alkyl, -lower alkylene-aryl, or -lower alkylene-heteroaryl:
R⁷ is a substituent on the nitrogen atom of the cyclic amine;
R⁸ to R¹⁴: the same or different, and a hydrogen atom, or a lower alkyl;
n: an integer of 1, 2 or 3;
wherein R⁵ and R⁶, R⁴ and R⁵, or R³ and R⁴ may together form a lower alkylene, -O-lower alkylene-O-, -O-lower alkylene-, -lower alkylene-O-, -S-lower alkylene-, -lower alkyleneS-, -N(R¹⁹)-lower alkylene-, -lower alkylene-N(R¹⁹)-, -C(R¹⁵)=C(R¹⁶)-O-, -O-C(R¹⁵)=C(R¹⁶)-, -C(R¹⁵)=C(R¹⁶)-C(R¹⁷)=C(R¹⁸)-, -S-C(R¹⁵)=C(R¹⁶)-, -C(R¹⁵)=C(R¹⁶)-S-, -N(R¹⁹)-C(R¹⁵)=C(R¹⁶)-, -C(R¹⁵)=C(R¹⁶)-N(R¹⁹)-, -N(R¹⁹)-C(R¹⁵)=N-, -N=C(R¹⁵)-N(R¹⁹)-, -N=C(R¹⁵)-C(R¹⁶)=C(R¹⁷)-, -C(R¹⁵)=C(R¹⁶)-C(R¹⁷)=N-, -C(R¹⁵)=N-C(R¹⁶)=C(R¹⁷)-, or -C(R¹⁵)=C(R¹⁶)-N=C(R¹⁷)-; R³ and Y¹ may together form -O-lower alkylene-O-, -lower alkylene-O-, or -N(R¹⁹)-lower alkylene-O-; R¹ and Y¹ may together form -lower alkylene-O-; and Y¹ and the branch on -X¹-A may together form -O-, or -O-lower alkylene;
R¹⁵ to R¹⁸: the same or different, and a hydrogen atom, a halogen atom, or a lower alkyl group;
R¹⁹: a hydrogen atom, or a lower alkyl group;
provided that 6-chloro-2,2-dimethyl-1-(1-methyl-4-piperidinyl)indan-1-ol is excluded from the compound).

2. A cyclic amine derivative and its salt of the following formula (II): 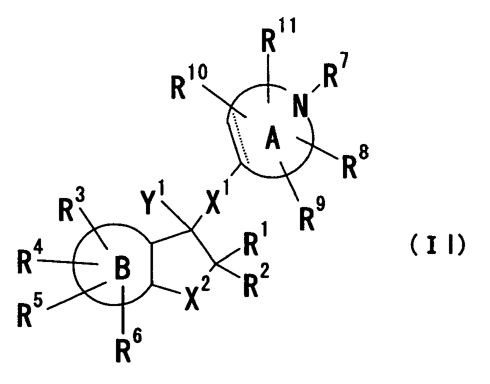 (wherein the symbols have the following meanings:
Ring A: a 5- to 7-membered cyclic amine optionally having a double bond in the ring;
Ring B: benzene, thiophene, furan, pyrrole, a 5- to 7-membered cycloalkane, or a 5- to 7-membered cycloalkene;
X¹: a bond, or a lower alkylene;
X²: -(CR¹²R¹³)ₙ-, -N(R¹⁴)-, -N(R¹⁴)CO-, -CON(R¹⁴)-, -O-, -S-, -CO-, -N(R¹⁴)-(CR¹²R¹³)n-, -(CR¹²R¹³)n-N(R¹⁴)-, -CON(R¹⁴)-(CR¹²R¹³)n-, -N(R¹⁴)CO-(CR¹²R¹³)n-, -(CR¹²R¹³)n-N(R¹⁴)CO-, -(CR¹²R¹³)n-CON(R¹⁴)-, -CO-(CR¹²R¹³)n-, -(CR¹²R¹³)n-CO-, -O-(CR¹²R¹³)n--(CR¹²R¹³)n-O-, -S-(CR¹²R¹³)n-, or -(CR¹²R¹³)n-S-;
Y¹: -OH, -O-lower alkyl, -NH₂, or -N₃;
R¹ and R²: the same or different, and a halogen atom, or a lower alkyl;
R³ to R⁶: the same or different, and a hydrogen atom, a halogen atom, a lower alkyl, -O-lower alkyl, -OH, -CN, or -CF₃;
R⁷: a hydrogen atom, a lower alkyl, -lower alkylene-aryl, or -lower alkylene-heteroaryl:
R⁸ to R¹⁴ : the same or different, and a hydrogen atom, or a lower alkyl;
n: an integer of 1, 2 or 3;
provided that 6-chloro-2,2-dimethyl-1-(1-methyl-4-piperidinyl)indan-1-ol is excluded from the compound).

3. A cyclic amine and its salt of the following formula (III): 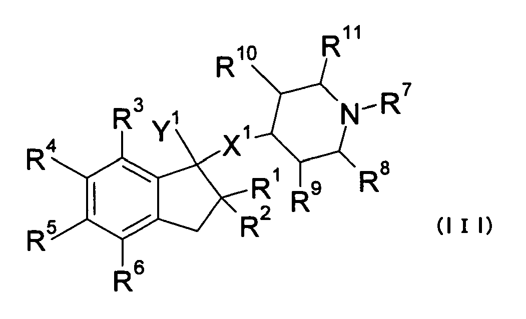 (wherein the symbols have the following meanings:
X¹: a bond, or a lower alkylene;
Y¹: -OH, -O-lower alkyl, -NH₂ or -N₃;
R¹ and R²: the same or different, and a halogen atom, or a lower alkyl;
R³ to R⁶: the same or different, and a hydrogen atom, a halogen atom, a lower alkyl, -O-lower alkyl, -OH, -CN, or -CF₃;
R⁷: a hydrogen atom, a lower alkyl, -lower alkylene-aryl, or -lower alkylene-heteroaryl;
R⁸ to R¹¹: the same or different, and a hydrogen atom, or a lower alkyl;
provided that 6-chloro-2,2-dimethyl-1-(1-methyl-4-piperidinyl)indan-1-ol is excluded from the compound).

4. The compound and its salt as claimed in claim 1, selected from 2,2-dimethyl-1-(1-methylpiperidin-4-yl)indan-1-ol, 2,2,6-trimethyl-1-(1-methylpiperidin-4-yl)indan-1-ol, 6-fluoro-2,2-dimethyl-1-(1-methylpiperidin-4-yl)indan-1-ol, 5-bromo-2,2-dimethyl-1-(1-methylpiperidin-4-yl)indan-1-ol, 6-chloro-5-fluoro-2,2-dimethyl-1-(1-methylpiperidin-4-yl)indan-1-ol, 6-bromo-4-fluoro-2,2-dimethyl-1-(1-methylpiperidin-4-yl)indan-1-ol, 7-bromo-6-fluoro-2,2-dimethyl-1-(1-methylpiperidin-4-yl)indan-1-ol, 7-bromo-4-fluoro-2,2-dimethyl-1-(1-methylpiperidin-4-yl)indan-1-ol, 6-fluoro-7-methoxy-2,2-dimethyl-1-(1-methylpiperidin-4-yl)indan-1-ol, 4-fluoro-7-methoxy-2,2-dimethyl-1-(1-methylpiperidin-4-yl)indan-1-ol, 2,2-dimethyl-1-(1-methylpiperidin-4-yl)-1,2,3,4-tetrahydronaphthalen-1-ol, 4-fluoro-6-methoxy-2,2-dimethyl-1-(1-methylpiperidin-4-yl)indan-1-ol, 7-ethoxy-6-fluoro-2,2-dimethyl-1-(1-methylpiperidin-4-yl)indan-1-ol, 4-fluoro-6,7-dimethoxy-2,2-dimethyl-1-(1-methylpiperidin-4-yl)indan-1-ol, 5,6-difluoro-7-methoxy-2,2-dimethyl-1-(1-methylpiperidin-4-yl)indan-1-ol, 6-cyano-4-fluoro-7-methoxy-2,2-dimethyl-1-(1-methylpiperidin-4-yl)indan-1-ol, 1-(2-amino-2-methylpropyl)-2,2,6-trimethylindan-1-ol, 5-fluoro-7,7-dimethyl-8-(1-methylpiperidin-4-yl)-3,6,7,8-tetrahydro-2H-indeno[4,5-b]furan-8-ol, 4-(9-fluoro-5,5-dimethyl-2,3,5,6-tetrahydro-4aH-indeno[1,7-ef][1,4]dioxepin-4a-yl-1-methylpiperidine, 1-[(6'-fluoro-7'-methoxy-2',2'-dimethyl-2',3',4,5-tetrahydro-3H-spiro[furan-2,1'-inden]-5-yl)methyl]pyrrolidine, 7-bromo-2,2-dimethyl-(1-methylpiperidin-4-yl)indan-1-ol.

5. A pharmaceutical composition comprising a cyclic amine derivative or a salt thereof according to claim 1.

6. The pharmaceutical composition as claimed in claim 1, which is an NMDA receptor antagonist.

7. The pharmaceutical composition as claimed in claim 1, which is an agent for treating dementia.

8. Use of a cyclic amine or a salt thereof according toclaim 1 for the production of an NMDA receptor antagonist or an agent for treating dementia.

9. A method for treating dementia, which comprises administering a therapeutically effective amount of a cyclic amine derivative or a salt thereof according to claim 1 to a patient.
